# EUROPEAN PATENT APPLICATION

(11) **EP 4 169 904 A1**
(43) Date of publication of application: **26.04.2023**
(21) Application number: 21315226.7
(22) Date of filing: 25.10.2021
(51) Int. Cl.: C07D 215/44, C07D 401/04, A61P 35/00, C07D 401/12, C07D 405/12, C07D 409/12, C07D 417/04, C07D 487/08, C07D 487/10, A61K 31/4706, A61P 37/00

(54) **SUBSTITUTED QUINOLINES AS IMPROVED NF-KB-INDUCING KINASE (NIK) INHIBITORS**

(71) Applicant: Yukin Therapeutics, 06902 Sophia Antipolis Cedex (FR); Institut National de la Santé et de la Recherche Médicale (INSERM), 75013 Paris (FR); Centre Hospitalier Universitaire de Nice (CHU Nice), 06000 Nice (FR); Université Côte d'Azur, 06103 Nice Cedex 2 (FR); Centre National de la Recherche Scientifique (CNRS), 75016 Paris (FR)
(72) Inventor: Houacine, Jemila, 06902 Sophia Antipolis Cedex (FR); Dao, Pascal, 06902 Sophia Antipolis Cedex (FR); Passeron, Thierry, 06204 Nice cedex 3 (FR); Beranger, Guillaume, 06204 Nice cedex 3 (FR); Benhida, Rachid, 06108 Nice cedex 02 (FR); Gourhant, Mathilde, 06902 Sophia Antipolis Cedex (FR); Foussat, Arnaud, 06902 Sophia Antipolis Cedex (FR)
(74) Representative: Vossius & Partner Patentanwälte Rechtsanwälte mbB

(57) **Abstract**

The present invention provides novel compounds of formula (I) and pharmaceutical compositions containing these compounds. The compounds of formula (I) can act as NF-κB-inducing kinase (NIK) inhibitors and, in particular, can induce the intracellular degradation of NIK, which renders these compounds highly advantageous for use in therapy, including in the treatment or prevention of cancer, inflammatory diseases and autoimmune diseases.

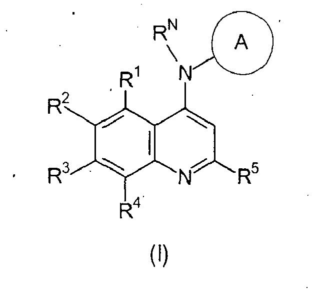

## Description

The present invention provides novel compounds of formula (I) and pharmaceutical compositions containing these compounds. The compounds of formula (I) can act as NF-κB-inducing kinase (NIK) inhibitors and, in particular, can induce the intracellular degradation of NIK, which renders these compounds highly advantageous for use in therapy, including in the treatment or prevention of cancer, inflammatory diseases and autoimmune diseases.

Activation of the NF-κB. pathways has been involved in many pathological conditions including cancer and inflammatory diseases. Besides the canonical NF-κB pathway mainly triggered during pro-inflammatory processes, the non-canonical NF-κB pathway (or NF-κB2) is also overactive in many cancers and in B-cell mediated autoimmune pathologies. This non-canonical NF-κB pathway is known to be regulated by the activity of the kinase NIK (NF-κB-Inducing Kinase) also called MAP3K14. In adult tissues, NIK is constitutively expressed as a protein but is also constitutively degraded via the proteasome. Triggering of specific receptors (CD40LR, BAFFR, Lymphotoxin Beta R) uncouples NIK from its protein partners, inhibits its constitutive degradation and consequently increases its stability within the cytosol, boosting thus its activity as governor of the NF-KB2 cascade (reviewed in Sun SC, Nat Rev Immunol, 2017,17(9):545-558, doi:10.1038/nri.2017.52). NIK is overexpressed in many cancers of hematologic and solid origin and has been described as one of the key molecules involved in cancer cell proliferation, tumorigenicity and immune escape (Maubach G et al., Biochim Biophys Acta Rev Cancer, 2019, 1871(1):40-49, doi:10.1016/j.bbcan.2018.10.002). As an example, in melanoma cells, NIK regulates positively the expression of EZH2, an oncogenic lysine methyltransferase repressing the gene expression of immunogenic factors and genes involved in cellular senescence (De Donatis GM et al., Oncogene, 2016, 35(21):2735-45, doi:10.1038/onc.2015.331). More recently, a pool of intracellular NIK located in the mitochondrial outer membrane of glioblastoma cells has been discovered and has been found involved in mitochondrial fission, a process allowing cancer cells to multiply the number of mitochondria per cell to adapt to the high energy demand linked to intense proliferation. Interestingly, the role of NIK in mitochondrial fission is independent from its activity on the NF-KB2 pathway. In addition, NIK is involved in the energetic supply of cancer cells in limited nutrient environment, allowing malignant cells to switch from glycolysis to oxidative phosphorylation for ATP production (Kamradt ML et al., Cell Death Dis, 2021, 12(3):271, doi:10.1038/s41419-020-03383-z). In B cells, NIK is involved in the differentiation from naive cells to memory antibody-secreting B cells and is a critical factor in autoimmune diseases driven by pathologic production of autoantibodies such a systemic lupus erythematosus (SLE) (Brightbill HD et al., Nat Commun, 2018, 9(1):179, doi:10.1038/s41467-017-02672-0).

In view of the prominent role of NIK in the pathogenesis of cancers, inflammatory diseases and autoimmune diseases, intense research and development efforts have been made to discover small molecules with the ability to specifically modulate NIK kinase activity.

To date, a wide range of small molecules have been described as NIK inhibitors. For example, staurosporine, a pan kinase inhibitor, was found to inhibit the kinase activity at the micromolar level (MortierJ et al., Bioorganic Med Chem Lett, 2010, 20(15):4515-4520, doi:10.1016/j.bmcl.2010.06.027). In 2005, a first series of pyrazolo[4,3-c]isoquinoline derivatives have been characterized by Sanofi (WO 2004/005287). Following this first identification, in 2010, a virtual screening led to the identification of 49 hits from 67 500 scaffolds (Mortier J et al., *loc*. *cit*.). These 49 hits were selected and tested in a radiometric assay for the identification of a compound with an IC₅₀ value of 51 µM.

In 2012, Genentech reported new potent NIK inhibitors from a high-throughput screening and subsequent optimization, leading to compounds with nanomolar IC₅₀ value (De Leon-Boenig G et al., Structure, 2012, 20(10):1704-1714, doi:10.1016/j.str.2012.07.013). This first identification of NIK inhibitors via co-crystallization with the catalytic domain of NIK was the starting point of many researchers in academic groups and in the pharmaceutical industry. Thus, in 2013, Amgen reported another high-throughput screening with the identification of a new hit with an IC₅₀ value of 0.60 µM (Li K et al., Bioorganic Med Chem Lett, 2013, 23(5):1238-1244, doi:10.1016/j.bmcl.2013.01.012).

Then the first set of identified NIK inhibitors were used to develop a molecular docking-based QSAR model, which allowed the identification and evaluation of new indole-aminopyrimidine derivatives as potent inhibitors of NIK with a significative improvement of the inhibitory activity (Ye Q et al., Chem Phys Lett, 2019, 718(October 2018):38-45, doi:10.1016/j.cplett.2019.01.031).

Genentech further pursued the optimization of their NIK inhibitors, which in 2017 led to the discovery of a series of tricyclic inhibitors with the co-crystallization of the lead compound with NIK (Castanedo GM et al., J Med Chem, 2017, 60(2):627-640, doi:10.1021/acs.jmedchem.6b01363). Following the development of these compounds, in 2018, a new series of 3-hydroxypyrrolidin-2-one derivatives were identified (Blaquiere N et al., J Med Chem, 2018, 61(15):6801-6813, doi:10.1021/acs.jmedchem.8b00678). With the aim of improving metabolic stability, the researchers further identified a new 4-methoxy-2-amidopyridine compound with the highest potency and metabolic stability, also called NIK SMI1 (Blaquiere N et al., *loc. cit*.). Based on this work and the identification of the binding mode, the replacement of the 2-amidopyridine group of NIK SMI1 led to a new 2-amine-5H-pyrrolo[3,2-d]-pyrimidine structure by Zhao's group (Li Z et al., J Med Chem, 2020, 63(8):4388-4407, doi:10.1021/acs.jmedchem.0c00396).

In 2018, a screening of different Prestwick libraries led to the selection of N-acetyl-3-aminopyrazoles, and further modification increased the activity to an IC₅₀ value of 8.4 µM (Pippione AC et al., Medchemcomm, 2018, 9(6):963-968, doi:10.1039/c8md00068a).

In 2020, Chen's group reported a series of 7H-pyrrolo[2,3-d]pyrimidin-4-amines as potent NIK inhibitors (Chen Y et al., J Med Chem, 2020, 63(13):6748-6773, doi:10.1021/acs.jmedchem.0c00055). Two new co-crystal structures containing NIK co-crystallized with an indoline and aza-indoline have been reported by Janssen (Jacoby E et al., Futur Drug Discov, 2020, 2(3):FDD43, doi:10.4155/fdd-2020-0004). Genentech have pursued the optimization of their series to obtain recently an azabicyclo[3.1.0]hexanone-containing inhibitor (Crawford JJ et al., Synth, 2020, 52(22):3420-3426, doi:10.1055/s-0040-1707279).

Further notable reports on 4-anilino-quinolines as NIK inhibitors and their use in the treatment of cancer, including cutaneous melanoma, lymphomas and leukemia, include the patent applications WO 2015/032800, WO 2018/007648, WO 2019/134969, and WO 2019/134975.

Yet, despite these intense research and development efforts, none of the prior attempts to provido small molecules with the ability to specifically modulate NIK kinase activity in cancers and in inflammatory or autoimmune diseases has led to the development of any medicine approved by regulatory authorities. Therefore, there is still an urgent and unmet need for the provision of novel and improved compounds modulating NIK for use in therapy, particularly for the therapeutic intervention in cancer, inflammatory diseases, and autoimmune diseases.

The present invention addresses this need and provides novel compounds targeting NIK and having a highly advantageous therapeutic profile. In particular, the invention provides novel compounds that have been found to effectively induce the intracellular degradation of NIK. Considering the large impact of NIK on different cell functions implicated in the pathogenesis of cancers as well as inflammatory and autoimmune diseases, massively decreasing its intracellular concentration is an efficient way to shut-down NIK activities, whether in the cytosol.or at the mitochondria, and whether dependent or independent of the NF-κB2 pathway. The NIK specific degradation inducers provided in accordance with the present invention thus constitute a fundamentally novel and promising therapeutic approach. The clinically beneficial consequences of NIK degradation induced by these novel molecules are manifold and encompass inhibition of cell proliferation and induction of cell senescence without cellular toxicity. In addition, these novel inducers of NIK degradation are able to modulate the role of NIK in mitochondrial fission by inhibiting the NIK induced phosphorylation of DRP1, a major actor in this phenomenon. These effects render the compounds provided herein particularly advantageous for therapeutic use, including for the treatment of cancers, inflammatory diseases, and autoimmune diseases.

The present invention thus provides a compound of the following formula (I) or a pharmaceutically acceptable salt thereof:

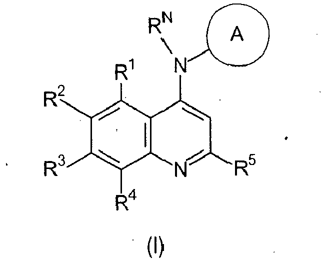

In formula (I), ring A is an aromatic group selected from the following groups (A-1), (A-2), (A-3), (A-4), (A-5), (A-6), and (A-7): 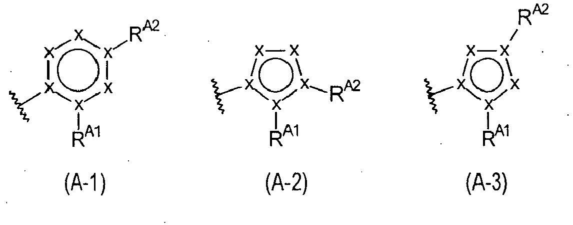 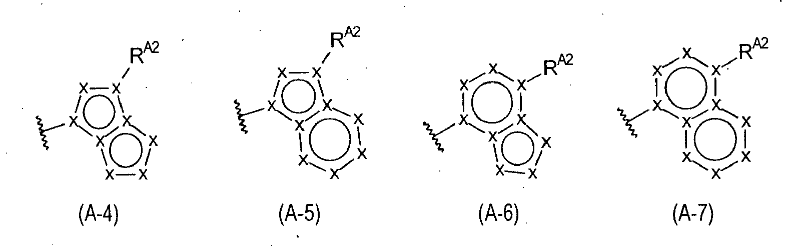
wherein the group (A-1) is phenyl or a 6-membercd monocyclic heteroaryl, wherein said phenyl or said heteroaryl is substituted with a group R^{A1} and a group R^{A2} in the indicated positions, and is optionally further substituted with one or more groups R^{A3};
wherein the groups (A-2) and (A-3) are each a 5-membered monocyclic heteroaryl which is substituted with a group R^{A1} and a group R^{A2} in the indicated positions, and is optionally further substituted with one or more groups R^{A3}; wherein the group (A-4) is an 8-membered bicyclic heteroaryl which is substituted with a group R^{A2} in the indicated position and is optionally further substituted with one or more groups R^{A3};
wherein the groups (A-5) and (A-6) are each a 9 membered bicyclic heteroaryl which is substituted with a group R^{A2} in the indicated position and is optionally further substituted with one or more groups R^{A3}; and
wherein the group (A-7) is naphthyl or a 10-membered bicyclic heteroaryl, wherein said naphthyl or said heteroaryl is substituted with a group R^{A2} in the indicated position and is optionally further substituted with one or more groups R^{A3}.

In the context of the present invention, it has surprisingly been found that the compounds of formula (I) having a ring A with the two substituents R^{A1} and R^{A2} in the specific positions indicated in formulae (A-1), (A-2) and (A-3) above, or a ring A with a second fused ring and the substituent R^{A2} in the specific positions indicated in formulae (A-4) (A-5), (A-6) and (A-7) above, exhibit advantageously improved therapeutic properties, including a considerably more potent inhibitory effect on cell proliferation and a considerably enhanced senescence-inducing activity. Thus, as summarized in the table below, the compounds of Examples 9 and 10 according to the invention have been demonstrated to exhibit improved activities in the proliferation and senescence assay described in Example 100 as compared to corresponding reference compounds having a ring A but lacking one of the substituents R^{A1} and R^{A2} in the specific positions required by the present invention:

| **Compound** | **Structure** | **Proliferation IC₅₀ (µM)** | **Senescence EC₅₀ (µM)** |
|---|---|---|---|
| Example 9 | 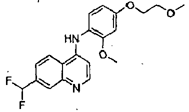 | 0.205 | 0.20 |
| Reference compound | 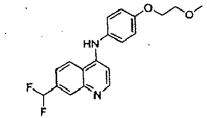 | 1.74 | 2.31 |
| Example 10 | 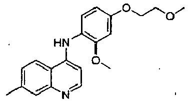 | 0.195 | 0.67 |
| Reference compound | 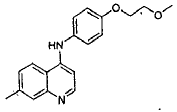 | 1.90 | 2.46 |

In formula (I), R^{A1} is selected from -O(C₁₋₅ alkyl), -O(C₂₋₅ alkenyl), C₁₋₅ alkyl, C₂₋₅ alkenyl, -N(C₁₋₅ alkyl)(C₁₋₅ alkyl), halogen, C₁₋₅ haloalkyl, -O-(C₁₋₅ haloalkyl), -CN, -(C₀₋₃ alkylene)-cycloalkyl, and -(C₀₋₃ alkylene)-heterocycloalkyl, wherein the cycloalkyl group in said -(C₀₋₃ alkylene)-cycloalkyl and the heterocycloalkyl group in said -(C₀₋₃ alkylene)-heterocycloalkyl are each optionally substituted with one or more groups R^{Cyc}, and further wherein one or more -CH₂- units comprised in the alkylene in said -(C₀₋₃ alkylene)-cycloalkyl or said -(C₀₋₃ alkylene)-heterocycloalkyl are each optionally replaced by a group independently selected from -O-, -NH-, -N(C₁₋₅ alkyl)-, -CO-, -S-, -SO-, and -SO₂-.

R^{A2} is selected from C₁₋₁₂ alkyl, -(C₁₋₁₂ alkylene)-OH, -(C₀₋₅ alkylene)-O(C₁₋₁₂ alkyl), -(C₀₋₅. alkylene)-O(C₁₋₁₂ haloalkyl), -(C₀₋₅ alkylene)-O-(C₁₋₁₂ alkylene)-OH, -(C₀₋₅ alkylene)-O-(C₁₋₅ alkylene)-O(C₁₋₁₂ alkyl), -(C₀₋₅ alkylene)-O-(C₁₋₅ alkylene)-O(C₁₋₁₂ haloalkyl), -CO(C₁₋₁₂ alkyl), -CO(C₁₋₁₂ haloalkyl), -SO₂-(C₁₋₁₂ alkyl), -SO₂-(C₁₋₁₂ haloalkyl), halogen, C₁₋₅ haloalkyl, -(C₀₋₅ alkylene)-carbocyclyl, and -(C₀₋₅ alkylene)-heterocyclyl, wherein one or more -CH₂- units in said C₁₋₁₂ alkyl, said -(C₁₋₁₂ alkylene)-OH, said -(C₀₋₅ alkylene)-O(C₁₋₁₂ alkyl), said -(C₀₋₅ alkylene)-O(C₁₋₁₂ haloalkyl), said -(C₀₋₅ alkylene)-O-(C₁₋₁₂ alkylene)-OH, said -(C₀₋₅ alkylene)-O-(C₁₋₅ alkylene)-O(C₁₋₁₂ alkyl), said -(C₀₋₅ alkylene)-O-(C₁₋₅ alkylene)-O(C₁₋₁₂ haloalkyl), said -CO(C₁₋₁₂ alkyl), said -CO(C₁₋₁₂ haloalkyl), said -SO₂-(C₁₋₁₂ alkyl) or said -SO₂-(C₁₋₁₂ haloalkyl) are each optionally replaced by a group independently selected from -O-, -NH-, -N(C₁₋₅ alkyl)-, -CO-, and -CH=CH-, wherein the carbocyclyl group in said -(C₀₋₅ alkylene)-carbocyclyl and the heterocyclyl group in said -(C₀₋₅ alkylene)-heterocyciyl are each optionally substituted with one or more groups R^{Cyc}, and further wherein one or more -CH₂- units comprised in the alkylene in said -(C₀₋₅ alkylene)-carbocyclyl or said -(C₀₋₅ alkylene)-heterocyclyl are each optionally replaced by a group independently selected from -O-, -NH-, -N(C₁₋₅ alkyl)-, -CO-, -S-, -SO-, and -SO₂-.

Each R^{A3}, if present, is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene)-heterocyclyl are each optionally substituted . with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -0-, -NH-, -N(C₁₋₅ alkyl)-, -CO-, -S-, -SO-, and -SO₂-.

R^{N} is selected from hydrogen, C₁₋₅ alkyl, and -CO-(C₁₋₅ alkyl).

R¹, R⁴ and R⁵ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-.

R² is selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene) carbocyclyl, and -(C₀₋₃ alkylene)-hetcrocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, and -SO-.

R³ is selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-CHF₂, -(C₀₋₃ alkylene)-CH₂F, -(C₀₋₃ alkylene)-(C₂₋₅ haloalkyl), -(C₀₋₃ alkylene)-O-(C₁₋₅ haloalkyl), -(C₀₋₃ alkylene)-CHO, -(C₀₋₃ alkylene)-CO-(C₁₋₅ alkyl), -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-; provided that R³ is not morpholin-4-yl or -NH-phenyl, wherein the phenyl group in said -NH-phenyl is optionally substituted with one or more groups R^{Cyc}.

Each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -(C₁₋₃ alkylene)-OH, -(C₁₋₃ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), -(C₀₋₃ alkylene)-cycloalkyl, -(C₀₋₃ alkylene)-heterocycloalkyl, and -R⁶-R⁷, wherein the cycloalkyl group in said -(C₀₋₃ alkylene)-cycloalkyl and the heterocycloalkyl group in said -(C₀₋₃ alkylene)-heterocycloalkyl are each optionally substituted with one or more groups independently selected from C₁₋₅ alkyl, C₂₋₅ alkenyl, C₂₋₅ alkynyl, halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), and -SO-(C₁₋₅ alkyl).

Each R⁶ is independently selected from a covalent bond, C₁₋₅ alkylene, C₂₋₅ alkenylene, and C₂₋₅ alkynylene, wherein said alkylene, said alkenylene and said alkynylene are each optionally substituted with one or more groups independently selected from halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-.

Each R⁷ is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl; said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).

The present invention also relates to a pharmaceutical composition comprising a compound of formula (I), or a pharmaceutically acceptable salt thereof, in combination with a pharmaceutically acceptable excipient Accordingly, the invention relates to a compound of formula (I) or a pharmaceutically acceptable salt thereof, or a pharmaceutical composition comprising any of the aforementioned entities and a pharmaceutically acceptable excipient, for use as a medicament.

The invention further relates to a compound of formula (I) or a pharmaceutically acceptable salt thereof, or a pharmaceutical composition comprising any of the aforementioned entities and a pharmaceutically acceptable excipient, for use in the treatment or prevention of cancer, an inflammatory disease, or an autoimmune disease. Thus, the invention in particular provides a pharmaceutical composition comprising, as an active ingredient, a compound of formula (I) or a pharmaceutically acceptable salt thereof, together with a pharmaceutically acceptable excipient, for use in the treatment or prevention of cancer, an inflammatory disease, or an autoimmune disease.

Moreover, the present invention relates to the use of a compound of formula. (I) or a pharmaceutically acceptable salt thereof in the preparation of a medicament for the treatment or prevention of cancer, an inflammatory disease, or an autoimmune disease.

The invention likewise relates to a method of treating or preventing cancer, an inflammatory disease, or an autoimmune disease, the method comprising administering a compound of formula (I) or a pharmaceutically acceptable salt thereof, or a pharmaceutical composition comprising any of the aforementioned entities in combination with a pharmaceutically acceptable excipient, to a subject (preferably a human) in need thereof. It will be understood that a therapeutically effective amount of the compound of formula (I) or the pharmaceutically acceptable salt thereof (or of the pharmaceutical composition) is to be administered in accordance with this method.

As explained above, the diseases/disorders to be treated or prevented with a compound of formula (I) or a pharmaceutically acceptable salt thereof (or a corresponding pharmaceutical composition) in accordance with the present invention include, in particular, cancer, inflammatory diseases, and autoimmune diseases.

The cancer to be treated or prevented may be, e.g., a solid cancer or a blood cancer (or hematological cancer). In particular, it is preferred that the cancer to be treated or prevented in accordance with the invention is selected from breast cancer (e.g., triple-negative breast cancer), prostate cancer, liver cancer (e.g., hepatocellular carcinoma), lung cancer (e.g., small cell lung cancer or non-small cell lung cancer), colon cancer, colorectal cancer, gastrointestinal cancer, pancreatic cancer, cervical cancer, ovarian cancer, kidney cancer, head cancer, neck cancer, blood cancer, Merkel carcinoma, lymphoma (e.g., Hodgkin lymphoma, non-Hodgkin lymphoma, extranodal lymphoma, or non-extranodal lymphoma), leukemia (e.g., acute lymphoblastic leukemia, acute myeloid leukemia, chronic lymphocytic leukemia, or chronic myeloid leukemia), bone cancer, bone marrow cancer, brain cancer (e.g., glioblastoma), esophagus cancer, gum cancer, nasopharynx cancer, skin cancer, melanoma, stomach cancer, tongue cancer, uterus cancer, anal cancer, genitourinary cancer, bladder cancer, endometrial cancer, vaginal cancer, vulvar cancer, testicular cancer, biliary tract cancer, hepatobiliary cancer, neuroblastoma, epidermoid cancer, squamous cell carcinoma, fibrosarcoma, Ewing's sarcoma, malignant mesothelioma, laryngeal cancer, mouth cancer, thymoma, neuroendocrine cancer, or multiple myeloma. The cancer (including any one of the above-mentioned specific types of cancer) may be, e.g., a primary cancer or a metastatic cancer.

As explained above, the cancer to be treated or prevented in accordance with the present invention may be a brood cancer (or hematological cancer), particularly lymphoma or leukemia. Preferably, the blood cancer to be treated or prevented is selected from: Hodgkin's lymphoma (or Hodgkin disease), including, e.g., nodular sclerosing subtype of Hodgkin's lymphoma, mixed-cellularity subtype of Hodgkin's lymphoma, lymphocyte-rich subtype of Hodgkin's lymphoma, lymphocyte-depleted subtype of Hodgkin's lymphoma; non-Hodgkin's lymphoma, including, e.g., follicular non-Hodgkin's lymphoma, mantle cell lymphoma, or diffuse non-Hodgkin's lymphoma (e.g., diffuse large B-cell lymphoma or Burkitt's lymphoma); nodular lymphocyte predominant Hodgkin's lymphoma; peripheral/cutaneous T-cell lymphoma, including, e.g., mycosis fungoides, Sézary's disease, T-zone lymphoma, lymphoepithelioid lymphoma (e.g., Lennert's lymphoma), or peripheral T-cell lymphoma; lymphosarcoma; a malignant immunoproliferative disease, including, e.g., Waldenström's macroglobulinaemia, alpha heavy chain disease, gamma heavy chain disease (e.g., Franklin's disease), or immunoproliferative small intestinal disease (e.g., Mediterranean disease); multiple myeloma, including, e.g., Kahler's disease, or myelomatosis; plasma cell leukemia; lymphoid leukemia, including, e.g., acute lymphoblastic leukemia, chronic lymphocytic leukemia, subacute lymphocytic leukemia, prolymphocytic leukemia, hairy-cell leukemia (e.g., leukemic reticuloendotheliosis), or adult T-cell leukemia; myeloid leukemia, including, e.g., acute myeloid leukemia, chronic myeloid leukemia, subacute myeloid leukemia, myeloid sarcoma (e.g., chloroma, or granulocytic sarcoma), acute promyelocytic leukemia, or acute myelomonocytic leukemia; chronic BCR-ABL negative myeloproliferative disorders, including, e.g., polycythaemia vera, essential thrombocythemia, or idiopathic myelofibrosis; monocytic leukemia; acute erythraemia or erythroleukemia, including, e.g., acute erythraemic myelosis, or Di Guglielmo's disease; chronic erythraemia, including, e.g., Heilmeyer-Schöner disease; acute megakaryoblastic leukemia; mast cell leukemia; acute panmyelosis; acute myelofibrosis; and Letterer-Siwe disease.

Moreover, the cancer may also be, e.g., a malignant neoplasm, carcinoma, undifferentiated carcinoma, giant and spindle cell carcinoma, small cell carcinoma, papillary carcinoma, squamous cell carcinoma, lymphoepithelial carcinoma, basal cell carcinoma, pilomatrix carcinoma, transitional cell carcinoma, papillary transitional cell carcinoma, adenocarcinoma, malignant gastrinoma, cholangiocarcinoma, hepatocellular carcinoma, combined hepatocellular carcinoma and cholangiocarcinoma, trabecular adenocarcinoma, adenoid cystic carcinoma, adenocarcinoma in adenomatous polyp, familial polyposis coli type adenocarcinoma, solid carcinoma, malignant carcinoid tumor, branchiolo-alveolar adenocarcinoma, papillary adenocarcinoma, chromophobe carcinoma, acidophil carcinoma, oxyphilic adenocarcinoma, basophil carcinoma, clear cell adenocarcinoma, granular cell carcinoma, follicular adenocarcinoma, papillary and follicular adenocarcinoma, non-encapsulating sclerosing carcinoma, adrenal cortical carcinoma, endometroid carcinoma, skin appendage carcinoma, apocrine adenocarcinoma, sebaceous adenocarcinoma, ceruminous carcinoma, adenocarcinoma, mucoepidermoid carcinoma, cystadenocarcinoma, papillary stadenocarcinoma, papillary serous cystadenocarcinoma, mucinous cystadenocarcinoma, mucinous adenocarcinoma, signet ring cell carcinoma, infiltrating duct carcinoma, medullary carcinoma, lobular carcinoma, inflammatory carcinoma, Paget's disease, acinar cell carcinoma, adenosquamous carcinoma, adenocarcinoma with squamous metaplasia, malignant thymoma, malignant ovarian stromal tumor, malignant thecoma, malignant granulosa cell tumor, malignant roblastoma, Sertoli cell carcinoma, malignant Leydig cell tumor, malignant lipid cell tumor, malignant paraganglioma, malignant extra-mammary paraganglioma, pheochromocytoma, glomangiosarcoma, malignant melanoma, amelanotic melanoma, superficial spreading melanoma, malign melanoma in giant pigmented nevus, epithelioid cell melanoma; malignant blue nevus, sarcoma, fibrosarcoma, malignant fibrous histiocytoma, myxosarcoma, liposarcoma, leiomyosarcoma, rhabdomyosarcoma, embryonal rhabdomyosarcoma, alveolar rhabdomyosarcoma, stromal sarcoma, malignant mixed tumor, mullerian mixed tumor, nephroblastoma, hepatoblastoma, carcinosarcoma, malignant mesenchymoma, malignant Brenner tumor, malignant phyllodes tumor, synovial sarcoma, malignant mesothelioma, dysgerminoma, embryonal carcinoma, malignant teratoma, malignant struma ovarii, choriocarcinoma, malignant mesonephroma, hemangiosarcoma, malignant hemangioendothelioma, Kaposi's sarcoma, malignant hemangiopericytoma, lymphangiosarcoma, osteosarcoma, juxtacortical osteosarcoma, chondrosarcoma, malignant chondroblastoma, mesenchymal chondrosarcoma, giant cell tumor of bone, Ewing's sarcoma, malignant odontogenic tumor, ameloblastic odontosarcoma, malignant ameloblastoma, ameloblastic fibrosarcoma, malignant pinealoma, chordoma, malignant glioma, ependymoma, astrocytoma, protoplasmic astrocytoma, fibrillary astrocytoma, astroblastoma, glioblastoma, oligodendroglioma, oligodendroblastoma, primitive neuroectodermal, cerebellar sarcoma, ganglioneuroblastoma, neuroblastoma, retinoblastoma, olfactory neurogenic tumor, malignant meningioma, neurofibrosarcoma, malignant neurilemmoma, malignant granular cell tumor, malignant lymphoma, natural killer (NK) leukemia, NK lymphoma (e.g., extranodal or non-extranodal natural killer/T-cell (NK/T) lymphoma), NK cell derived malignancy, acute NK leukemia, Hodgkin's disease, Hodgkin's lymphoma, paragranuloma, malignant small lymphocytic lymphoma, malignant large cell diffuse lymphoma, malignant follicular lymphoma, mycosis fungoides, non-Hodgkin's lymphoma, malignant histiocytosis, multiple myeloma, mast cell sarcoma, immunoproliferative small intestinal disease, leukemia, lymphoid leukemia, plasma cell leukemia, erythroleukemia, lymphosarcoma cell leukemia, myeloid leukemia, basophilic leukemia, eosinophilic leukemia, monocytic leukemia, mast cell leukemia, megakaryoblastic leukemia, myeloid sarcoma, or hairy cell leukemia.

Preferably, the inflammatory disease to be treated or prevented in accordance with the invention is selected from arthritis, rheumatoid arthritis, ankylosing spondylitis, osteoarthritis, psoriatic arthritis, juvenile idiopathic arthritis, juvenile rheumatoid arthritis, arthritis uratica, gout, chronic polyarthritis, periarthritis humeroscapularis, cervical arthritis, lumbosacral arthritis, enteropathic arthritis, ankylosing spondylitis, asthma, dermatitis, psoriasis, scleroderma, polymyositis, dermatomyositis, juvenila dermatomyositis, primary biliary cirrhosis, fibrosis, cystic fibrosis, pulmonary fibrosis, cirrhosis, endomyocardial fibrosis, dediastinal fibrosis, myelofibrosis, retroperitoneal fibrosis; nephrogenic fibrosis, Keloids, scleroderma, arthrofibrosis, post-transplantation late and/or chronic solid organ rejection, multiple sclerosis, systemic lupus erythematosus (SLE), lupus nephritis, pemphigus, pemphigus vulgaris, pemphigus herpetiformis, pemphigus vegetans, IgA pemphigus, pemphigus erythematosus, bullous pemphigoid, pemphigoid gestationis, mucous membrane dermatosis, pemphigoid nodularis, linear IgA bullous dermatosis, bullous lichen planus, epidermolysis bullosa acquisita, autoimmune diabetes, diabetic retinopathy, diabetic nephropathy, diabetic vasculopathy, ocular inflammation, uveitis, rhinitis, ischemiareperfusion injury, post-angioplasty restenosis, chronic obstructive pulmonary disease (COPD), glomerulonephritis, Graves disease, gastrointestinal allergy, conjunctivitis, atherosclerosis, coronary artery disease, angina, small artery disease, acute disseminated encephalomyelitis, idiopathic thrombocytopenic purpura, systemic sclerosis, antiphospholipid syndrome, Sjögren's syndrome, autoimmune hemolytic anemia, colitis, Crohn's disease, ulcerative colitis, inflammatory bowel disease (IBD), embolism, pulmonary embolism, arterial embolism, venous embolism, allergic inflammation, cardiovascular disease, graft-related disease, graft-versus-host disease (GVHD), disorder associated with graft transplantation rejection, degeneration after trauma, stroke, transplant rejection, an allergic condition, hypersensitivity, allergic rhinitis, allergic eczema, and a skin/dermal inflammatory disorder.

Morever, it is preferred that the autoimmune disease to be treated or prevented in accordance with the invention is selected from lupus (e.g., lupus erythematosus or lupus nephritis), Hashimoto's thyroiditis, Wegener's disease, primary myxedema, Graves' disease, pernicious anemia, autoimmune atrophic gastritis, Addison's disease, diabetes (e.g., insulin dependent diabetes mellitus, type I diabetes mellitus, or type II diabetes mellitus), good pasture's syndrome, myasthenia gravis, pemphigus, an intestinal inflammatory disease, Crohn's disease, ulcerative colitis, sympathetic ophthalmia, autoimmune uveitis, multiple sclerosis, autoimmune hemolytic anemia, idiopathic thrombocytopenia, primary biliary cirrhosis, chronic action hepatitis, ulcerative colitis, Sjögren's syndrome, arthritis, rheumatoid arthritis, psoriatic arthritis, ankylosing spondylitis, juvenile idiopathic arthritis, polymyositis, scleroderma, psoriasis, primary sclerosing cholangitis, asthma, transplant rejection, host-versus-graft disease, graft-versus-host disease, and mixed connective tissue disease.

Further diseases that can be treated or prevented with the compounds according to the present invention include, e.g., any of the diseases/disorders referred to in: Thu YM et al., Cytokine Growth Factor Rev, 2010, 21(4):213-226, doi:10.1016/j.cytogfr.2010.06.002; or Pflug KM et al., Int J Mol Sci, 2020, 21:8470, doi:10.3390/ijms21228470.

The present invention furthermore relates to the use of a compound of formula (I) or a pharmaceutically acceptable salt thereof as an inhibitor of NF-KB-inducing kinase (NIK) in research, i.e., as a research tool compound for inhibiting NIK. In particular, the invention relates to the use of a compound of formula (I) or a pharmaceutically acceptable salt thereof as an inducer of intracellular NIK degradation in research, i.e., as a research tool compound for inducing NIK degradation. Accordingly, the invention refers to the *in vitro* use of a compound of formula (I) or a pharmaceutically acceptable salt thereof as a NIK inhibitor and, in particular, to the *in vitro* use of a compound of formula (I) or a pharmaceutically acceptable salt thereof as a research tool compound acting as a NIK inhibitor. The invention further relates to a method (particularly an *in vitro* method) of inhibiting NIK, the method comprising applying a compound of formula (I) or a pharmaceutically acceptable salt thereof to a test sample (e.g., a biological sample) or a test animal (i.e., a non-human test animal). The present invention likewise relates to the *in vitro* use of a compound of formula (I) or a pharmaceutically acceptable salt thereof as a NIK degradation inducer and, in particular, to the *in vitro* use of a compound of formula (I) or a pharmaceutically acceptable salt thereof as a research tool compound acting as a NIK degradation inducer. Moreover, the invention relates to a method (particularly an *in vitro* method) of inducing the intracellular degradation of NIK, the method comprising applying a compound of formula (I) or a pharmaceutically acceptable salt thereof to a test sample (e.g., a biological sample) or a test animal (i.e., a non-human test animal). The terms "sample", "test sample" and "biological sample" include, without being limited thereto: a cell, a cell culture or a cellular or subcellular extract; biopsied material obtained from an animal (e.g., a human), or an extract thereof; or blood, serum, plasma, saliva, urine, feces, or any other body fluid, or an extract thereof. It is to be understood that the term "*in vitro*" is used in this specific context in the sense of "outside a living human or animal body", which includes, in particular, experiments performed with cells, cellular or subcellular extracts, and/or biological molecules in an artificial environment such as an aqueous solution or a culture medium which may be provided, e.g., in a flask, a test tube, a Petri dish, a microtiter plate, etc.

The compounds of formula (I) and the pharmaceutically acceptable salts thereof will be described in more detail in the following:

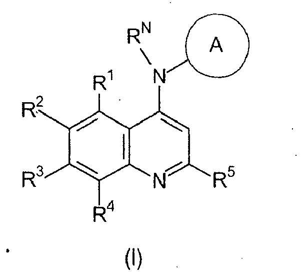

In formula (I), ring A is an aromatic group selected from the following groups (A-1), (A-2), (A-3), (A-4), (A-5), (A-6), and (A-7): 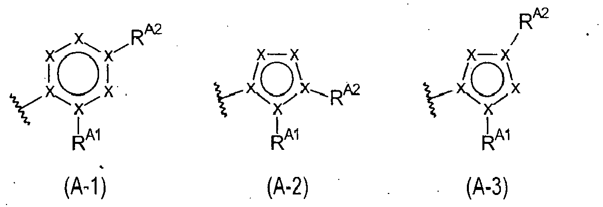 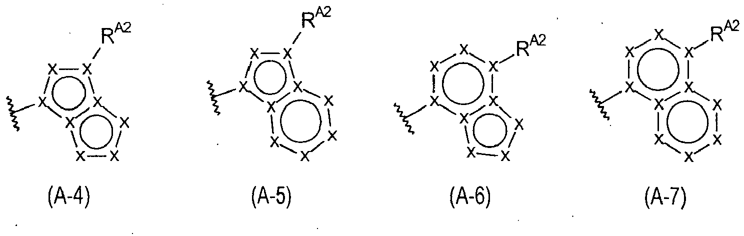
wherein the group (A-1) is phenyl or a 6-membered monocyclic heteroaryl, wherein said phenyl or said heteroaryl is substituted with a group R^{A1} and a group R^{A2} in the indicated positions, and is optionally further substituted with one or more (e.g., one or two) groups R^{A3};
wherein the groups (A-2) and (A-3) are each a 5-membered monocyclic heteroaryl which is substituted with a group R^{A1} and a group R^{A2} in the indicated positions, and is optionally further substituted with one or more groups R^{A3}; wherein the group (A-4) is an 8-membered bicyclic heteroaryl which is substituted with a group R^{A2} in the indicated position and is optionally further substituted with one or more (e.g., one, two or three) groups R^{A3};
wherein the groups (A-5) and (A-6) are each a 9-membered bicyclic heteroaryl which is substituted with a group R^{A2} in the indicated position and is optionally further substituted with one or more (e.g., one, two or three) groups R^{A3}; and
wherein the group (A-7) is naphthyl or a 10-membered bicyclic heteroaryl, wherein said naphthyl or said heteroaryl is substituted with a group R^{A2} in the indicated position and is optionally further substituted with one or more (e.g., one, two or three) groups R^{A3}.

It will be understood that the ring atoms of the groups (A-1), (A-2), (A-3), (A-4) (A-5), (A-6) and (A-7) may each be a carbon atom or a heteroatom selected from oxygen, sulfur and nitrogen, which is reflected in the above-depicted formulae of these ring groups by the symbol "x". It will further be understood that depending on the size of the monocyclic ring or the bicyclic ring system, certain ring atoms may only be selected from carbon and nitrogen, and certain ring atoms may only be carbon atoms. Moreover, it will be understood that if group (A-1) is phenyl or if group (A-7) is naphthyl, then all ring atoms are carbon atoms. As also depicted in the formulae of the groups (A-1), (A-2), (A-3), (A-4) (A-5), (A-6) and (A-7), these groups are substituted with one group R^{A1} and one group R^{A2} (in the case of A-1, A-2 and A-3) or with one group R^{A2} (in the case of A-4, A-5, A-6 and A-7) in specific positions. For example, the group (A-1), which is phenyl or a 6-membered monocyclic heteroaryl, is substituted in 2-position with a group R^{A1} and in 4-position with a group R^{A2}, wherein the 1-position refers to the attachment point of said phenyl or said heteroaryl to the remainder of the compound of formula (I) [i.e., the ring atom of group (A-1) that carries the nitrogen atom N(R^{N}) is designated as the 1-position]. Thus, in other words, if ring A is a group (A-1), then the substituent R^{A1} is attached to the group (A-1) in ortho-position and the substituent R^{A2} is attached to the group (A-1) in para-position. The group (A-2), which is a 5-membered monocyclic heteroaryl, is substituted in 2-position with a group R^{A1} and in 3-position with a group R^{A2}, wherein the 1-position refers to the attachment point of said heteroaryl to the remainder of the compound of formula (I). The group (A-3), which is a 5-membered monocyclic heteroaryl, is substituted in 2-position with a group R^{A1} and in 4-position with a group R^{A2}, wherein the 1-position refers to the attachment point of said heteroaryl to the remainder of the compound of formula (I).

If group (A-1) is a 6-membered monocyclic heteroaryl, then said heteroaryl may, for example, comprise one or more (e.g., one, two or three) nitrogen ring atoms, wherein the remaining ring atoms are carbon atoms. Accordingly, the group (A-1) may be, e.g., pyridinyl (such as, e.g., 3-R^{A1}-5-R^{A2}-pyridin-2-yl, 2-R^{A1}-6-R^{A2}-pyridin-3-yl, or 4-R^{A1}-6-R^{A2}-pyridin-3-yl), pyridazinyl (such as, e.g., 4-R^{A1}-6-R^{A2}-pyridazin-3-yl), pyrimidinyl (such as, e.g., 4-R^{A1}-2-R^{A2}-pyrimidin-5-yl), pyrazinyl (such as, e.g., 3-R^{A1}-5-R^{A2}-pyrazin-2-yl), or 1,2,4-triazinyl (such as, e.g., 5-R^{A1}-3-R^{A2}-1,2,4-triazin-6-yl), wherein each of the aforementioned groups may be optionally substituted with one or more R^{A3}.

The group (A-2) or (A-3), which is a 5-membered monocyclic heteroaryl, may comprise one more (e.g., one, two or three) heteroatoms selected independently from nitrogen, oxygen and sulfur, while the remaining ring atoms are carbon atoms. Thus, the group (A-2) may be, e.g., 1H-pyrrolyl (such as, e.g., 2-R^{A1}-3-R^{A2}-1H-pyrrol-1-yl, 1-R^{A1}-5-R^{A2}-1H-pyrrol-2-yl, 3-R^{A1}-4-R^{A2}-1H-pyrrol-2-yl, 2-R^{A1}-1-R^{A2}-1H-pyrrol-3-yl, or 4-R^{A1}-5-R^{A2}-1H-pyrrol-3-yl), furanyl (such as, e.g., 3-R^{A1}-4-R^{A2}-furan-2-yl or 4-R^{A1}-5-R^{A2}-furan-3-yl), thiophenyl (such as, e.g., 3-R^{A1}-R^{A2}-thiophen-2-yl or 4-R^{A1}-5-R^{A2}-thiophen-3-yl), pyrazolyl (such as, e.g., 5-R^{A1}-4-R^{A2}-pyrazol-1-yl, 4-R^{A1}-5-R^{A2}-pyrazol-3-yl, or 4-R^{A1}-3-R^{A2}-pyrazol-5-yl), imidazolyl (such as, e.g., 5-R^{A1}-4-R^{A2}-imidazol-1-yl, 1-R^{A1}-5-R^{A2}-imidazol-2-yl, or 1-R^{A1}-2-R^{A2}-imidazol-5-yl), 1,2,3-triazolyl (such as, e.g., 5-R^{A1}-4-R^{A2}-1,2,3-triazol-1-yl), isoxazolyl (such as, e.g., 4-R^{A1}-5-R^{A2}-isoxazol-3-yl or 4-R^{A1}-3-R^{A2}-isoxazol-5-yl), or isothiazolyl (such as, e.g., 4-R^{A1}-5-R^{A2}-isothiazol-3-yl or 4-R^{A1}-3-R^{A2}-isothiazol-5-yl), wherein each of the aforementioned groups may be optionally substituted with one or more R^{A3}. Likewise, the group (A-3) may be, e.g., 1H-pyrrolyl (such as, e.g., 2-R^{A1}-4-R^{A2}-1H-pyrrol-1-yl, 1-R^{A1}-4-R^{A2}-1H-pyrrol-2-yl, 3-R^{A1}-5-R^{A2}-1H-pyrrol-2-yl, or 4-R^{A1}-1-R^{A2}-1H-pyrrol-3-yl), furanyl (such as, e.g., 3-R^{A1}-5-R^{A2}-furan-2-yl), thiophenyl (such as, e.g., 3-R^{A1}-5-R^{A2}-thiophen-2-yl), pyrazolyl (such as, e.g., 5-R^{A1}-3-R^{A2}-pyrazol-1-yl, 4-R^{A1}-1-R^{A2}-pyrazol-3-yl, 3-R^{A1}-1-R^{A2}-pyrazol-4-yl, or 1-R^{A1}-3-R^{A2}-pyrazol-5-yl), imidazolyl (such as, e.g., 2-R^{A1}-4-R^{A2}- imidazol-1-yl, 1-R^{A1}-4-R^{A2}-imidazol-2-yl, or 4-R^{A1}-2-R^{A2}-imidazol-5-yl), 1,2,4-triazolyl (such as, e.g., 5-R^{A1}-3-R^{A2}-1,2,4-triazol-1-yl or 1-R^{A1}-3-R^{A2}-1,2,4-triazol-5-yl), oxazolyl (such as, e.g., 5-R^{A1}-2-R^{A2}-oxazol-4-yl or 4-R^{A1}-2-R^{A2}-oxazol-5-yl), or thiazolyl (such as, e.g., 5-R^{A1}-2-R^{A2}-thiazol-4-yl or 4-R^{A1}-2-R^{A2}-thiazol-5-yl), wherein each of the aforementioned groups may be optionally substituted with one or more R^{A3}.

It will be understood that in the bicyclic aromatic groups (A-4), (A-5), (A-6) and (A-7), at least one ring of the bicyclic fused ring system is aromatic; preferably, both rings of the bicyclic fused ring system are aromatic. Moreover, it will be understood that in the groups (A-4), (A-5) and (A-6), which are bicyclic heteroaryl groups, at least one ring of the bicyclic fused ring system comprises one or more (e.g., one, two or three) heteroatoms selected independently from nitrogen, oxygen and sulfur, while the remaining ring atoms are carbon atoms; in particular, both rings of the bicyclic fused ring system may each comprise one or more (e.g., one, two or three) heteroatoms selected independently from nitrogen, oxygen and sulfur, while the remaining ring atoms are carbon atoms. Likewise, if the group (A-7) is a bicyclic heteroaryl group, then at least one ring of the bicyclic fused ring system comprises one or more (e.g., one, two or three) heteroatoms selected independently from nitrogen, oxygen and sulfur, while the remaining ring atoms are carbon atoms; in particular, both rings of the bicyclic fused ring system may each comprise one or more (e.g., one, two or three) heteroatoms selected independently from nitrogen, oxygen and sulfur, while the remaining ring atoms are carbon atoms.

Thus, the group (A-4) may be, e.g., 1,4-dihydropyrrolo[3,2-b]pyrrolyl (such as, e.g., 3-R^{A2}-1,4-dihydropyrrolo[3,2-b]pyrrol-1-yl or 1-R^{A2}-1,4-dihydropyrrolo[3,2-b]pyrrol-3-yl), 1,6-dihydropyrrolo[2,3-b]pyrrolyl (such as, e.g., 3-R^{A2}-1,6-dihydropyrrolo[2,3-b]pyrrol-1-yl or 1-R^{A2}-1,6-dihydropyrrolo[2,3-b]pyrrol-3-yl), 6H-furo[2,3-b]pyrrolyl (such as, e.g., 6-R^{A2}-6H-furo[2,3-b]pyrrol-4-yl or 4-R^{A2}-6H-furo[2,3-b]pyrrol-6-yl), 4H-furo[3,2-b]pyrrolyl (such as, e.g., 6-R^{A2}-4H-furo[3,2-b]pyrrol-4-yl or 4-R^{A2}-4H-furo[3,2-b]pyrrol-6-yl), 4H-thieno[3,2-b]pyrrolyl (such as, e.g., 6-R^{A2}-4H-thieno[3,2-b]pyrrol-4-yl or 4-R^{A2}-4H-thieno[3,2-b]pyrrol-6-yl), or 6H-thieno[2,3-b]pyrrolyl (such as, e.g., 6-R^{A2}-6H-thieno[2,3-b]pyrrol-4-yl or 4-R^{A2}-6H-thieno[2,3-b]pyrrol-6-yl), wherein each of the aforementioned groups may be optionally. substituted with one or more R^{A3}.

The group (A-5) may be, e.g., 1-R^{A2}-indol-3-yl, 1-R^{A2}-1H-pyrrolo[2,3-b]pyridin-3-yl, 1-R^{A2}-1H-pyrrolo[3,2-b]pyridin-3-yl, 1-R^{A2}-1H-pyrrolo[2,3-c]pyridin-3-yl, 1-R^{A2}-1H-pyrrolo[3,2-c]pyridin-3-yl, 3-R^{A2}-indolizin-1-yl, 1-R^{A2}-indolizin-3-yl, 8-R^{A2}-pyrrolo[1,2-a]pyrimidin-6-yl, 6-R^{A2}-pyrrolo[1,2-a]pyrimidin-8-yl, 5-R^{A2}-pyrrolo[1,2-c]pyrimidin-7-yl, or 7-R^{A2}-pyrrolo[1,2-c]pyrimidin-5-yl.

The group (A-6) may be, e.g., indolizinyl (such as, e.g., 8-R^{A2}-indolizin-5-yl or 5-R^{A2}-indolizin-8-yl), imidazo[1,5-a]pyridinyl (such as, e.g., 8-R^{A2}-imidazo[1,5-a]pyridin-5-yl or 5-R^{A2}-imidazo[1,5-a]pyridin-8-yl), or benzodioxolyl (such as, e.g., 7-R^{A2}-benzodioxol-4-yl or 4-R^{A2}-benzodioxol-7-yl), wherein each of the aforementioned groups may be optionally substituted with one or more R^{A3}.

The group (A-7) may be, e.g., 8-R^{A2}-quinolin-5-yl, 5-R^{A2}-quinolin-8-yl, 8-R^{A2}-isoquinolin-5-yl, 5-R^{A2}-isoquinolin-8-yl, 4-R^{A2}-isoquinolin-1-yl, 1-R^{A2}-isoquinolin-4-yl, 8-R^{A2}-quinoxalin-5-yl, 8-R^{A2}-phthalazin-5-yl, 4-R^{A2}-phthalazin-1-yl, 8-R^{A2}-cinnolin-5-yl, 5-R^{A2}-cinnolin-8-yl, 8-R^{A2}-quinazolin-5-yl, 5-R^{A2}-quinazolin-8-yl, 5-R^{A2}-pyrido[3,4-d]pyrimidin-8-yl, 8-R^{A2}-pyrido[3,4-d]pyrimidin-5-yl, 5-R^{A2}-pyrido[4,3-d)pyrimidin-8-yl, 8-R^{A2}-pyrido[4,3-d]pyrimidin-5-yl, or 8-R^{A2}-benzodioxan-5-yl.

Furthermore, as explained above, the groups (A-1), (A-2), (A-3), (A-4) (A-5), (A-6) and (A-7) are each optionally substituted with one or more (e.g., one, two or three) groups R^{A3}. It will be understood that the maximum number of optional substituents R^{A3} depends on the number of available hydrogen atoms on the specific group (A-1), (A-2), (A-3), (A-4) (A-5), (A-6) or (A-7). For example, if the group (A-1) is phenyl, then said phenyl may carry 0, 1, 2 or 3 substituent(s) R^{A3}; however, if the group (A-1) is pyridinyl, then said pyridinyl may carry 0,1 or 2 (but not more than 2) substituent(s) R^{A3}, and if the group (A-1) is, e.g., pyrazinyl or pyridazinyl, then said pyrazinyl or said pyridazinyl may carry 0 or 1 (but not more than 1) substituent(s) R^{A3}. Unless defined otherwise, it is generally preferred that each of the groups (A-1), (A-2), (A-3), (A-4) (A-5), (A-6) and (A-7) is optionally substituted with one or two R^{A3}, more preferably with one R^{A3}, and it is even more preferred that each of said groups (A-1), (A-2), (A-3), (A-4) (A-5), (A-6) and (A-7) is not substituted with any optional substituent R^{A3}.

Preferably, ring A is a group (A-1), (A-6) or (A-7). More preferably, ring A is a group (A-1). Even more preferably, ring A is phenyl (i.e., 2-R^{A1}-4-R^{A2}-phenyl) or pyridinyl (e.g., 3-R^{A1}-5-R^{A2}-pyridin-2-yl, 2-R^{A1}-6-R^{A2}-pyridin-3-yl, or 4-R^{A1}-6-R^{A2}-pyridin-3-yl), wherein said phenyl or said pyridinyl is optionally substituted with one or more R^{A3}. Yet even more preferably, ring A is 2-R^{A1}-4-R^{A2}-phenyl which is optionally substituted with one or more (e.g., one, two or three) groups R^{A3}_{.}

The group R^{A1} is selected from -O(C₁₋₅ alkyl), -O(C₂₋₅ alkenyl), C₁₋₅ alkyl, C₂-₅ alkenyl, -N(C₁₋₅ alkyl)(C₁₋₅ alkyl), halogen, C₁₋₅ haloalkyl, -O-(C₁₋₅ haloalkyl), -CN, -(C₀₋₃ alkylene)-cycloalkyl, and -(C₀₋₃ alkylene)-heterocycloalkyl, wherein the cycloalkyl group in said -(C₀₋₃ alkylene)-cycloalkyl and the heterocycloalkyl group in said -(C₀₋₃ alkylene)-heterocycloalkyl are each optionally substituted with one or more (e.g., one, two or three) groups R^{Cyc}, and further wherein one or more (e.g., one or two) -CH₂- units comprised in the alkylene in said -(C₀₋₃ alkylene)-cycloalkyl or said -(C₀₋₃ alkylene)-heterocycloalkyl are each optionally replaced by a group independently selected from -0-, -NH-, -N(C₁₋₅ alkyl)-, -CO-, -S-, -SO-, and -SO₂-.

Preferably, R^{A1} is selected from -O(C₁₋₅ alkyl), -O(C₂₋₅ alkenyl), C₁₋₅ alkyl, C₂-₅ alkenyl, -N(C₁₋₅ alkyl)(C₁₋₅ alkyl), halogen, C₁₋₅ haloalkyl, -O-(C₁₋₅ haloalkyl), -CN, -(C₀₋₃ alkylene)-cycloalkyl, and -(C₀₋₃ alkylene)-heterocycloalkyl, wherein two -CH₂- units comprised in the alkylene in said -(C₀₋₃ alkylene)-cycloalkyl or said -(C₀₋₃ alkylene)-heterocycloalkyl are each optionally replaced by a group independently selected from -O-, -NH-, -N(C₁₋₅ alkyl)-, -CO-, -S-, -SO-, and -SO₂-. More preferably, R^{A1} is selected from -O(C₁₋₅ alkyl), -O(C₂₋₅ alkenyl), C₁₋₅ alkyl, C₂₋₅ alkenyl, -N(C₁₋₅ alkyl)(C₁₋₅ alkyl), halogen; C₁₋₅ haloalkyl, -O-(C₁₋₅ haloalkyl), -CN, cycloalkyl, -O-cycloalkyl, heterocycloalkyl, and -O-heterocycioalkyl. Even more preferably, R^{A1} is selected from -O(C₁₋₅ alkyl) (e.g., methoxy, ethoxy, n-propoxy, or isopropoxy), C₁₋₅ alkyl (e.g., methyl, ethyl, n-propyl, or isopropyl), -N(C₁₋₅ alkyl)(C₁₋₅ alkyl) (e.g., dimethylamino or diethylamino), halogen (e.g., -F, -Cl, or -Br), C₁₋₅ haloalkyl (e.g., -CF₃), -O-(C₁₋₅ haloalkyl) (e.g., - OCF₃), cycloalkyl (e.g., cyclopropyl, cyclobutyl, cyclopentyl, or cyclohexyl), -O-cycloalkyl (e.g., cyclopropoxy, cyclobutoxy, cyclopentoxy, or cyclohexoxy), and heterocycloalkyl (e.g., a monocyclic heterocycloalkyl which is attached to the remainder of the compound of formula (I) through a nitrogen ring atom; such as, e.g., pyrrolidin-1-yl, piperidin-1-yl, or morpolin-4-yl). Yet even more preferably, R^{A1} is selected from -O(C₁₋₅ alkyl), halogen, -O-(C₁₋₅ haloalkyl), and -O-cycloalkyl. Still more preferably, R^{A1} is -O(C₁₋₅ alkyl) (e.g., -OCH₃) or halogen (e.g., -F or -Br). Accordingly, particularly preferred examples of group R^{A1} include methoxy (-OCH₃), -F or -Br.

As explained further below, one or more (or all) hydrogen atoms comprised in R^{A1} (including any of the aforementioned preferred or exemplary groups R^{A1}) may also be replaced by deuterium ("D" or ²H). Accordingly, R^{A1} may be, e.g., a group -OCD₃.

The group R^{A2} is selected from C₁₁₂ alkyl, -(C₁₋₁₂ alkylene)-OH, -(C₀₋₅ alkylene)-O(C₁₋₁₂ alkyl), -(C₀₋₅ alkylene)-O(C₁₋₁₂ haloalkyl), -(C₀₋₅ alkylene)-O-(C₁₋₁₂ alkylene)-OH, -(C₀₋₅ alkylene)-O-(C₁₋₅ alkylene)-O(C₁₋₁₂ alkyl), -(C_{0.5} alkylene)-O-(C₁₋₅ alkylene)-O(C₁₋₁₂ haloalkyl), -CO(C₁₋₁₂ alkyl), -CO(C₁₋₁₂ haloalkyl), -SO₂-(C₁₋₁₂ alkyl), -SO₂-(C₁₋₁₂ haloalkyl), halogen, C₁₋₅ haloalkyl, -(C₀₋₅ alkylene)-carbocyclyl, and -(C₀₋₅ alkylene)-heterocyclyl, wherein one or more (e.g., one, two or three) -CH₂- units in said C₁₋₁₂ alkyl, said -(C₁₋₁₂ alkylene)-OH, said -(C₀₋₅ alkylene)-O(C₁₋₁₂ alkyl), said -(C₀₋₅ alkylene)-O(C₁₋₁₂ haloalkyl), said -(C₀₋₅alkylene)-O-(C₁₋₁₂ alkylene)-OH, said -(C₀₋₅ alkylene)-O-(C₁₋₅ alkylene)-O(C₁₋₁₂ alkyl), said -(C₀₋₅ alkylene)-O-(C₁₋₅ alkylene)-O(C₁₋₁₂ haloalkyl), said -CO(C₁₋₁₂ alkyl), said -CO(C₁₋₁₂ haloalkyl), said -SO₂-(C₁₋₁₂ alkyl) or said -SO₂-(C₁₋₁₂ haloalkyl) are each optionally replaced by a group independently selected from -O-, -NH-, -N(C₁₋₅ alkyl)-, -CO-, and -CH=CH-, wherein the carbocyclyl group in said -(C₀₋₅ alkylene)-carbocyclyl and the heterocyclyl group in said -(C₀₋₅ alkylene)-heterocyclyl are each optionally substituted with one or more (e.g., one, two or three) groups R^{Cyc}, and further wherein one or more (e.g., one, two or three) -CH₂- units comprised in the alkylene in said -(C₀₋₅ alkylene)-carbocyclyl or said -(C₀₋₅ alkylene)-heterocyclyl are each optionally replaced by a group independently selected from -O-, -NH-, -N(C₁₋₅ alkyl)-, -CO-, -S-, -SO-, and -SO₂-. Said C₁₋₁₂ alkyl is preferably a linear C₁₋₁₂ alkyl, such as -(CH₂)₀₋₁₁-CH₃; said -(C₁₋₁₂ alkylene)-OH is preferably a linear -(C₁₋₁₂ alkylene)-OH, such as -(CH₂)₁₋₁₂-OH; said -(C₀₋₅ alkylene)-O(C₁₋₁₂ alkyl) is preferably a linear -(C₀₋₅alkylene)-O(C₁₋₁₂ alkyl), such as -(CH₂)₀₋₅-O-(CH₂)₀₋₁₁-CH₃; said -(C₀₋₅ alkylene)-O(C₁₋₁₂ haloalkyl) is preferably a linear -(C₀₋₅ alkylene)-O(C₁₋₁₂ haloalkyl); said -(C₀₋₅ alkylene)-O-(C₁₋₁₂ alkylene)-OH is preferably a linear -(C₀₋₅ alkylene)-O-(C₁₋₁₂ alkylene)-OH, such as -(CH₂)₀₋₅-O-(CH₂)₁₋₁₂-OH; said -(C₀₋₅ alkylene)-O-(C₁₋₅ alkylene)-O(C₁₋₁₂ alkyl) is preferably a linear -(C₀₋₅ alkylene)-O-(C₁₋₅ alkylene)-O(C₁₋₁₂ alkyl), such as -(CH₂)₀₋₅-O-(CH₂)₁₋₅-O-(CH₂)₀₋₁₁-CH₃; said -(C₀₋₅alkylene)-O-(C₁₋₅ alkylene)-O(C₁₋₁₂ haloalkyl) is preferably a linear -(C₀₋₅ alkylene)-O-(C₁₋₅ alkylene)-O(C₁₋₁₂ haloalkyl); said -CO(C₁₋₁₂ alkyl) is preferably a linear -CO(C₁₋₁₂ alkyl), such as -CO-(CH₂)₀₋₁₁-CH₃; said -CO(C₁₋₁₂ haloalkyl) is preferably a linear-CO(C₁₋₁₂ haloalkyl); said -SO₂-(C₁₋₁₂ alkyl) is preferably a linear -SO₂(C₁₋₁₂ alkyl), such as -SO₂-(CH₂)₀₋₁₁-CH₃; said -SO₂-(C₁₋₁₂ haloalkyl) is preferably a linear -SO₂-(C₁₋₁₂ haloalkyl).

Preferably, R^{A2} is selected from C₁₋₅ alkyl, -(C₁₋₅ alkylene)-OH, -O(C₁₋₅ alkyl) (e.g., -OCH₃), -O-(C₁₋₅ alkylene)-OH, -0-(C₁₋₅ alkylene)-O(C₁₋₅ alkyl), -(C₁₋₅ alkylene)-O-(C₁₋₅ alkylene)-OH, -(C₁₋₅ alkylene)-O-(C₁₋₅ alkylene)-O(C₁₋₅ alkyl), -O(C₁₋₅ alkylene)-O(C₁₋₅ alkyl) (e.g., -OCH₂CH₂OCH₃ or -OCH₂CH₂OCH(-CH₃)CH₃), -O(C₁₋₃ alkylene)-O(C₁₋₃ alkylene)-O(C₁₋₃ alkyl), -O(C₂₋₅ alkenyl) (e.g., -OCH=CH₂), -(C₁₋₅ alkylene)-O(C₁₋₅ alkyl), -(C₁₋₅ alkylene)-O(C₁₋₅ alkylene)-O(C₁₋₅ alkyl), -(C₁₋₃ alkylene)-O(C₁₋₃ alkylene)-O(C₁₋₃ alkylene)-O(C₁₋₃ alkyl), -O-(C₁₋₅ haloalkyl) (e.g., -OCF₃), -O(C₁₋₅ alkylene)-O-(C₁₋₅ haloalkyl) (e.g., -OCH₂CH₂OCF₃ or -OCH₂CH₂OCF₂H), -O(C₁₋₃ alkylene)-O(C₁₋₃ alkylene)-O-(C₁₋₃ haloalkyl), -(C₁₋₅ alkylene)-O-(C₁₋₅ haloalkyl), -(C₁₋₅ alkylene)-O(C₁₋₅ alkylene)-O-(C₁₋₅ haloalkyl), -(C₁₋₃ alkylene)-O(C₁₋₅ alkylene)-O(C₁₋₃ alkylene)-O-(C₁₋₃ haloalkyl), -O(C₁₋₅ alkylene)-N(C₁₋₅ alkyl)(C₁₋₅ alkyl) (e.g., -OCH₂CH₂N(CH₃)₂), -(C₁₋₅ alkylene)-O(C₁₋₅ alkylene)-N(C₁₋₅ alkyl)(C₁₋₅ alkyl), -CO-NH-(C₁₋₅ alkyl) (e.g., -CO-NH-CH(CH₃)-CH₃), -CO-N(C₁₋₅ alkyl)(C₁₋₅ alkyl), -SO₂-(C₁₋₁₂ alkyl) (e.g., -SO₂₋CH₃), -SO₂-(C₁₋₁₂ haloalkyl), halogen (e.g., -F, -Cl, or -Br), C₁₋₅ haloalkyl (e.g., -CF₃), -(C₀₋₅ alkylene)-carbocyclyl (e.g., -(C₀₋₅ alkylene)-cycloalkyl or -(C₀₋₅ alkylene)-aryl), -(C₀₋₅ alkylene)-heterocyclyl (e.g., -(C₀₋₅ alkylene)-heteroaryl or -(C₀₋₅ alkylene)-heterocycloalkyl; such as pyrazolyl [e.g., pyrazol-1-yl, pyrazol-3-yl, pyrazol-4-yl, or pyrazol-5-yl], imidazolyl [e.g., imidazol-1-yl, imidazol-2-yl, imidazol-4-yl, or imidazol-5-yl], isoxazolyl [e.g., isoxazol-3-yl, isoxazol-4-yl, or isoxazol-5-yl], 1,2,4-triazolyl [e.g., 1,2,4-triazol-1-yl, 1,2,4-triazol-3-yl, or 1,2,4-triazol-5-yl], 1H-pyrrolyl [e.g., 1H-pyrrol-1-yl, 1H-pyrrol-2-yl, or 1H-pyrrol-3-yl], furanyl [e.g., furan-2-yl or furan-3-yl], thiophenyl [e.g., thiophen-2-yl orthiophen-3-yl], pyridinyl [e.g., pyridin-2-yl, pyridin-3-yl, or pyridin-4-yl], or pyrimidinyl [e.g., pyrimidin-2-yl, pyrimidin-4-yl, or pyrimidin-5-yl]), -O-(C₀₋₄ alkylene)-carbocyclyl (e.g., -O-(C₀₋₄ alkylene)-aryl or -O-(C₀₋₄ alkylene)-cycloalkyl; such as -O-cyclopentyl), -O(C₀₋₄ alkylene)-heterocyclyl (e.g., -O-(C₀₋₄ alkylene)-heteroaryl or -O-(C₀₋₄ alkylene)-heterocycloalkyl; such as -OCH₂CH₂-(morpholin-4-yl) or -OCH₂CH₂-(6-oxa-3-aza-bicyclo[2.1.2]heptan-3-yl)), -(C₀₋₄ alkylene)-O-carbocyclyl (e.g., -(C₀₋₄ alkylene)-O-aryl or -(C₀₋₄ alkylene)-O-cycloalkyl), -(C₀₋₄ alkylene)-O-heterocyclyl (e.g., -(C₀₋₄ alkylene)-O-heteroaryl or -(C₀₋₄ alkylene)-O-heterocycloalkyl), -CO-(C₀₋₄ alkylene)-carbocyclyl (e.g., -CO-(C₀₋₄ alkylene)-aryl or -CO-(C₀₋₄ alkylene)-cycloalkyl), and -CO-(C₀₋₄ alkylene)-heterocyclyl (e.g., -CO-(C₀₋₄ alkylene)-heteroaryl or -CO-(C₀₋₄ alkylene)-heterocycloalkyl; such as -CO-(4-morpholinyl)), wherein the carbocyclyl in said -(C₀₋₅ alkylene)-carbocyclyl, the heterocyclyl in said -(C₀₋₅ alkylene)-heterocyclyl, the carbocyclyl in said -O-(C₀₋₄ alkylene)-carbocyclyl, the heterocyclyl in said -O-(C₀₋₄ alkylene)-heterocyclyl, the carbocyclyl in said -(C₀₋₄ alkylene)-O-carbocyclyl, the heterocyclyl in said -(C₀₋₄ alkylene)-O-heterocyclyl, the carbocyclyl in said -CO-(C₀₋₄ alkylene)-carbocyclyl, and the heterocyclyl in said -CO-(C₀₋₄ alkylene)-heterocyclyl are each optionally substituted with one or more (e.g., one, two or three) groups R^{Cyc}. More preferably, R^{A2} is selected from C₁₋₅ alkyl, -(C₁₋₅ alkylene)-OH, -O(C₁₋₅ alkyl), -O(C₁₋₅ alkylene)-O(C₁₋₅ alkyl), -O(C₁₋₃ alkylene)-O(C₁₋₃ alkylene)-O(C₁₋₃ alkyl), -O(C₂₋₅ alkenyl), -(C₁₋₅ alkylene)-O(C₁₋₅ alkyl), -(C₁₋₅ alkylene)-O(C₁₋₅ alkylene)-O(C₁₋₅ alkyl), -(C₁₋₃ alkylene)-O(C₁₋₃ alkylene)-O(C₁₋₃ alkylene)-O(C₁₋₃ alkyl), -O-(C₁₋₅ haloalkyl), -O(C₁₋₅ alkylene)-O-(C₁₋₅ haloalkyl), -O(C₁₋₃ alkylene)-O(C₁₋₃ alkylene)-O-(C₁₋₃ haloalkyl), -(C₁₋₅ alkylene)-O-(C₁₋₅ haloalkyl), -(C₁₋₅ alkylene)-O(C₁₋₅ alkylene)-O-(C₁₋₅ haloalkyl), -(C₁₋₃ alkylene)-O(C₁₋₅ alkylene)-O(C₁₋₃ alkylene)-O-(C₁₋₃ haloalkyl), -O(C₁₋₅ alkylene)-N(C₁₋₅ alkyl)(C₁₋₅ alkyl), -(C₁₋₅ alkylene)-O(C₁₋₅ alkylene)-N(C₁₋₅ alkyl)(C₁₋₅ alkyl), -CO-NH-(C₁₋₅ alkyl), -CO-N(C₁₋₅ alkyl)(C₁₋₅ alkyl), -SO₂-(C₁₋₁₂ alkyl), -SO₂-(C₁₋₁₂ haloalkyl), halogen, C₁₋₅ haloalkyl, aryl (e.g., phenyl), and heteroaryl (e.g., a monocyclic heteroaryl, particularly a 5-membered or 6-membered monocyclic heteroaryl, such as, e.g., pyrazolyl), wherein said aryl and said heteroaryl are each optionally substituted with one or more (e.g., one, two or three) groups R^{Cyc}. Even more preferably, R^{A2} is selected from -O(C₁₋₅ alkyl), -O(C₁₋₅ alkylene)-O(C₁₋₅ alkyl), -O(C₁₋₃ alkylene)-O(C₁₋₃ alkylene)-O(C₁₋₃ alkyl), -(C₁₋₅ alkylene)-O(C₁₋₅ alkyl), -(C₁₋₅ alkylene)-O(C₁₋₅ alkylene)-O(C₁₋₅ alkyl), -(C₁₋₃ alkylene)-O(C₁₋₃ alkylene)-O(C₁₋₃ alkylene)-O(C₁₋₅ alkyl), -SO₂(C₁₋₁₂ alkyl), halogen, C₁₋₅ haloalkyl, phenyl, and heteroaryl, wherein said phenyl and said heteroaryl are each optionally substituted with one or more groups R^{Cyc}. Yet even more preferably, R^{A2} is selected from -O(C₁₋₅ alkyl), -O(C₁₋₅ alkylene)-O(C₁₋₅ alkyl), -(C₁₋₅ alkylene)-O(C₁₋₅ alkyl), -(C₁₋₅ alkylene)-O(C₁₋₅ alkylene)-O(C₁₋₅ alkyl), and monocyclic heteroaryl, wherein said monocyclic heteroaryl is optionally substituted with one or more groups R^{Cyc}. Accordingly, particularly preferred examples of R^{A2} include -OCH₂CH₂OCH₃, -OCH₂CH₂OCF₃, or pyrazolyl (e.g., pyrazol-1-yl, pyrazol-3-yl, pyrazol-4-yl, or pyrazol-5-yl).

Each R^{A3} (if present) is independently selected from C₁₋₅ alkyl, C₂-₅ alkenyl, C₂₋₅ alkynyl, -(C₀₋₃ alkylene)-OH, -(C₀₋₅ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C_{0.3} alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene) CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene) CO-NH₂, (C₀₋₃ alkylene)-CO-NH(C₁₋₅ alkyl), -(C_{0.3} alkylene)-CO-N(C₁₋₅ alkyl)(C₁₋₅ alkyl), -(C_{0.3} alkylene)-NH-CO-(C₁₋₅ alkyl), -(C₀₋₃ alkylene)-N(C₁₋₅ alkyl)-CO-(C₁₋₅, alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), (C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂(C₁₋₅ alkyl), -(C₀₋₃ alkylene)-SO-(C₁₋₅ alkyl), -(C₀₋₃ alkylene)-SO₂(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene) heterocyclyl arc each optionally substituted with one or more (e.g., one, two or three) groups R^{Cyc}, and further wherein one or more (e.g., one or two) -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-.

Preferably, each R^{A3} (if present) is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -(C₁₋₃ alkylene)-OH, -(C₁₋₃ alkylene)-O(C₁₋₅ alkyl), -(C₁₋₃ alkylene)-O(C₁₋₅ alkylene)-OH, -(C₁₋₃ alkylene)-O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl),. -SO₂-NH₂, -SO₂NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C_{0.3} alkylene)-carbocyclyl and the heterocyclyl group in said -(C₀₋₃ alkylene)-heterocyclyl are each optionally substituted with one or more (i.e., one, two or three) groups R^{Cyc}, and further wherein one or more (e.g., one or two) -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂- . More preferably, each R^{A3} (if present) is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -(C₁₋₃ alkylene)-OH, -(C₁₋₃ alkylene)-O(C₁₋₅ alkyl), -(C₁₋₃ alkylene)-O(C₁₋₅ alkylene)-OH, -(C₁₋₃ alkylene)-O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), and -CN. Even more preferably, each R^{A3} (if present) is independently selected from C₁₋₅ alkyl (e.g., methyl), -OH, -O(C₁₋₅ alkyl) (e.g., -OCH₃ or -OCH₂CH₃), -O(C₁₋₅ alkylene)-OH (e.g., -O-(CH₂)₂-OH or -O-(CH₂)₃-OH), -O(C₁₋₅ alkylene)-O(C₁₋₅ alkyl) (e.g., -O-(CH₂)₂-O-CH₃, -O-(CH₂)₂-O-CH₂CH₃, -O-(CH₂)₃-O-CH₃, or -O-(CH₂)₃-O-CH₂CH₃), -(C₁₋₃ alkylene)-OH, -(C₁₋₃ alkylene)-O(C₁₋₅ alkyl) (e.g., -CH₂-O-CH₃), -(C₁₋₃ alkylene)-O(C₁₋₅ alkylene)-OH, -(C₁₋₃ alkylene)-O(C₁₋₅ alkylene)-O(C₁₋₅ alkyl), halogen (e.g., -F or -Cl), C₁₋₅ haloalkyl (e.g., -CF₃), and -O-(C₁₋₅ haloalkyl) (e.g., -OCF₃).

R^{N} is selected from hydrogen, C₁₋₅ alkyl, and -CO-(C₁₋₅ alkyl).

Preferably, R^{N} is hydrogen or C₁₋₅ alkyl. More preferably, R^{N} is hydrogen, methyl or ethyl. Even more preferably, R^{N} is hydrogen.

R¹, R⁴ and R⁵ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C_{0.3} alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C_{0.3} alkylene)-halogen, -(C₀₋₃ alkylene)-(C₁₋₅ haloalkyl), -(C_{0.3} alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C_{0.3} alkylene)-COOH, -(C₀₋₃ alkylene)-CO-O-(C₁₋₅ alkyl), -(C₀₋₃ alkylene)O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C_{0.3} alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C_{0.3} alkylene)-N(C₁₋₅ alkyl)-COO(C₁₋₅ alkyl), -(C₀₋₃ alkylene)-O-CO-NH(C₁₋₅ alkyl), -(C_{0.3} alkylene)-O-CO-N(C₁₋₅ alkyl)(C₁₋₅ alkyl), -(C₀₋₃ alkylene)-SO₂-NH₂, -(C_{0.3} alkylene)-SO₂NH(C₁₋₅ alkyl), -(C₀₋₃ alkylene)-SO₂-N(C₁₋₅ alkyl)(C₁₋₅ alkyl), -(C₀₋₃ alkylene)-NH-SO₂(C₁₋₅ alkyl), -(C₀₋₃ alkylene)-N(C₁₋₅ alkyl)-SO₂₋(C₁₋₅ alkyl), -(C_{0.3} alkylene)-SO-(C₁₋₅ alkyl), -(C₀₋₃ alkylene)-SO₂-(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene)-heterocyclyl are each optionally substituted with one or more (e.g., one, two or three) groups R^{Cyc}, and further wherein one or more (e.g., one or two) -CH₂- units comprised in the alkylene in said -(C_{0.3} alkylene)-carbocyclyl or said -(C₀₋₃ alkylene)-heterocyclyl are each optionally replaced: by a group independently selected from -O-, -NH-, -N(C₁₋₅ alkyl)-, -CO-, -S-, -SO-, and -SO₂-.

Preferably, R¹, R⁴ and R⁵ are each independently selected from hydrogen, C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO-(C₁₋₅ alkyl), -COOH, -CO-O-(C₁₋₅ alkyl), -O-CO-(C₁₋₅ alkyl), .-CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alky))(C₁₋₅ alkyl), -NH-SO₂-(C₁₋₅ alkyl), -N(C₁₋₅ alkyl)-SO₂(C₁₋₅ alkyl), -SO-(C₁₋₅ alkyl), -SO₂-(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene)-heterocyclyl are each optionally substituted with one or more (i.e., one, two or three) groups R^{Cyc}, and further wherein one or more (e.g., one or two) -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂- . More preferably, R¹, R⁴ and R⁵ are each independently selected from hydrogen, C₁₋₅ alkyl, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), and -CN. It is particularly preferred that R¹, R⁴ and R⁵ are each hydrogen.

R² is selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH; -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C_{0.3} alkylene)-CHO, -(C_{0.3} alkylene)-CO-(C₁₋₅ alkyl), -(C_{0.3} alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂₋N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C_{0.3} alkylene)-carbocyclyl, and -(C₀₋₃ alkylene)-heterocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more (e.g., one, two or three) groups R^{Cyc}, and further wherein one or more (e.g., one or two) -CH₂₋ units comprised in the alkylene in said -(C₀₋₃ alkylene)-carbocyclyl or said -(C₀₋₃ alkylene)-heterocyclyl are each optionally replaced by a group independently selected from -O-, -NH- , -N(C₁₋₅ alkyl)-, -CO-, -S-, and -SO-.

Preferably, R² is selected from hydrogen, C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂(C₁₋₅ alkyl), -SO-(C₁₋₅ alkyl), -(C₀₋₃ alkylene)-carbocyclyl, and -(C₀₋₃ alkylene)-heterocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more (i.e., one, two or three) groups R^{Cyc}, and further wherein one or more (e.g., one or two) -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, and -SO-. More preferably, R² is selected from hydrogen, C₁₋₅ alkyl, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), and -CN. It is particularly preferred that R² is hydrogen.

R³ is selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-CHF₂, -(C₀₋₃ alkylene)-CH₂F, -(C_{0.3} alkylene)-(C₂₋₅ haloalkyl), -(C₀₋₃ alkylene)-O-(C₁₋₅ haloalkyl), -(C₀₋₃ alkylene)-CHO, -(C₀₋₃ alkylene)-CO-(C₁₋₅ alkyl), -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C_{0.3} alkylene)-CO-NH(C₁₋₅ alkyl), -(C₀₋₃ alkylene)-CO-N(C₁₋₅ alkyl)(C₁₋₅ alkyl), -(C_{0.3} alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C_{0.3} alkylene)-SO₂NH₂, -(C₀₋₃ alkylene)-SO₂NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂(C₁₋₅ alkyl), -(C₀₋₃ alkylene)-carbocyclyl, and -(C₀₋₃ alkylene)-heterocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more (e.g., one, two or three) groups R^{Cyc}, and further wherein one or more (e.g., one or two) -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-; provided that R³ is not morpholin-4-yl or -NH-phenyl, wherein the phenyl group in said - NH-phenyl is optionally substituted with one or more groups R^{Cyc}.

Preferably, R³ is selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), -CHF₂, -CH₂F, C₂₋₅ haloalkyl, -O-(C₁₋₅ haloalkyl), -CHO, -CO-(C₁₋₅ alkyl), -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), -(C₀₋₃ alkylene)-carbocyclyl (e.g., -(C₀₋₃ alkylene)-aryl or -(C₀₋₃ alkylene)-cycloalkyl; such as -(C₀₋₃ alkylene)-phenyl), and -(C₀₋₃ alkylene)-heterocyclyl (e.g., -(C₀₋₃ alkylene)-heteroaryl, -(C₀₋₃ alkylene)-heterocycloalkenyl, or -(C₀₋₃ alkylene)-heterocycloalkyl; such as azetidinyl, pyrrolidinyl, piperidinyl, piperazinyl, 2,3,5,6,7-pentahydro-1,4-diazepinyl, 1,2,3,6-tetrahydropyridinyl, pyrazolyl, thiazolyl, pyridinyl, pyrazinyl, or spiro[azetidine-3,3'-azetidin]-1-yl), wherein the carbocyclyl group in said -(C₀₋₃ alkylene)-carbocyclyl and the heterocyclyl group in said -(C₀₋₃ alkylene)-heterocyclyl are each optionally substituted with one or more (i.e., one, two or three) groups R^{Cyc}, and further wherein one or more (e.g., one or two) -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-; provided that R³ is not morpholin-4-yl or -NH-phenyl, wherein the phenyl group in said -NH-phenyl is optionally substituted with one or more groups R^{Cyc}. More preferably, R³ is selected from C₁₋₅ alkyl, C₂₋₅ alkenyl, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CHF₂, -CH₂F, C₂₋₅ haloalkyl, -O-(C₁₋₅ haloalkyl), phenyl, cycloalkyl, cycloalkenyl, heteroaryl, heterocycloalkyl, and heterocycloalkenyl, wherein said phenyl, said cycloalkyl, said cycloalkenyl, said heteroaryl, said heterocycloalkyl and said heterocycloalkenyl are each optionally substituted with one or more (e.g., one, two or three) groups R^{Cyc}; provided that R³ is not morpholin-4-yl. Even more preferably, R3 is selected from C₁₋₅ alkyl (e.g., methyl), -O(C₁₋₅ alkyl) (e.g., methoxy), -CHF₂, -CH₂F, C₂₋₅ haloalkyl, -O-(C₁₋₅ haloalkyl), phenyl, cycloalkyl, heteroaryl, and heterocycloalkyl, wherein said phenyl, said cycloalkyl, said heteroaryl and said heterocycloalkyl are each optionally substituted with one or more (e.g., one, two or three) groups R^{Cyc}; provided that R³ is not morpholin-4-yl. Particularly preferred examples of R³ include methyl or -CHF₂.

Each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -(C₁₋₃ alkylene)-OH, -(C₁₋₃ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl); -N(C₁₋₅ alkyl)-O(C₁₋₅ alkyl), halogen, C₁₋₅ haloalkyl, -O-(C₁₋₅ haloalkyl), -CN, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SC₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂(C₁₋₅ alkyl), -SO₂-(C₁₋₅ alkyl), -SO-(C₁₋₅ alkyl), -(C₀₋₃ alkylene)-cycloalkyl, -(C₀₋₃ alkylene)-heterocycloalkyl, and -R⁶-R⁷, wherein the cycloalkyl group in said -(C₀₋₃ alkylene)-cycloalkyl and the heterocycloalkyl group in said -(C₀₋₃ alkylene)-heterocycloalkyl are each optionally substituted with one or more (e.g., one, two or three) groups independently selected from C₁₋₅ alkyl, C₂₋₅ alkenyl, C₂₋₅ alkynyl, halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂(C₁₋₅ alkyl), -SO₂-(C₁₋₅ alkyl), and -SO-(C₁₋₅ alkyl).

Each R⁶ is independently selected from a covalent bond, C₁₋₅ alkylene, C₂₋₅ alkenylene, and C₂₋₅ alkynylene, wherein said alkylene, said alkenylene and said alkynylene are each optionally substituted with one or more (e.g., one, two or three) groups independently selected from halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more (e.g., one, two or three) -CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-.

Each R⁷ is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more (e.g., one, two or three) groups independently selected from C₁₋₅ alkyl, C₂₋₅ alkenyl, C₂₋₅ alkynyl, halogen, C₁₋₅ haloalkyl, -O-(C₁₋₅ haloalkyl), -CN, -OH, -O(C₁₋₅ alkyl), -SH, -S(C₁₋₅ alkyl), -NH₂; -NH(C₁₋₅ alkyl), and -N(C₁₋₅ alkyl)(C₁₋₅ alkyl).

It is particularly preferred that the compound of formula (I) is any one of the specific compounds of formula (I) described in the examples section of this specification, including any one of Examples 1 to 99 described further below, either in non-salt form or as a pharmaceutically acceptable salt of the respective compound.

Accordingly, it is particularly preferred that the compound of formula (I) is any one of the following compounds or a pharmaceutically acceptable salt thereof:
N-(2-methoxy-4-(2-methoxyethoxy)phenyl)-7-phenylquinolin-4-amine;
N-(2-methoxy-4-(2-methoxyethoxy)phenyl)-7-(pyrazin-2-yl)quinolin-4-amine;
7-methoxy-N-(2-methoxy-4-(2-methoxyethoxy)phenyl)quinolin-4-amine;
N-(2-methoxy-4-(2-methoxyethoxy)phenyl)-7-(1-methyl-1H-pyrazol-4-yl)quinolin-4-amine;
N-(2-methoxy-4-(2-methoxyethoxy)phenyl)-7-(1-methyl-1,2,3,6-tetrahydropyridin-4-yl)quinolin-4-amine;
N-(2-methoxy-4-(2-methoxyethoxy)phenyl)-7-(4-methylpiperazin-1-yl)quinolin-4-amine;
7-(4,4-difluoropiperidin-1-yl)-N-(2-methoxy-4-(2-methoxyethoxy)phenyl)quinolin-4-amine;
7-(3,3-difluoroazetidin-1-yl)-N-(2-methoxy-4-(2-methoxyethoxy)phenyl)quinolin-4-amine;
7-(difluoromethyl)-N-(2-methoxy-4-(2-methoxyethoxy)phenyl)quinolin-4-amine;
N-(2-methoxy-4-(2-methoxyethoxy)phenyl)-7-methylquinolin-4-amine;
N-(2-methoxy-4-(2-methoxyethoxy)phenyl)-7-(1-methylpiperidin-4-yl)quinolin-4-amine;
N-(2-methoxy-4-(2-methoxyethoxy)phenyl)-7-(prop-1-en-2-yl)quinolin-4-amine;
7-isopropyl-N-(2-methoxy-4-(2-methoxyethoxy)phenyl)quinolin-4-amine;
N-(2-methoxy-4-(2-methoxyethoxy)phenyl)-7-(pyridin-2-yl)quinolin-4-amine;
N-(2-methoxy-4-(2-methoxyethoxy)phenyl)-7-(thiazol-4-yl)quinolin-4-amine;
N-(2-methoxy-4-(2-methoxyethoxy)phenyl)-7-(4-(oxetan-3-yl)piperazin-1-yl)quinolin-4-amine;
(S)-7-(3,4-dimethylpiperazin-1-yl)-N-(2-methoxy-4-(2-methoxyethoxy)phenyl)quinolin-4-amine;
(R)-7-(3,4-dimethylpiperazin-1-yl)-N-(2-methoxy-4-(2-methoxyethoxy)phenyl)quinolin-4-amine;
N-(2-methoxy-4-(2-methoxyethoxy)phenyl)-7-(4-methyl-1,4-diazepan-1-yl)quinolin-4-amine;
N-(2-methoxy-4-(2-methoxyethoxy)phenyl)-7-(4-(2-methoxyethyl)piperazin-1-yl)quinolin-4-amine;
N-(2-methoxy-4-(2-methoxyethoxy)phenyl)-7-(6-methyl-2,6-diazaspiro[3.3]heptan-2-yl)quinolin-4-amine;
7-(4-ethylpiperazin-1-yl)-N-(2-methoxy-4-(2-methoxyethoxy)phenyl)quinolin-4-amine;
(S)-7-(2,4-dimethylpiperazin-1-yl)-N-(2-methoxy-4-(2-methoxyethoxy)phenyl)quinolin-4-amine;
tert-butyl 3-(4-(4-((2-methoxy-4-(2-methoxyethoxy)phenyl)amino)quinolin-7-yl)piperazin-1-yl)azetidine-1-carboxylate;
N-(2-methoxy-4-(2-methoxyethoxy)phenyl)-7-(piperazin-1-yl)quinolin-4-amine;
N-(2-methoxy-4-(2-methoxyethoxy)phenyl)-7-(4-(1-methylazetidin-3-yl)piperazin-1-yl)quinolin-4-amine;
N-(2-methoxy-4-(2-methoxyethoxy)phenyl)-7-(4-(2,2,2-trifluoroethyl)piperazin-1-yl)quinolin-4-amine;
7-(4-(azetidin-3-yl)piperazin-1-yl)-N-(2-methoxy-4-(2-methoxyethoxy)phenyl)quinolin-4-amine;
(R)-(4-(4-((2-methoxy-4-(2-methoxyethoxy)phenyl)amino)quinolin-7-yl)-1-methylpiperazin-2-yl)methanol;
(S)-(4-(4-((2-methoxy-4-(2-methoxyethoxy)phenyl)amino)quinolin-7-yl)-1-methylpiperazin-2-yl)methanol;
tert-butyl 5-(4-((2-methoxy-4-(2-methoxyethoxy)phenyl)amino)quinolin-7-yl)-2,5-diazabicyclo[2.2.1]heptane-2-carboxylate;
N-(2-methoxy-4-(2-methoxyethoxy)pheny))-7-(5-methy)-2,5-diazabicyclo[2.2.1]heptan-2-yl)quinolin-4-amine;
N-(2-methoxy-4-(2-methoxyethoxy)phenyl)-7-(6-methyl-3,6-diazabicyclo[3.1.1]heptan-3-yl)quinolin-4-amine;
4-(4-((2-methoxy-4-(2-methoxyethoxy)phenyl)amino)quinolin-7-yl)piperazin-2-one;
N-(2-methoxy-4-(2-(trifluoromethoxy)ethoxy)phenyl)-7-(1-methyl-1)2,3,6-tetrahydropyridin-4-yl)quinolin-4-amine;
N-(2-methoxy4-(2-(trifluoromethoxy)ethoxy)phenyl)-7-(4-methylpiperazin-1-yl)quinolin-4-amine;
N-(2-methoxy-4-(2-(trifluoromethoxy)ethoxy)phenyl)-7-(1-methylpiperidin-4-yl)quinolin-4-amine;
N-(2-methoxy-4-(2-(trifluoromethoxy)ethoxy)phenyl)-7-(1-methyl-1H-pyrazol-4-yl)quinolin-4-amine;
N-(2-methoxy-4-(2-(trifluoromethoxy)ethoxy)phenyl)-7-methylquinolin-4-amine;
7-methoxy-N-(2-methoxy-4-(2-(trifluoromethoxy)ethoxy)phenyl)quinolin-4-amine;
N-(2-methoxy-4-(2-(trifluoromethoxy)ethoxy)phenyl)-7-(pyrazin-2-yl)quinolin-4-amine;
N-(2-methoxy-4-(2-(trifluoromethoxy)ethoxy)phenyl)-7-(pyridin-2-yl)quinolin-4-amine;
N-(2-methoxy-4-(2-(trifluoromethoxy)ethoxy)phenyl)-7-(thiazol-4-yl)quinolin-4-amine;
7-(difluoromethyl)-N-(2-methoxy4-(2-(trifluoromethoxy)ethoxy)phenyl)quinolin-4-amine;
N-(2,4-dimethoxyphenyl)-7-phenylquinolin-4-amine;
7-(difluoromethyl)-N-(4-methoxy-6-(2-methoxyethoxy)pyridin-3-yl)quinolin-4-amine;
7-(difluoromethyl)-N-(2-(methoxy-d3)-4-(2-methoxyethoxy)phenyl)quinolin-4-amine;
7-(difluoromethyl)-N-(2-methoxy-6-(2-methoxyethoxy)pyridin-3-yl)quinolin-4-amine;
7-(difluoromethyl)-N-(2-methoxy-6-(2-(trifluoromethoxy)ethoxy)pyridin-3-yl)quinolin-4-amine;
7-(difluoromethyl)-N-(2-methoxy-4-(1H-pyrazol-4-yl)phenyl)quinolin-4-amine;
7-(difluoromethyl)-N-(2-methoxy-4-(2-(trifluoromethoxy)ethoxy)phenyl)quinolin-4-amine;
7-(difluoromethyl)-N-(4-fluoro-2-methoxyphenyl)quinolin-4-amine;
N-(4-chloro-2-methoxyphenyl)-7-(difluoromethyl)quinolin-4-amine;
7-(difluoromethyl)-N-(4-methoxy-6-(2-methoxyethoxy)pyridin-3-yl)quinolin-4-amine;
7-(difluoromethyl)-N-(4-(2-methoxyethoxy)-2-morpholinophenyl)quinolin-4-amine;
N-(2-cyclopropoxy-4-(2-methoxyethoxy)phenyl)-7-(difluoromethyl)quinolin-4-amine;
7-(difluoromethyl)-N-(2-(methoxy-d3)-4-(2-(trifluoromethoxy)ethoxy)phenyl)quinolin-4-amine;
N-(4-chloro-2-methoxyphenyl)-7-(difiuoromethyl)quinolin-4-amine;
N-(4-(1,1-difluoroethyl)-2-methoxyphenyl)-7-(difluoromethyl)quinolin-4-amine;
7-(difluoromethyl)-N-(2,4-dimethoxyphenyl)quinolin-4-amine;
7-(difluoromethyl)-N-(4-methoxy-2-(2-methoxyethoxy)pyrimidin-5-yl)quinolin-4-amine;
7-(difluoromethy))-N-(2-methoxy-4-(methylsulfonyl)phenyl)quinolin-4-amine;
N-cyclopropyl-4-((7-(difluoromethyl)quinolin-4-yl)amino)-3-methoxybenzamide;
7-(difluoromethyl)-N-(2-methoxy-4-(2-(trifluoromethoxy)ethoxy)phenyl)quinolin-4-amine;
7-(difluoromethyl)-N-(2-methoxy-6-(1H-pyrazol-4-yl)pyridin-3-yl)quinolin-4-amine;
N-(4-(3,5-dimethyl-1H-pyrazol-4-yl)-2-methoxyphenyl)-7-isopropylquinolin-4-amine;
N-(4-methoxy-6-(2-methoxyethoxy)pyridin-3-yl)-7-methylquinolin-4-amine;
N-(2-methoxy-6-(2-methoxyethoxy)pyridin-3-yl)-7-methylquinolin-4-amine;
N-(2-methoxy-6-(2-(trifluoromethoxy)ethoxy)pyridin-3-yl)-7-methylquinolin-4-amine;
N-(2-methoxy-4-(1H-pyrazol-4-yl)phenyl)-7-methylquinolin-4-amine;
N-(4-(2-(difluoromethoxy)ethoxy)-2-methoxyphenyl)-7-methylquinolin-4-amine;
N-(4-fluoro-2-methoxyphenyl)-7-methylquinolin-4-amine;
N-(4-bromo-2-methoxyphenyl)-7-methylquinolin-4-amine;
N-(4-(2-methoxyethoxy)-2-morpholinophenyl)-7-methylquinolin-4-amine;
N-(2-methoxy-4-(trifluoromethyl)phenyl)7-methylquinolin-4-amine;
N-(2-methoxy-4-(methylsulfonyl)phenyl)-7-methylquinolin-4-amine;
N-(2-cyclopropoxy-4-(2-(trifluoromethoxy)ethoxy)phenyl)-7-methylquinolin-4-amine;
N-(2-(methoxy-d3)-4-(2-(trifluoromethoxy)ethoxy)phenyl)-7-methylquinolin-4-amine;
N-(5-bromo-3-methoxypyrazin-2-yl)-7-methylquinolin-4-amine;
N-(4-methoxy-2-(2-methoxyethoxy)pyrimidin-5-yl)-7-methylquinolin-4-amine;
N-(4-(cyclopentyloxy)-2-methoxyphenyl)-7-methylquinolin-4-amine;
N-(4-methoxy-2-(2-(trifluoromethoxy)ethoxy)pyrimidin-5-yl)-7-methylquinolin-4-amine;
N-(4-chloro-2-methoxyphenyl)-7-methylquinolin-4-amine;
N-(4-(2-isopropoxyethoxy)-2-methoxyphenyl)-7-methylquinolin-4-amine;
N-(2-methoxy-6-(1H-pyrazol-4-yl)pyridin-3-yl)-7-methylquinolin-4-amine;
N-(2-methoxy-4-(1H-pyrazol-1-yl)phenyl)-7-methylquinolin-4-amine;
N-(4-(3,5-dimethyl-1H-pyrazol-4-yl)-2-methoxyphenyl)-7-methylquinolin-4-amine;
N-(4-(furan-2-yl)-2-methoxyphenyl)-7-methylquinolin-4-amine;
N-(4-(furan-3-yl)-2-methoxyphenyl)-7-methylquinolin-4-amine;
N-(2-methoxy-4-(1H-pyrazol-5-yl)phenyl)-7-methylquinolin-4-amine;
N-(2-methoxy-4-(4-methyl-1H-pyrazol-1-yl)phenyl)-7-methylquinolin-4-amine;
N-(4-(1,3-dimethyl-1H-pyrazol4-yl)-2-methoxyphenyl)-7-methylquinolin-4-amine;
N-(2-methoxy-4-(thiophen-2-yl)phenyl)-7-methylquinolin-4-amine;
N-(2-methoxy-4-(thiophen-3-yl)phenyl)-7-methylquinolin-4-amine;
N-(7-bromobenzo[d][1,3]dioxol-4-yl)-7-methylquinolin-4-amine;
N-(7-(1H-pyrazol-4-yl)benzo[d][1,3]dioxol-4-yl)-7-methylquinolin-4-amine;
7-methoxy-N-(2-(methoxy-d3)-4-(2-methoxyethoxy)phenyl)quinolin-4-amine;
N-(4-(2-(difluoromethoxy)ethoxy)-2-methoxyphenyl)-7-methoxyquinolin-4-amine; or
N-(4-(2-(difluoromethoxy)ethoxy)-2-methoxyphenyl)-7-(1-methyl-1H-pyrazol-4-yl)quinolin-4-amine.

The present invention also relates to each one of the intermediates described further below in the examples section of this specification, including any one of these intermediates in non-salt form or in the form of a salt (e.g., a pharmaceutically acceptable salt) of the respective compound. Such intermediates can be used, in particular, in the synthesis of the compounds of formula (I).

For a person skilled in the field of synthetic chemistry, various ways for the preparation of the compounds of formula (I) and their pharmaceutically acceptable salts will be readily apparent. For example, the compounds of the invention can be prepared in accordance with, or in analogy to, the synthetic routes described in detail in the examples section.

• The following definitions apply throughout the present specification and the claims, unless specifically indicated otherwise.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl,

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "alkenylene" refers to an alkenediyl group, i.e. a divalent unsaturated acyclic hydrocarbon group which may be linear or branched and comprises one or more (e.g., one or two) carbon to carbon double bonds while it does not comprise any carbon-to-carbon triple bond. A "C₂₋₅ alkenylene" denotes an alkenylene group having 2 to 5 carbon atoms. Unless defined otherwise, the term "alkenylene" preferably refers to C₂₋₄ alkenylene (including, in particular, linear C₂₋₄ alkenylene).

As used herein, the term "alkynylene" refers to an alkynediyl group, i.e. a divalent unsaturated acyclic hydrocarbon group which may be linear or branched and comprises one or more (e:g., one or two) carbon-to-carbon triple bonds and optionally one or more (e.g., one or two) carbon-to-carbon double bonds. A "C₂₋₅ alkynylene" denotes an alkynylene group having 2 to 5 carbon atoms. Unless defined otherwise, the term "alkynylene" preferably refers to C₂₋₄ alkynylene (including, in particular, linear C₂₋₄ alkynylene).

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two O atoms (which may optionally be oxidized) and/or ono, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). If the aryl is a bridged and/or fused ring system which contains, besides one or more aromatic rings, at least one non-aromatic ring (e.g., a saturated ring or an unsaturated alicyclic ring), then one or more carbon ring atoms in each non-aromatic ring may optionally be oxidized (i.e., to form an oxo group). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from 0, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two 0 atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e,g., 2-pyridyl, 3-pyridyl, or 4-pyridyl), pyrazinyl, pyrimidinyl, pyridazinyl, indolyl (e.g., 1H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, particularly preferred examples of a "heteroaryl" include pyridinyl (e.g., 2-pyridyl, 3-pyridyl, or 4-pyridyl), imidazolyl, thiazolyl, 1H-tetrazolyl, 2H-tetrazolyl, thienyl (i.e., thiophenyl), or pyrimidinyl.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members. Moreover, unless defined otherwise, particularly preferred examples of a "cycloalkyl" include cyclohexyl or cyclopropyl, particularly cyclohexyl.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three; or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two 0 atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, thiepanyl, decahydroquinolinyl, decahydroisoquinolinyl, or 2-oxa-5-aza-bicyclo[2.2.1]hept-5-yl. Unless defined otherwise, "heteracycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, particularly preferred examples of a "heterocycloalkyl" include tetrahydropyranyl, piperidinyl, piperazinyl, morpholinyl, pyrrolidinyl, or tetrahydrofuranyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a G₃-₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from 0, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two; three, or four) ring heteroatoms independently selected from 0, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from 0, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-CI), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint.

As used herein, the term "comprising" (or "comprise", "comprises", "contain", "contains", or "containing"), unless explicitly indicated otherwise or contradicted by context, has the meaning of "containing, inter alia", i.e_{.}, "containing, among further optional elements, ...". In addition thereto, this term also includes the narrower meanings of "consisting essentially of" and "consisting of. For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

The scope of the invention embraces all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such.as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Further pharmaceutically acceptable salts are described in the literature, e.g., in Stahl PH & Wermuth CG (eds.), "Handbook of Pharmaceutical Salts: Properties, Selection, and Use", Wiley-VCH, 2002 and in the references cited therein. Preferred pharmaceutically acceptable salts of the compounds of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) is in the form of a hydrochloride salt.

The present invention also specifically relates to the compound of formula (I), including any one of the specific compounds of formula (I) described herein, in non-salt form.

Moreover, the scope of the invention embraces the compounds of formula (I) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compounds of formula (I) are also encompassed within the scope of the invention. It is to be understood that such solvates and physical forms of pharmaceutically acceptable salts of the compounds of the formula (I) are likewise embraced by the invention.

Furthermore, the compounds of formula (I) may exist in the form of different isomers, in particular stereoisomers (including, e.g., geometric isomers (or cis/trans isomers), enantiomers and diastereomers) or tautomers (including, in particular, prototropic tautomers, such as keto/enol tautomers or thione/thiol tautomers). All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds of formula (I). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D), The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces compounds of formula (I), In which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers, trackers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

The compounds provided herein may be administered as compounds *per se* or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor^{®} HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin. γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-P-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

The pharmaceutical compositions may also comprise one or more preservatives, particularly one or more antimicrobial preservatives, such as, e.g., benzyl alcohol, chlorobutanol, 2-ethoxyethanol, m-cresol, chlorocresol (e.g., 2-chlom-3-methyl-phenol or 4-chloro-3-methyl-phenol), benzalkonium chloride, benzethonium chloride, benzoic acid (or a pharmaceutically acceptable salt thereof), sorbic. acid (or a pharmaceutically acceptable salt thereof), chlorhexidine, thimerosal, or any combination thereof.

The pharmaceutical compositions can be formulated by techniques known to the person skilled in the art, such as the techniques published in "Remington: The Science and Practice of Pharmacy", Pharmaceutical Press, 22nd edition.

The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or the above described pharmaceutical compositions comprising a compound of formula (I) may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrastemal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications. The tablets may contain excipients such as microcrystalline cellulose, lactose, sodium citrate, calcium carbonate, dibasic calcium phosphate and glycine, disintegrants such as starch (preferably com, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the compound may be combined with various sweetening or flavoring agents, coloring matter or dyes, with emulsifying and/or suspending agents and with diluents such as water, ethanol, propylene glycol and glycerin, and combinations thereof.

For oral administration, the compounds or pharmaceutical compositions are preferably administered by oral ingestion, particularly by swallowing. The compounds or pharmaceutical compositions can thus be administered to pass through the mouth into the gastrointestinal tract, which can also be referred to as "oral-gastrointestinal" administration.

Alternatively, said compounds or pharmaceutical compositions can be administered in the form of a suppository or pessary, or may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped compounds. The present invention thus also relates to liposomes containing a compound of the invention.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to an emulsification/spray drying process.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrastemal, intraventricular, intraurethral, or intracranial route; pulmonary route, including by inhalation or insulation therapy; gastrointestinal route; intrauterine route; intraocular route; subcutaneous route; ophthalmic route, including by intravitreal, or intracameral route; rectal route; or vaginal route.

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

The compound of formula (I) or a pharmaceutical composition comprising the compound of formula (I) can be administered in monotherapy (e.g., without concomitantly administering any further therapeutic agents, or without concomitantly administering any further therapeutic agents against the same disease that is to be treated or prevented with the compound of formula (I)). However, the compound of formula (I) or a pharmaceutical composition comprising the compound of formula (I) can also be administered in combination with one or more further therapeutic agents. If the compound of formula (I) is used in combination with a second therapeutic agent active against the same disease or condition, the dose of each compound may differ from that when the corresponding compound is used alone, in particular, a lower dose of each compound may be used. The combination of the compound of formula (I) with one or more further therapeutic agents may comprise the simultaneous/concomitant administration of the compound of formula (I) and the further therapeutic agent(s) (either in a single pharmaceutical formulation or in separate pharmaceutical formulations), or the sequential/separate administration of the compound of formula (I) and the further therapeutic agent(s). If administration is sequential, either the compound of formula (I) according to the invention or the one or more further therapeutic agents may be administered first. If administration is simultaneous, the one or more further therapeutic agents may be included in the same pharmaceutical formulation as the compound of formula (I), or they may be administered in two or more different (separate) pharmaceutical formulations.

For the treatment or prevention of cancer, the one or more further therapeutic agents to be administered in combination with a compound of the present invention (or a corresponding pharmaceutical composition) are preferably anticancer drugs. The anticancer drug(s) to be administered in combination with a compound of formula (I) according to the invention may, e.g., be selected from: a tumor angiogenesis inhibitor (e.g., a protease inhibitor, an epidermal growth factor receptor kinase inhibitor, or a vascular endothelial growth factor receptor kinase inhibitor); a cytotoxic drug (e.g., an antimetabolite, such as purine and pyrimidine analog antimetabolites); an antimitotic agent (e.g., a microtubule stabilizing drug or an antimitotic alkaloid); a platinum coordination complex; an anti-tumor antibiotic; an alkylating agent (e.g., a nitrogen mustard or a nitrosourea); an endocrine agent (e.g., an adrenocorticosteroid, an androgen, an anti-androgen, an estrogen, an anti-estrogen, an aromatase inhibitor, a gonadotropin-releasing hormone agonist, or a somatostatin analog); or a compound that targets an enzyme or receptor that is overexpressed and/or otherwise involved in a specific metabolic pathway that is deregulated (or misregulated) in the tumor cell (e.g., ATP and GTP phosphodiesterase inhibitors, histone deacetylase inhibitors, protein kinase inhibitors (such as serine, threonine and tyrosine kinase inhibitors, e.g., Abelson protein tyrosine kinase inhibitors) and the various growth factors, their receptors and corresponding kinase inhibitors (such as epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors)); methionine, aminopeptidase inhibitors, proteasome inhibitors, cyclooxygenase inhibitors (e.g., cyclooxygenase-1 or cyclooxygenase-2 inhibitors), topoisomerase inhibitors (e.g., topoisomerase I inhibitors or topoisomerase II inhibitors), poly ADP ribose polymerase inhibitors (PARP inhibitors), and epidermal growth factor receptor (EGFR) inhibitors/antagonists.

An alkylating agent which can be used as an anticancer drug in combination with a compound of the present invention may be, for example, a nitrogen mustard (such as cyclophosphamide, mechlorethamine (chlormethine), uramustine, melphalan, chlorambucil, ifosfamide, bendamustine, or trofosfamide), a nitrosourea (such as carmustine, streptozocin, fotemustine, lomustine, nimustine, prednimustine, ranimustine, or semustine), an alkyl sulfonate (such as busulfan, mannosulfan, or treosulfan), an aziridine (such as hexamethylmelamine (altretamine), triethylenemelamine, ThioTEPA (N,N'N'-triethylenethiophosphoramide), carboquone, or triaziquone), a hydrazine (such as procarbazine), a triazene (such as dacarbazine), or an imidazotetrazine (such as temozolomide). A platinum coordination complex which can bo used as an anticancer drug in combination with a compound of the present invention may be, for example, cisplatin, carboplatin, nedaplatin, oxaliplatin, satraplatin, or triplatin tetranitrate. A cytotoxic drug which can be used as an anticancer drug in combination with a compound of the present invention may be, for example, an antimetabolite, including folic acid analogue antimetabolites (such as aminopterin, methotrexate, pemetrexed, or raltitrexed), purine analogue antimetabolites (such as cladribine, clofarabine, fludarabine, 6 mereaptopurine (including its prodrug form azathioprino), pentostatin, or 6 thioguanine), and pyrimidine analogue antimetabolites (such as cytarabine, decitabine, 5 fluorouracil (including its prodrug forms capecitabine and tegafur), floxuridine, gemcitabine, enocitabine, or sapacitabine). An antimitotic agent which can be used as an anticancer drug in combination with a compound of the present invention may be, for example, a taxane (such as docetaxel, larotaxel, ortataxel, paclitaxel/taxol, tesetaxel, or nab-paclitaxel (e.g., Abraxane^{®})), a Vinca alkaloid (such as vinblastine, vincristine, vinflunine, vindesine, or vinorelbine), an epothilone (such as epothilone A, epothilone B, epothilone C, epothilone D, epothilone E, or epothilone F) or an epothilone B analogue (such as ixabepilone/azaepothHone B). An anti-tumor antibiotic which can be used as an anticancer drug in combination with a compound of the present invention may be, for example, an anthracycline (such as aclarubicin, daunorubicin, doxorubicin, epirubicin, idarubicin, amrubicin, pirarubicin, valrubicin, or zorubicin), an anthracenedione (such as mitoxantrone, or pixantrone) or an anti-tumor antibiotic isolated from Streptomyces (such as actinomycin (including actinomycin D), bleomycin, mitomycin (including mitomycin C), or plicamycin). A tyrosine kinase inhibitor which can be used as an anticancer drug in combination with a compound of the present invention may be, for example, axitinib, bosutinib, cediranib, dasatinib, erlotinib, gefitinib, imatinib, lapatinib, lestaurtinib, nilotinib, semaxanib, sorafenib, sunitinib, axitinib, nintedanib, ponatinib, vandetanib, or vemurafenib. A topoisomerase inhibitor which can be used as an anticancer drug in combination with a compound of the present invention may be, for example, a topoisomerase I inhibitor (such as irinotecan, topotecan, camptothecin, belotecan, rubitecan, or lamellarin D) or a topoisomerase II inhibitor (such as amsacrine, etoposide, etoposide phosphate, teniposide, or doxorubicin). A PARP inhibitor which can be used as an anticancer drug in combination with a compound of the present invention may be, for example, niraparib, olaparib, rucaparib, talazoparib, veliparib, pamiparib (BGB-290), BMN-673, CEP 9722, MK 4827, E7016, or 3-aminobenzamide. An EGFR inhibitor/antagonist which can be used as an anticancer drug in combination with a compound of the present invention may be, for example, gefitinib, erlotinib, lapatinib, afatinib, neratinib, osimertinib, brigatinib, dacomitinib, vandetanib, pelitinib, canertinib, icotinib, poziotinib, ABT-414, AV-412, PD 153035, PKI-166, BMS-690514, CUDC-101, AP26113, XL647, cetuximab, panitumumab, zalutumumab, nimotuzumab, or matuzumab. Further anticancer drugs may also be used in combination with a compound of the present invention. The anticancer drugs may comprise biological or chemical molecules, like TNF-related apoptosis-inducing ligand (TRAIL), tamoxifen, amsacrine, bexarotene, estramustine, irofulven, trabectedin, cetuximab, panitumumab, tositumomab, alemtuzumab, bevacizumab, edrecolomab, gemtuzumab, alvocidib, seliciclib, aminolevulinic acid, methyl aminolevulinate, efaproxiral, porfimer sodium, talaporfin, temoporfin, verteporfin, alitretinoin, tretinoin, anagrelide, arsenic trioxide, atrasentan, bortezomib, carmofur, celecoxib, demecolcine, elesclomol, elsamitrucin, etoglucid, lonidamine, lucanthone, masoprocol, mitobronitol, mitoguazone, mitotane, oblimersen, omacetaxine, sitimagene, ceradenovec, tegafur, testolactone, tiazofurine, tipifarnib, vorinostat, iniparib, or copanlisib.

An anticancer drug which can be used in combination with a compound of the present invention may also be an immunooncology therapeutic (such as an antibody (e.g., a monoclonal antibody or a polyclonal antibody), an antibody fragment, an antibody construct (e.g., a single-chain construct), or a modified antibody (e.g., a CDR-grafted antibody, a humanized antibody, or a "fully human" antibody) targeting, e.g., any one of CTLA-4, PD-1, PD-L1, TIGIT, TIM3, LAG3, OX40, CSF1R, IDO, or CD40. Such immunooncology therapeutics include, e.g., an anti-CTLA-4 antibody (particularly an antagonistic or pathway-blocking anti-CTLA-4 antibody; e.g., ipilimumab or tremelimumab), an anti-PD-1 antibody (particularly an antagonistic or pathway-blocking anti-PD-1 antibody; e.g., nivolumab (BMS-936558), pembrolizumab (MK-3475), pidilizumab (CT-011), cemiplimab, dostarlimab, spartalizumab, camrelizumab, sintilimab, tislelizumab, toripalimab, zimberelimab, AMP-224, AMP-514 (or MEDI0680), JTX-4014, INCMGA00012 (or MGA012), or APE02058), an anti-PD-L1 antibody (particularly a pathway-blocking anti-PD-L1 antibody; e.g., atezolizumab, avelumab, durvalumab, KN035, CK-301, BMS-936559, MEDI4736, MPDL3280A (RG7446), MDX-1105, MEDI6469, or bintrafusp alfa), an anti-TIGIT antibody (e.g., tiragolumab, vibostolimab, domvanalimab, etigilimab, BMS-986207, EOS-448, COM902, ASP8374, SEA-TGT, BGB-A1217, IBI-939, or M6223), an anti-TIM3 antibody (particularly a pathway-blocking anti-TIM3 antibody), an anti-LAG3 antibody (particularly an antagonistic or pathway-blocking anti-LAG3 antibody; e.g., relatlimab (or BMS-986016), ieramilimab (or LAG525), encelimab (or TSR-033), tebotelimab (or MGD013), REGN3767 (or R3767), FS118, IMP701, or IMP731), an anti-OX40 antibody (particularly an agonistic anti-OX40 antibody; e.g., MEDI0562); an anti-CSF1R antibody (particularly a pathway-blocking anti-CSF1 R antibody; e.g., IMC-CS4 or RG7155), an anti-IDO antibody (particularly a pathway-blocking anti-IDO antibody), or an anti-CD40 antibody (particularly an agonistic anti-CD40 antibody; e.g., CP-870,893 or Chi Lob 7/4). Further examples of immunooncology therapeutics include anti-HER2 antibodies (e.g. trastuzumab), anti-CD20 antibodies (e.g. rituximab), anti-CD19/CD3 constructs, and anti-TNF antibodies.

In particular, a compound of formula (I) or a pharmaceutical composition comprising a compound of formula (I) may be administered in combination with an immune checkpoint inhibitor, preferably an antibody (or an antigen-binding fragment thereof, or an antibody construct) directed against CTLA-4, PD-1, PD-L1, TIGIT, or LAG3. Corresponding preferred examples include, but are not limited to, any one of the anti-CTLA-4 antibodies ipilimumab or tremelimumab, any one of the anti-PD-1 antibodies nivolumab, pembrolizumab, pidilizumab, cemiplimab, dostarlimab, spartalizumab, camrelizumab, sintilimab, tislelizumab, toripalimab, zimberelimab, AMP-224, AMP-514, JTX-4014, INCMGA00012, or APE02058, any one of the anti-PD-L1 antibodies atezolizumab, avelumab, durvalumab, KN035, CK-301, BMS-936559, MEDI4736, MPDL3280A, MDX-1105, MEDI6469 or bintrafusp alfa, any one of the anti-TIGIT antibodies tiragolumab, vibostolimab, domvanalimab, etigilimab, BMS-986207, EOS-448, COM902, ASP8374, SEA-TGT, BGB-A1217, IBI-939 or M6223, and/or any one of the anti-LAG3 antibodies relatlimab, ieramilimab, encelimab, tebotelimab, REGN3767, FS118, IMP701, or IMP731. The present invention thus relates to a compound of formula (I) or a pharmaceutically acceptable salt thereof, or a pharmaceutical composition comprising any of the aforementioned entities in combination with a pharmaceutically acceptable excipient, for use in the treatment or prevention of cancer, wherein the compound or the pharmaceutical composition is to be administered in combination with one or more immune checkpoint inhibitors, wherein said one or more immune checkpoint inhibitors are preferably selected from anti-CTLA-4 antibodies, anti-PD-1 antibodies, anti-PD-L1 antibodies, anti-TIGIT antibodies, and/or anti-LAG3 antibodies; more preferably, said one or more immune checkpoint inhibitors are selected from ipilimumab, tremelimumab, nivolumab, pembrolizumab, pidilizumab, cemiplimab, dostarlimab, spartalizumab, camrelizumab, sintilimab, tislelizumab, toripalimab, zimberelimab, AMP-224, AMP-514, JTX-4014, INCMGA00012, APE02058, atezolizumab, avelumab, durvalumab, KN035, CK-301, BMS-936559, MEDI4736, MPDL3280A, MDX-1105, MEDI6469, bintrafusp alfa, tiragolumab, vibostolimab, domvanalimab, etigilimab, BMS-986207, EOS-448, COM902, ASP8374, SEA-TGT, BGB-A1217, IBI-939, M6223, relatlimab, ieramilimab, encelimab, tebotelimab, REGN3767, FS118, IMP701, and IMP731.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the compound of the present invention (i.e., the compound of formula (I) or a pharmaceutically acceptable salt thereof) or the further therapeutic agent(s) may be administered first. When administration is simultaneous, the combination may be administered either in the same pharmaceutical composition or in different pharmaceutical compositions. When combined in the same formulation, it will be appreciated that the two or more compounds must be stable and compatible with each other and the other components of the formulation. When formulated separately, they may be provided in any convenient formulation and may be administered by any convenient route.

The compounds of formula (I) can also be administered in combination with physical therapy, such as radiotherapy. Radiotherapy may commence before, after, or simultaneously with administration of the compounds of the invention. For example, radiotherapy may commence about 1 to 10 minutes, about 1 to 10 hours, or about 24 to 72 hours after administration of the compound of formula (I). The subject/patient is exposed to radiation, preferably gamma radiation, whereby the radiation may be provided in a single dose or in multiple doses that are administered over several hours, days and/or weeks. Gamma radiation may be delivered according to standard radiotherapeutic protocols using standard dosages and regimens.

The present invention thus relates to a compound of formula (I) or a pharmaceutically acceptable salt thereof, or a pharmaceutical composition comprising any of the aforementioned entities in combination with a pharmaceutically acceptable excipient, for use in the treatment or prevention of cancer, wherein the compound or the pharmaceutical composition is to be administered in combination with one or more anticancer drugs (including any one or more of the specific anticancer drugs described herein above) and/or in combination with radiotherapy.

Yet, the compounds of formula (I) can also be used in monotherapy, particularly in the monotherapeutic treatment or prevention of cancer (i.e., without administering any other anticancer agents until the treatment with the compound(s) of formula (I) is terminated). Accordingly, the invention also relates to a compound of formula (I) or a pharmaceutically acceptable salt thereof, or a pharmaceutical composition comprising any of the aforementioned entities in combination with a pharmaceutically acceptable excipient, for use in the monotherapeutic treatment or prevention of cancer.

Moreover, the compounds of formula (I) - either in combination with one or more further anticancer agents (including any of the exemplary anticancer agents described above) or without any further anticancer agents - can also be administered in combination with an antiemetic agent. The antiemetic agent may, for example, be selected from alosetron, azasetron, bemesetron, cilansetron, clozapine, dazopride, dolasetron, granisetron, lerisetron, metoclopramide, mianserin, mirtazapine, olanzapine, ondansetron, palonosetron (e.g., palonosetron alone, or palonosetron in combination with netupitant), quetiapine, ramosetron, ricasetron, tropisetron, zatosetron, clozapine, cyproheptadine, hydroxyzine, olanzapine, risperidone, ziprasidone, dronabinol, nabilone, tetrahydrocannabinol, alizapride, bromopride, chlorpromazine, clebopride, domperidone, haloperidol, hydroxyzine, itopride, metoclopramide, metopimazine, prochlorperazine, thiethylperazine, trimethobenzamide, cyclizine, dimenhydrinate, diphenhydramine, hydroxyzine, meclizine, promethazine, atropine, diphenhydramine, hyoscyamine, scopolamine, aprepitant, casopitant, ezlopitant, fosaprepitant, maropitant, netupitant, rolapitant, vestipitant, cerium oxalate, dexamethasone, lorazepam, midazolam, propofol, or a combination thereof. Preferably, the antiemetic agent is a 5-HT₃ antagonist (or a "setron"), such as, e.g., alosetron, azasetron, bemesetron, cilansetron, clozapine, dazopride, dolasetron, granisetron, lerisetron, metoclopramide, mianserin, mirtazapine, olanzapine, ondansetron, palonosetron (optionally in combination with netupitant), quetiapine, ramosetron, ricasetron, tropisetron, or zatosetron.

The subject or patient to be treated in accordance with the present invention may be an animal (e.g., a non-human animal): Preferably, the subject/patient is a mammal. More preferably, the subject/patient is a human (e.g., a male human or a female human) or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient to be treated in accordance with the invention is a human.

The term "treatment" of a disorder or disease, as used herein, is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, *inter alia,* comprise curative treatment (preferably leading to a complete response and eventually to healing of the disorder or disease) and palliative treatment (including symptomatic relief).

The term "prevention" of a disorder or disease, as used herein, is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of a compound of the present invention before any clinical and/or pathological symptoms are diagnosed or determined or can be diagnosed or determined by the attending physician.

It is to be understood that the present invention specifically relates to each and every combination of features and embodiments described herein, including any combination of general and/or preferred features/embodiments. In particular, the invention specifically relates to each combination of meanings (including general and/or preferred meanings) for the various groups and variables comprised in formula (I).

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

The present invention is also illustrated by the following figure:

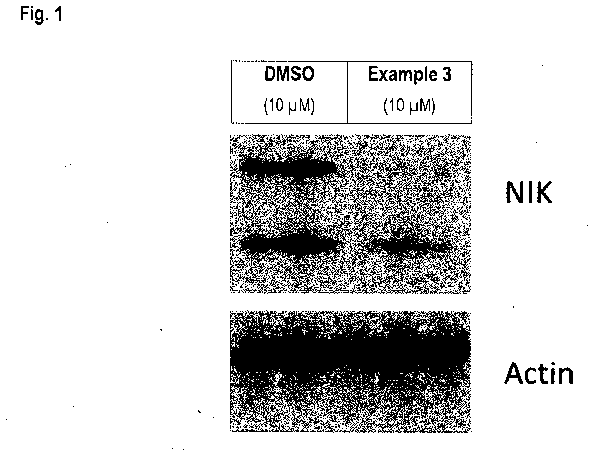

**Figure 1****:** Western blot analysis of NIK degradation on A375 cells treated with the compound of Example 3 at 10 µM for 72 h (see Example 100).

The following examples further illustrate the present invention. The compounds of formula (I) described in the examples section are defined by their chemical formulae and their corresponding chemical names. In case of conflict between any chemical formula and the corresponding chemical name indicated herein, the present invention relates to both the compound defined by the chemical formula and the compound defined by the chemical name, and particularly relates to the compound defined by the chemical formula.

### EXAMPLES

### Introductory section

The compounds of the present invention may be synthesized, e.g., as illustrated with reference to the compounds of Examples 1 to 99 in the following Schemes 1 to 4:

### Scheme 1

Example 1 can be prepared according to the following reaction Scheme 1:

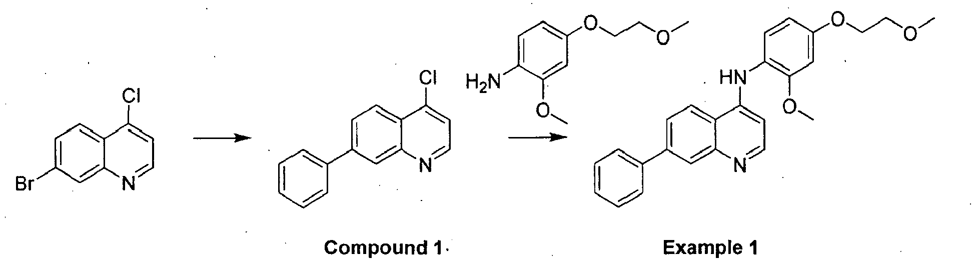

Compound 1 can be synthetized from 7-bromo-4-chloroquinoline and phenylboronic acid using a Suzuki coupling reaction, then the coupling between compound 1 and 2-methoxy-4-(2-methoxyethoxy)aniline leads to example 1.

### Scheme 2

Examples 3, 9-10, 46-55, 58-76, 79-86, 91, 95 and 97-99. can be prepared according to the following reaction Scheme 2:

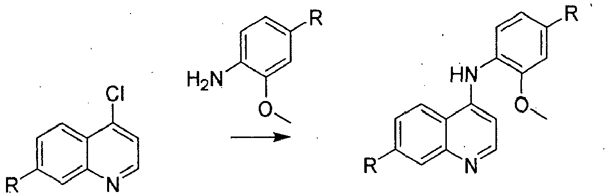

A mixture of the desired 4-chloroquinoline and the corresponding aniline were refluxed at 80°C.

### Scheme 3

Examples 2, 4, 5-8, 11-44, 56-57 and 77-78 can be prepared according to the following reaction Scheme 3: 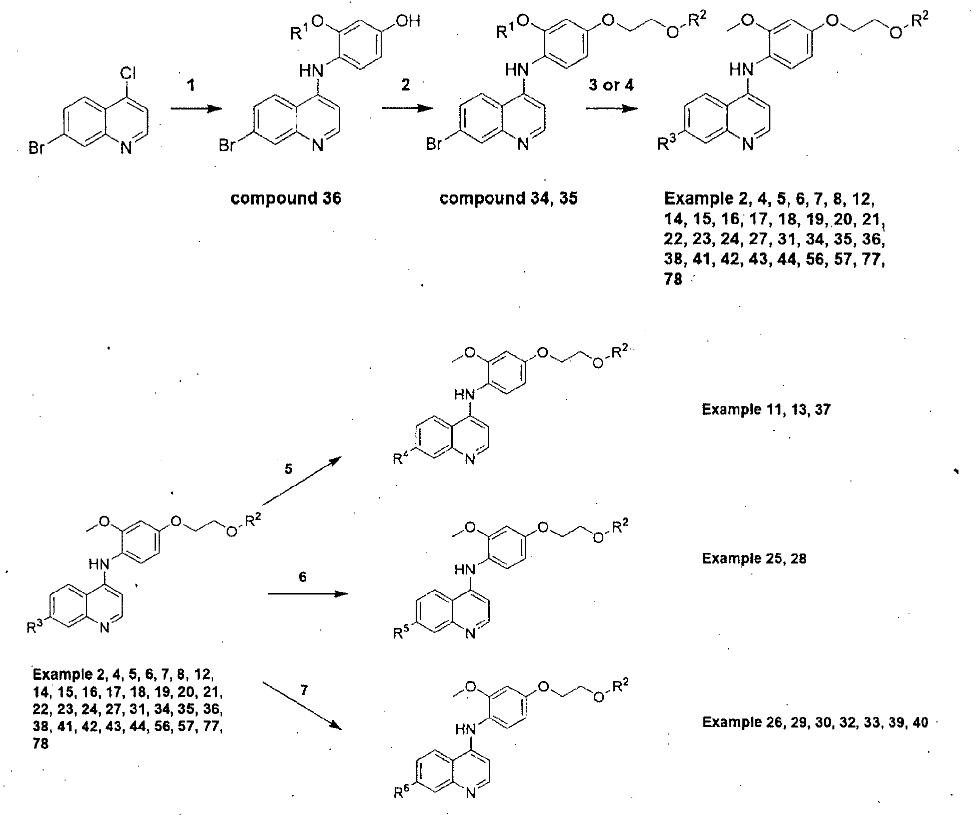
**1:** A mixture of desired 4-chloroquinoline and 4-amino-3-methoxyphenol was stirred at 80°C overnight.
**2:** A mixture of 4-((7-bromoquinolin-4-yl)amino)-3-methoxyphenol and 2-methoxyethyl-4-methylbenzenesulfonate or . 1-bromo-2-(trifluoromethoxy)ethane in the presence of caesium carbonate was stirred at 60°C.
**3:** The desired 7-bromoquinoline was coupled with the desired aryl-tributylstannyl using a Stille coupling reaction.
**4:** The desired 7-bromoquinoline was coupled with the desired boronic acid using a Suzuki coupling reaction.
**5:** The desired alkene was hydrogenated using Pd/C with H₂ (5 bars).
**6:** The desired Boc-protected amine was deprotected in acidic conditions.
**7:** The desired Boc-protected amine was reduced to corresponding methylamines using lithium aluminium hydride.

### Scheme 4

Examples 87-90, 92-94 and 96 can be prepared according to the following reaction Scheme 4:

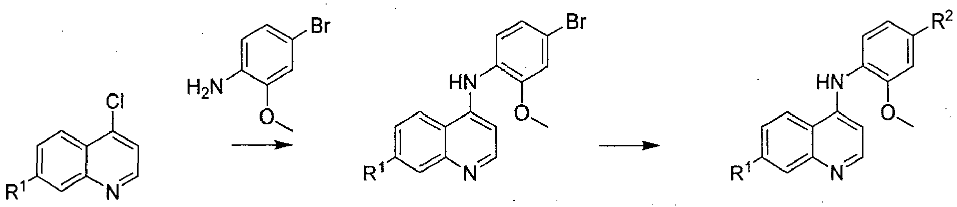

A mixture of the desired 4-chloroquinoline and the corresponding aniline were refluxed at 80°C, then the corresponding bromine derivatives and desired boronic acid were coupled using Suzuki coupling conditions.

### Experimental section

Several methods for preparing the compounds of this invention are illustrated in the following examples. Reagents were obtained from commercial sources and used without further purification. All temperatures are in °C. TLC was performed on aluminium backed silica gel plates with fluorescence indicator at 254 nM (median pore size 60 Å). Flash column chromatography was performed using a Biotage Isolera One system using KP-Sil or Ultra silica gel columns or an Isco CombiFlash Rf using FlashPure or RediSep Rf/RediSep Rf Gold silica gel columns. NMR spectra were recorded on a 400 or 500 MHz spectrometer at ambient probe temperature (nominal 298 K). Chemical shifts (δ) are given in ppm and calibrated by using the residual peak of the solvent as the internal standard (CDCl₃, δ = 7.26 ppm; DMSO-*d*₆, δ = 2.50 ppm). Coupling constants are given in Hertz (Hz). LRMS were recorded using an Advion Plate Express expression^{®} compact mass spectrometer equipped with an APCI ion source.

LCMS analysis was performed using a Waters Acquity UPLC with either a CSH C18 or BEH C18 column (2.1 × 30 mm) at 40 °C at 0.77 mL/min with a linear 2-100% acetonitrile gradient appropriate for the lipophilicity of the compound over 3 or 10 minutes. The aqueous portion of the mobile phase was 0.1% formic acid (CSH C18 column) or 10 mM ammonia bicarbonate (BEH C18 column). LC-UV chromatograms were recorded using a Waters Acquity photodiode array detector between 210 and 400 nm. Mass spectra were recorded using a Waters Acquity QDa detector with ESI switching between positive and negative ion mode.

### Method A

Apparatus: Waters HClass; Quaternary Solvent Pump, SM-FTN, CMA, PDA:210-400 nm, QDa: ACQ-QDa ESI; Column: Waters CSH C18, 30 × 2.1 mm, 1.7µm, Temp: 40°C, Flow: 0.77 mL/min, Gradient: t0 = 2% B, t2.5min = 100% B, t3.0min = 100% B, Eluent A: 0.1% Formic acid in water, Eluent B: acetonitrile.

### Method B

Apparatus: Waters HClass; Binary Solvent Pump, SM-FTN, CMA, PDA:210-400 nm, QDa: ACQ-QDa ESI; Column: Waters BEH C18, 30 × 2.1 mm, 1.7µm, Temp: 40°C, Flow: 0.77 mL/min, Gradient: t0 = 2% B, t2.5min = 100% B, t3.0min = 100% B, Eluent A: 0.1% NH3 in water, Eluent B: acetonitrile.

### Method C

Apparatus: Waters HClass; Quaternary Solvent Pump, SM-FTN, CMA, PDA:210-400 nm, QDa: ACQ-QDa ESI; Column: Waters CSH C18, 30 × 2.1 mm, 1.7µm, Temp: 40°C, Flow: 0.77 mL/min, Gradient: t0 = 2% B, t9.5min = 100% B, t10.0min = 100% B, Eluent A: 0.1% Formic acid in water, Eluent B: acetonitrile.

### Method D

Apparatus: Waters HClass; Binary Solvent Pump, SM-FTN, CMA, PDA:210-400 nm, QDa: ACQ-QDa ESI; Column: Waters BEH C18, 30 × 2.1 mm, 1.7µm, Temp: 40°C, Flow: 0.77 mL/min, Gradient: t0 = 2% B, t9.5min = 100% B, t10.0min = 100% B, Eluent A: 0.1% NH3 in water, Eluent B: acetonitrile.

### Method E

Apparatus: Agilent 1260; Binary Pump, HiP Sampler, Column Compartment, DAD:260+/- 90nm, G6150 MSD: ESI; Column: Waters Cortecs C18, 30 × 2.1 mm, 2.7µm, Temp: 40°C, Flow: 1.35 mL/min, Gradient: t0 = 5% B, t2.5min = 100% B, t3.0min = 100% B, Eluent A: 0.1% Formic in water, Eluent B: acetonitrile.

### General Procedures

### General Procedure A for the S_{N}Ar of a substituted 4-chloroquinoline

A solution of the desired 4-chloroquinoline (1.0 eq) and 2-methoxy-4-(2-methoxyethoxy)aniline (1.2 eq) in EtOH (2 mL) was heated to 80 °C and stirred for 18 h. The solvent was evaporated, and the residue taken into acetone (5 mL) and then sonicated to form a suspension. The precipitate was collected by filtration, washing with acetone (10 mL × 3), and then dried in a vacuum desiccator at 50 °C to afford the title compound.

### General Procedure B for the acid-catalysed S_{N}Ar of a substituted 4-chloroquinoline

To a solution of the desired 4-chloroquinoline (1.0 eq) and the desired aniline (1.2 eq) in IPA (0.3 M) was added HCI, 4.0 M in dioxane (1.2 eq). The reaction mixture was heated to 80 °C and stirred for 18 h. The solvent was evaporated, and the residue taken into acetone (5 mL) and then sonicated to form a suspension. The precipitate was collected by vacuum filtration, washed with acetone (10 mL × 3), and then optionally purified by column chromatography on silica gel or reversed phase prep HPLC. If necessary, the final product was optionally converted to the HCI salt using 4.0 M HCI in dioxane and then dried in a vacuum desiccator at 50 °C to afford the title compound.

### General Procedure C for the Suzuki reaction of a 7-bromoquinoline

To a stirred solution of the desired 7-bromoquinoline (1.0 eq) in 1,4-dioxane (2 mL), at rt, was added K₂CO₃ (3.0 eq). The reaction mixture was degassed by bubbling a stream of nitrogen gas through it for 10 min. To this mixture was added the desired boronic acid (2.0 eq) and XPhos Pd G4 (5 mol%). The reaction mixture was heated and stirred at 80 °C for 18 h. The reaction mixture was cooled down to rt and adsorbed onto silica. The crude products were purified by chromatography on silica gel to afford the title compound.

### General Procedure D for the Buchwald reaction of a 7-bromoquinoline

To a stirred solution of the desired 7-bromoquinoline in 1,4-dioxane (4 mL), at rt, was added the desired amine (2.0 eq). The mixture was degassed by bubbling a stream of nitrogen gas through it for 10 min. To this mixture was added 2.0 M NaOt-Bu in THF (5.0 eq) and RuPhos Pd G3 (10 mol%). The reaction mixture was heated and stirred at 100 °C for 18 h. The reaction mixture was cooled to rt and adsorbed onto silica. The crude product was purified by chromatography on silica gel to afford the title compound.

### Procedure E for the Buchwald reaction of a 7-bromoquinoline and piperazin-2-one

A reaction vessel was charged with 7-bromo-N-(2-methoxy-4-(2-methoxyethoxy)phenyl)quinolin-4-amine (1.0 eq), piperazin-2-one (248 mg, 4.0 eq, 2.48 mmol), XantPhos Pd G3 (20 mol%) and K₃PO₄ (4.0 eq). *t*-BuOH (0.3 M) was added and the reaction mixture was degassed with nitrogen and heated under microwave irradiation to 120 °C for 2 h. Aqueous work-up (DCM-MeOH 9:1/water) followed by prep-HPLC gave the title compound.

### General Procedure F for hydrogenation of alkene intermediates and nitro intermediates

To a stirred solution of the alkene in a 4:1 mixture of EtOH and MeOH (5 mL) at room temperature was added 10% Pd/C (50 wt% water, 0.1 Eq) in EtOH (1 mL). Then, the mixture was hydrogenated with H₂ (5 bar) for 24 hours. The reaction mixture filtered and the solution was concentrated in vacuo to afford the title compound.

### General Procedure G for the Stille coupling of a 7-bromoquinoline

In a sealed tube, to a degassed stirred solution the desired 7-bromoquinoline (1 Eq) in DMF (0.15 molar), at room temperature, was added lithium chloride (3.0 Eq), Tetrakis(triphenylphosphine)-palladium(0) (10 mol%) then, the desired aryl-tributylstannyl (3.0 Eq). The reaction was heated to 110 °C and stirred for 3 h. The reaction was cooled to room temperature, then MeCN (40 mL) was added. The acetonitrile solution was extracted with heptane (5×10 mL). To the acetonitrile phase was added potassium fluoride (2 g) and sodium sulfate (2 g), then the mixture was stirred for 30 min at room temperature. The mixture was filtered through a celite pad and the filtrate was concentrated *in vacuo.* The crude product was purified by chromatography on silica gel (0.7 M NH₃ in MeOH/DCM). The product was further purified by prep-HPLC to afford the title compound.

### General Procedure H for deprotection of Boc-protected amines

A solution of the boc-protected amine (1 eq) and HCI (4 M in dioxne) (20 eq) in THF (1.00 mL) was stirred at 40 °C for 18 h. The solvent was removed *in vacuo* and the crude product was purified by chromatography on silica gel (0.7 M NH₃ in MeOH/DCM) to give the title compound.

### General Procedure I for reduction of Boc-protected amines to corresponding methylamines

A solution of boc-protected amine (1 eq) in THF (0.05 molar) was cooled to 0 °C before dropwise addition of LiAlH₄ 1.0 M in THF (5.49 eq). The reaction was stirred at 0 °C for 10 min, then at room temperature for 15 min before heating it to 65 °C for 3 h. The reaction mixture was then cooled to 0 °C and quenched with water (200 µL) and 2 M aq. NaOH (150 µL). The suspension was filtered and washed successively with THF (5 mL), EtOAc (10 mL) and DCM (20 mL). The filtrate was concentrated under reduced pressure. The crude product was further purified by trituration in cold Et₂O to afford the title compound.

### General Procedure J for alkylation of phenols with an alkyl tosylate

A mixture of the desired phenol (1 eq), 2-(trifluoromethoxy)ethyl 4-methylbenzenesulfonate (1.05 eq) and cesium carbonate (2.1 eq) in acetone (0.08 molar) was stirred at 60 °C for 18 h. The solvent was removed *in vacuo* and the residue was triturated in water (20 ml) and the solid was collected by vacuum filtration. The crude product was purified by chromatography on silica gel ([0.7 M ammonia/MeOH]/DCM) and the resulting material was taken up in .4 M HCI in dioxane (2 mL) and the solvent was removed *in vacuo.* The residue was taken up in MeOH (2 ml) and then concentrated (3 times). The residue was then triturated in diethyl ether and the resulting solid was collected and dried to give the title compound.

### General Procedure K for the Suzuki reaction with N-(4-bromo-2-methoxyphenyl)-7-methylquinolin-4-amine

A solution of N-(4-bromo-2-methoxyphenyl)-7-methylquinolin-4-amine (1 eq), desired boronic acid (2.0 eq) and K₂CO₃ (3eq) in a mixture of toluene/EtOH/H₂O (1/0.5/0.25) was degassed by bubbling a stream of argon gas. Tetrakis(triphenylphosphine)palladium(0) (5 mol%) was added and the mixture was stirred overnight at 90°C. After completion,'the product was extracted with EtOAc, washed with brine, dried over MgSO₄ and the solvent was removed *in vacuo.* The crude product was purified by chromatography on silica gel to afford the title compound.

### Preparation of synthetic intermediates

Preparation of compound 1 **(4-chloro-7-phenylquinoline):**
A reaction vessel was charged with 7-bromo-4-chloroquinoline (1.0 g, 4.12 mmol), cesium carbonate (2.7 g, 8.29 mmol), phenylboronic acid (530 mg, 4.35 mmol) and tetrakis(triphenylphosphine)-palladium(0) (500 mg, 433 µmol). The reaction vessel was evacuated and purged with nitrogen and then 1,4-dioxane (10.0 mL) and water (2.0 mL) was added. The reaction mixture was evacuated and purged with nitrogen three times and then stirred at 80 °C for 18 h. The reaction mixture was partitioned between EtOAc (20 mL) and sat. NH₄Cl (20 mL) and the phases were separated. The aqueous phase was extracted with EtOAc (10 mL × 2) and the combined organic phases were washed with brine (20 mL), dried over MgSO₄, filtered and the solvent was removed *in vacuo.* The crude product was dry-loaded on silica gel (40 g cartridge, gradient elution 0-50% (10% MeOH in DCM)/*iso*-hexane) to afford 4-chloro-7-phenylquinoline (570 mg, 55 %) as a white solid.

UPLC (Method A): m/z 240.3 [M+H]+ at 2.19 min.

¹H NMR (400 MHz, Chloroform-*d*) δ 8.81 (d, J = 4.7 Hz, 1H), 8.38 (d, J = 1.8 Hz, 1H), 8.31 (d, J = 8.7 Hz, 1H), 7.94 (dd, J = 8.7, 1.8 Hz, 1H), 7.80 - 7.73 (m, 2H), 7.57 - 7.48 (m, 3H), 7.48 - 7.40 (m, 1H).

Preparation of compound 2 **(4-chloro-7-(difluoromethyl)quinoline):**
To a solution of 4-chloroquinoline-7-carbaldehyde (166 mg, 1 eq, 866 µmol) in dry DCM (2 mL) at rt was added deoxofluor, 50% in toluene (420 mg, 350 µL, 50% Wt, 1.10 eq, 949 µmol). The reaction mixture was stirred at rt overnight. The reaction mixture was partitioned between DCM (10 mL) and sat. NaHCO₃ (10 mL) and the phases were separated. The aqueous phase was extracted with DCM (10 mL × 2) and the combined organic phases were washed with brine (20 mL), dried over MgSO₄, filtered and the solvent was removed *in vacuo.* The crude product was dry-loaded on silica and was purified by chromatography on silica gel (12 g cartridge, 0-50% MTBE/isohexane) to afford 4-chloro-7-(difluoromethyl)quinoline (119 mg, 0.53 mmol, 61 %, 95% Purity) as a white solid.

UPLC (Method A): m/z 214.1 [M+H]+ at 1.73 min. .

¹H NMR (400 MHz, DMSO-d6) δ 8.95 (d, J = 4.7 Hz, 1H), 8.38 (d, J = 8.7 Hz, 1H), 8.34 - 8.29 (m, 1H), 7.93 (d, J = 8.7 Hz, 1H), 7.90 (d, J = 4.7 Hz, 1H), 7.32 (t, J = 55.4 Hz, 1H).

Preparation of compound 3 **(4-chloro-7-(1-methyl-1H-pyrazol-4-yl)quinoline):**
A reaction vessel was charged with 7-bromo-4-chloroquinoline (1.0 eq), (1-methyl-1H-pyrazol-4-yl)boronic acid (1.3 eq), XPhos Pd G4 (5 mol%) and K₂CO₃ (3.0 eq). The reaction vessel was evacuated and purged with nitrogen and then a 4:1 mixture of 1,4-dioxane (0.3 M) was added. The reaction mixture was evacuated and purged with nitrogen three times and then heated to 80 °C and stirred under nitrogen for 18 h. The reaction mixture was cooled to room temperature and additional (1-methyl-1H-pyrazol-4-yl)boronic acid (0.5 eq) and XPhos Pd G4 (3 mol%) were added and the reaction mixture was evacuated and purged with nitrogen three times, heated to 80 °C and stirred under nitrogen for 3 h. The reaction mixture was cooled to room temperature and partitioned between 9:1 EtOAc/MeOH (50 mL) and water (50 mL) The phases were separated and the aqueous phase was extracted with 9:1 EtOAc/MeOH (25 mL × 2) and the combined organic phases were washed with brine (50 mL), dried over MgSO₄ filtered and the solvent was evaporated *in vacuo.* The crude product was purified by chromatography on silica gel, eluting with 0-10% (0.7 M NH₃/MeOH)/dichloromethane. The product was then triturated with MTBE (10 mL) and then Et₂O (10 mL × 2) and the solid was collected by vacuum filtration to give the title compound (124 mg, 12%) as a white solid.

UPLC (Method A): m/z 244.0 [M+H]⁺ at 1.38 min.

¹H NMR (400 MHz, DMSO-*d*₆) δ 8.80 (d, J = 4.7 Hz, 1H), 8.43 (s, 1H), 8.26 (d, J = 1.7 Hz, 1H), 8.17 (d, J = 8.7 Hz, 1H), 8.13 (d, J = 0.8 Hz, 1H), 8.01 (dd, J = 8.8,1.8 Hz, 1H), 7.67 (d, J = 4.7 Hz, 1H), 3.91 (s, 3H).

Preparation of compound 4 **(2-(methoxy-d3)-4-(2-methoxyethoxy)-1-nitrobenzene):**
In a sealed tube, to a stirred solution of 5-(2-methoxyethoxy)-2-nitrophenol (771 mg, 3.62 mmol) in acetone (4.0 mL), at rt, was added K₂CO₃(750 mg, 5.42 mmol) and iodomethane-d3 (1.0 g, 6.9 mmol). The reaction mixture was stirred at rt for 7 days. To the reaction was added a saturated aqueous NaHCO₃ (20 mL). The mixture was extracted with DCM (3 × 40 mL), the combined organic phase was washed with brine (20 mL), dried through a separator phase and solvent was removed *in vacuo.* The crude product was purified by chromatography on silica gel (40 g cartridge, 10-100% [1:4 MeOH in EtOAc]/*iso*-hexane) to afford 2-(methoxy-d3)-4-(2-methoxyethoxy)-1-nitrobenzene (237 mg, 1.03 mmol, 29%) as a pale yellow powder.

LCMS (Method E): m/z 231.1 [M+H]+ at 1.23 min.

¹H NMR (400 MHz, DMSO-d6) δ 7.95 (d, J = 9.1 Hz, 1H), 6.81 (d, J = 2.5 Hz, 1H), 6.67 (dd, J = 9.1,2.5 Hz, 1H), 4.28 - 4.21 (m, 2H), 3.71 - 3.64 (m, 2H), 3.31 (s, 3H).

Preparation of compound 5 **(2-(methoxy-d3)-4-(2-methoxyethoxy)aniline):**
Using General Procedure F afforded the title compound (134 mg, 68%) as a dark red oil.

UPLC (Method A): m/z 201.5 [M+H]+ at 0.46 min.

1H NMR (400 MHz, DMSO-d6) δ 6.53 (d, J = 8.4 Hz, 1H), 6.45 (d, J = 2.6 Hz, 1H), 6.28 (dd, J = 8.4, 2.6 Hz, 1H), 4.23 (br s, 2H), 3.99 - 3.92 (m, 2H), 3.63 - 3.56 (m, 2H), 3.29 (s, 3H).

Preparation of compound 6 **(4-(benzyloxy)-2-cyclopropoxy-1-nitrobenzene):**
To a suspension of 4-(benzyloxy)-2-fluoro-1-nitrobenzene (1.0 eq) and Cs2CO3 (1.6 eq) in DMF (0.3 M) was added cyclopropanol (1.5 eq) and the reaction mixture was heated to 80 °C for 18 h. The reaction mixture was cooled to room temperature and then partitioned between EtOAc (40 mL) and water (30 mL). The phases were separated and the aqueous phase was extracted with EtOAc (30 mL × 2), The combined organic phases were washed with brine (30 mL), dried over MgSO4, filtered and the solvent was evaporated in vacuo. The crude product was purified by chromatography on silica gel, eluting with 0-100% (25% EtOAc in iso-hexane)/iso-hexane, to afford the title compound (192 mg, 81 %) as a yellow oil.

UPLC (Method A): m/z Not observed [M+H]+ at 2.13 min.

1H NMR (400 MHz, DMSO-d6) δ 7.96 (d, J = 9.1 Hz, 1H), 7.52 - 7.46 (m, 2H), 7.45 - 7.39 (m, 2H), 7.39 - 7.34 (m, 1H), 7.15 (d, J = 2.6 Hz, 1H), 6.79 (dd, J = 9.1, 2.6 Hz, 1H), 5.26 (s, 2H), 4.11 - 4.04 (m, 1H), 0.90 - 0.81 (m, 2H), 0.74 - 0.66 (m, 2H).

Preparation of compound 7 **(4-(benzyloxy)-2-cyclopropoxy-1-nitrobenzene):**
Using General Procedure F afforded the title compound (91 mg, 78%) as a black solid.

UPLC (Method B): m/z 164.0 [M+H]+ at 0.74 min.

1H NMR (400 MHz, DMSO-d6) δ 8.46 (s, 1H), 6.59 (d, J = 2.5 Hz, 1H), 6.43 (d, J = 8.3 Hz, 1H), 6.13 (dd, J = 8.3, 2.5 Hz, 1H), 3.95 (s, 2H), 3.72 (tt, J = 6.1, 3.0 Hz, 1H), 0.76 - 0.68 (m, 2H), 0.68 - 0.60 (m, 2H).

Preparation of compound 8 **(4-(benzyloxy)-2-(methoxy-d3)-1-nitrobenzene):**
To a solution of 4-(benzyloxy)-2-fluoro-1-nitrobenzene (1.0 eq) in THF (3 mL) was added methan-d3-ol (3.5 eq) and then NaOH (1.2 eq). The reaction mixture was stirred at room temperature for 18 h. The reaction mixture was partitioned between EtOAc (30 mL) and water (30 mL) and the phases were separated. The aqueous phase was extracted with EtOAc (30 mL × 2) and the combined organic phases were washed with brine (30 mL), dried over MgSO4, filtered and the solvent was evaporated in vacuo to afford the title compound (312 mg, 98%) as a pale orange solid.

UPLC (Method A): m/z 263.2 [M+H]+ at 1.97 min.

1H NMR (400 MHz, DMSO-d6) δ 7.96 (d, J = 9.1 Hz, 1H), 7.52 - 7.44 (m, 2H), 7.44 - 7.40 (m, 2H), 7.40 - 7.32 (m, 1H), 6.91 (d, J = 2.5 Hz, 1H), 6.75 (dd, J = 9.1, 2.5 Hz, 1H), 5.25 (s, 2H).

Preparation of compound 9 **(4-amino-3-(methoxy-d3)phenol):**
Using General Procedure F afforded the title compound (165 mg, 88%) as a dark brown solid.

UPLC (Method B): m/z Not observed [M+H]+ at 0.70 min.

1H NMR (400 MHz, DMSO-d6) δ 8.44 (s, 1H), 6.43 (d, J = 8.3 Hz, 1H), 6.28 (d, J = 2.6 Hz, 1H), 6.11 (dd, J = 8.3, 2.6 Hz, 1H), 4.03 (s, 2H).

Preparation of compound 10 **(4-(cyclopentyloxy)-2-methoxy-1-nitrobenzene):**
To a solution of 4-fluoro-2-methoxy-1-nitrobenzene (1.0 eq) and Cs2CO3 (3.0 eq) in MeCN (0.3 M) was added cyclopentanol (2.5 eq). The reaction mixture was heated to 80 °C and stirred for 18 h. The reaction mixture was cooled to room temperature and partitioned between EtOAc (40 mL) and water (40 mL). The aqueous phase was extracted with EtOAc (20 mL × 3) and the combined organic phases were washed with brine (30 mL), dried over MgSO4, filtered and the solvent was evaporated in vacuo. The crude product was purified by chromatography on silica gel, eluting with 0-20% (25% EtOH in EtOAc)/iso-hexane, to afford the title compound (305 mg, 73%) as a yellow oil.

UPLC (Method A): m/z 238.0 [M+H]+ at 2.00 min.

1H NMR (400 MHz, DMSO-d6) δ 7.94 (d, J = 9.1 Hz, 1H), 6.72 (d, J = 2.5 Hz, 1H), 6.64 (dd, J = 9.1, 2.4 Hz, 1H), 5.03 - 4.97 (m, 1H), 3.91 (s, 3H), 2.03 - 1.91 (m, 2H), 1.79 -1.65 (m, 4H), 1.67 -1.54 (m, 2H).

Preparation of compound 11 **(4-(cyclopentyloxy)-2-methoxyaniline):**
Using General Procedure F, with purification by chromatography on silica gel, eluting with 0-5% (0.7 M NH3/MeOH)/DCM, afforded the title compound (229 mg, 86%) as a brown oil.

UPLC (Method A): m/z 208.2 [M+H]+ at 0.83 min.

1H NMR (400 MHz, DMSO-d6) δ 6.51 (d, J = 8.4 Hz, 1H), 6.39 (d, J = 2.6 Hz, 1H), 6.25 (dd, J = 2.6, 8.4 Hz, 1H), 4.64 (tt, J = 2.5, 5.8 Hz, 1H), 4.20 (s, 2H), 3.72 (s, 3H), 1.89 -1.75 (m, 2H), 1.72 - 1.61 (m, 4H), 1.61 -1.47 (m, 2H).

Preparation of compound 12 **(4-(2-isopropoxyethoxy)-2-methoxy-1-nitrobenzene):**
To a suspension of 4-fluoro-2-methoxy-1-nitrobenzene (1.0 eq) and 2-isopropoxyethan-1-ol (1.5 eq) in MeCN (0.4 M) at 0 °C was added NaOt-Bu, 2.0 M in THF (1.1 eq). The reaction mixture was warmed slowly to room temperature and stirred for 18 h. The reaction mixture was partitioned between EtOAc (40 mL) and water (40 mL) and the phases were separated. The aqueous phase was extracted with EtOAc (30 mL × 2) and the combined organic phases were washed with brine (30 mL), dried over MgSO4, filtered and the solvent was evaporated in vacuo. The crude product was dry-loaded on silica and then purified by chromatography on silica gel, eluting with 0-100% (25% EtOAc in iso-hexane)/iso-hexane, to afford the title compound (578 mg, 70%) as a pale orange oil.

LCMS (Method E): m/z 256.2 [M+H]+ at 1.72 min.

1H NMR (400 MHz, DMSO-d6) δ 7.94 (d; J = 9.1 Hz, 1H), 6.81. (d, J = 2.5 Hz, 1H), 6.67 (dd, J = 9.2, 2.5 Hz, 1H), 4.24 - 4.18 (m, 2H), 3.92 (s, 3H), 3.75 - 3.68 (m, 2H), 3.68 - 3.56 (m, 1H), 1.11 (d, J = 1.2 Hz, 6H).

Preparation of compound 13 **(4-(2-isopropoxyethoxy)-2-methoxyaniline):**
Using General Procedure F afforded the title compound (358 mg, 97%) as a dark red/brown oil.

LCMS (Method E): m/z 226.2 [M+H]+ at 0.41 min.

1H NMR (400 MHz, DMSO-d6) δ 6.53 (d, J = 8.4 Hz, 1H), 6.46 (d, J = 2.6 Hz, 1H), 6.28 (dd, J = 8.4, 2.6 Hz, 1H), 4.23 (s, 2H), 3.95 - 3.89 (m, 2H), 3.73 (s, 3H), 3.64 - 3.61 (m, 2H), 3.61 - 3.57 (m, 1H), 1.10 (d, J = 6.2 Hz, 6H).

Preparation of compound 14 **(4-methoxy-2-(2-methoxyethoxy)-5-nitropyridine):**
To a solution of 2-chloro-4-methoxy-5-nitropyridine (100 mg, 530 µmol) and Cs₂CO₃ (200 mg, 614 µmol) in MeCN (2 mL) at rt was added 2-methoxyethanol (50 µL, 0.63 mmol). The reaction mixture was stirred at 80 °C for 5 h. The reaction mixture was filtered and the solvent was evaporated. The crude product was purified by chromatography on silica gel (0-100% MTBE/*iso*-hexane) to afford 4-methoxy-2-(2-methoxyethoxy)-5-nitrbpyridine (31 mg, 0.13 mmol, 24%) as an orange solid.

UPLC (Method A): m/z 229.1 [M+H]+ at 1.36 min.

1H NMR (400 MHz, Chloroform-d) δ 8.76 (s, 1H), 6.39 (s, 1H), 4.59 - 4.52 (m, 2H), 3.96 (s, 3H), 3.77 - 3.70 (m, 2H), 3.44 (s, 3H).

Preparation of compound 15 **(4-methoxy-6-(2-methoxyethoxy)pyridin-3-amine):**
Using General Procedure F afforded the title compound (24 mg, 0.12 mmol, 87%) as a dark orange oil.

UPLC (Method B): m/z 199.2 [M+H]+ at 0.65 min.

1H NMR (400 MHz, Chloroform-d) δ 7.53 (s, 1H), 6.30 (s, 1H), 4.46 - 4.39 (m, 2H), 3.87 (s, 3H), 3.76 - 3.68 (m, 2H), 3.43 (s, 3H).

Preparation of compound 16 **(2-methoxy-6-(2-methoxyethoxy)-3-nitropyridine):**
To a solution of 6-chloro-2-methoxy-3-nitropyridine (100 mg, 530 µmol) and Cs₂CO₃ (200 mg, 614 µmol) in MeCN (2.0 mL) at rt was added 2-methoxyethanol (50 µL, 0.63 mmol). The reaction mixture was stirred at 80 °C for 5 h.

The reaction mixture was filtered and the solvent was evaporated. The crude product was purified by chromatography on silica gel (0-50% MTBE/iso-hexane) to afford 2-methoxy-6-(2-methoxyethoxy)-3-nitropyridine (65 mg, 0.27 mmol, 51%) as an off-white solid.

UPLC (Method A): m/z Not observed [M+H]+ at 1.48 min.

1H NMR (400 MHz, Chloroform-d) δ 8.35 (d, J = 8.8 Hz, 1H), 6.44 (d, J = 8.8 Hz, 1H), 4.59 - 4.52 (m, 2H), 4.10 (s, 3H), 3.79 - 3.73 (m, 2H), 3.45 (s, 3H).

Preparation of compound 17 **(2-methoxy-6-(2-methoxyethoxy)pyridin-3-amine):**
Using General Procedure F afforded the title compound (55 mg, 0.26 mmol, 95%) as a dark red oil.

UPLC (Method B): m/z 199.2 [M+H]+ at 0.83 min.

1H NMR (400 MHz, Chloroform-d) δ 7.05 (d, J = 8.0 Hz, 1H), 6.24 (d, J = 8.1 Hz, 1H), 4.46 - 4.34 (m, 2H), 3.95 (s, 3H), 3.77 - 3.69 (m, 2H), 3.43 (s, 3H).

Preparation of compound 18 **(4-methoxy-2-(2-methoxyethoxy)-5-nitropyrimidine):**
To a solution of 2-chloro-4-methoxy-5-nitropyrimidine (1.0 eq) and 2-methoxyethanol (1.1 eq) in anhydrous THF (2 mL) at 0 °C was added NaOt-Bu (1.2 eq). The reaction mixture was warmed slowly to room temperature and stirred overnight. The reaction mixture was partitioned between sat. NH4CI (20 mL) and dichloromethane (20 mL). The phases were separated and the aqueous phase was extracted with dichloromethane (10 mL × 2). The combined organic phases were passed through a phase separator and the solvent was evaporated in vacuo. The crude product was purified by chromatography on silica gel, eluted with 0-50% MTBE/iso-hexane, to afford the title compound (112 mg, 71%) as a pale yellow gum.

UPLC (Method A): m/z 200.1 [M-OMe+H]+ at 0.79 min.

1H NMR (400 MHz, Chloroform-d) δ 9.08 (s, 1H), 4.66 - 4.59 (m, 2H), 4.17 (s, 3H), 3.85 - 3.74 (m, 2H), 3.43 (s, 3H).

Preparation of compound 19 **(2-methoxy-6-(2-methoxyethoxy)pyrimidin-3-amine):**
Using General Procedure F afforded the title compound (149 mg, 91%) as a pale brown solid.

UPLC (Method B): m/z 200.1 [M+H]+ at 0.79 min.

1H NMR (400 MHz, DMSO-d6) δ 7.63 (s, 1H), 4.51 (s, 2H), 4.28 - 4.21 (m, 2H), 3.89 (s, 3H), 3.64 - 3.57 (m, 2H), 3.28 (s, 3H).

Preparation of compound 20 **(2-methoxy-3-nitro-6-(2-(trifluoromethoxy)ethoxy)pyridine):**
To a solution of 2-(trifluoromethoxy)ethan-1-ol (72.0 mg554 µmol) in THF (2 mL) at 0 °C was added NaO*t*Bu (61.0 mg, 635 µmol) and 6-chloro-2-methoxy-3-nitropyridine (100 mg, 530 µmol). The reaction mixture was warmed slowly to rt and stirred overnight The reaction mixture was partitioned between sat. NH₄Cl (20 mL) and DCM (20 mL). The phases were separated and the aqueous phase was extracted with DCM (10 mL × 2). The combined organic phases were filtered through a phase separator and the solvent was evaporated *in vacuo.* The crude product was purified by chromatography on silica gel (0-100% DCM/i*so*-hexane) to afford 2-methoxy-3-nitro-6,(2-(trifluoromethoxy)ethoxy)pyridine (112 mg, 0.38 mmol, 71%) as a pale yellow gum.

UPLC (Method A): m/z Not observed [M+H]+ at 1.91 min.

1H NMR (400 MHz, Chloroform-d) δ 8.38 (d, J = 8.8 Hz, 1H), 6.45 (d, J = 8.8 Hz, 1H), 4.68 - 4.61 (m, 2H), 4.35 - 4.28 (m, 2H), 4.11 (s, 3H).

Preparation of compound 21 **(2-methoxy-6-(2-(trifluoromethoxy)ethoxy)pyridin-3-amine):**
Using General Procedure F afforded the title compound (97 mg, 0.37 mmol, 95%) as a dark brown gum.

UPLC (Method A): m/z 253.1 [M+H]+ at 1.26 min.

1H NMR (400 MHz, DMSO-d6) δ 6.96 (d, J = 8.0 Hz, 1H), 6.20 (d, J = 8.0 Hz, 1H), 4.45 - 4.32 (m, 6H), 3.85 (s, 3H).

Preparation of compound 22 **(2-methoxy-3-nitro-6-(2-(trifluoromethoxy)ethoxy)pyrimidine):**
To a solution of 2-chioro-4-methoxy-5-nitropynmidine (1.0 eq) and 2-(trifluoromethoxy)ethan-1-ol (1.1 eq) in anhydrous THF (0.3 M) at 0 °C was added NaOt-Bu (1.2 eq). The reaction mixture was warmed slowly to room temperature and stirred for 18 h. The reaction mixture was partitioned between sat. NH4Cl (20 mL) and dichloromethane (20 mL). The phases were separated and the aqueous phase was extracted with dichloromethane. (10 mL × 2). The combined organic phases were passed through a phase separator and the solvent was evaporated in vacuo. The crude product was purified by chromatography on silica gel, eluted with 0-50% MTBE/iso-hexane, to afford the title compound (112 mg, 71%) as a pale yellow gum.

UPLC (Method A): m/z Not observed [M+H]+ at 1.36 min.

1H NMR (400 MHz, DMSQ-d6) δ 9.18 (s, 1H), 4.72 - 4.66 (m, 2H), 4.51 - 4.44 (m, 2H), 4.10 (s, 3H).

Preparation of compound 23 **(2-methoxy-6-(2-(trifluoromethoxy)ethoxy)pyrimidin-3-amine):**
Using General Procedure F afforded the title compound (165 mg, 91%) as a dark brown oil.

UPLC (Method B): m/z 254.2 [M+H]+ at 1.05 min.

1H NMR (400 MHz, DMSO-d6) δ 7.64 (s, 1H), 4.60 - 4.55 (m, 2H), 4.46 - 4.32 (m, 4H), 3.91 (s, 3H).

Preparation of compound 24 **(4-(3-methoxy-4-nitrophenyl)-1H-pyrazole):**

A vial was charged with PdOAc₂ (8 mg, 37 µmol), tricyclohexylphosphonium tetrafluoroborate (26 mg, 69.8 µmol), 4-bromo-2-methoxy-1-nitrobenzene (200 mg, 863.3 µmol), *tert*-butyl 4-(4,4,5,5-tetramethyl-1,3,2-dioxaborolan-2-yl)-1H-pyrazole-1-carboxylate (281 mg, 955 µmol) and K₂CO₃(476.7 mg, 3.449 mmol). The mixture was degassed with N₂ for 5 min. 1,4-Dioxane (4.0 mL) and water (0.20 mL) were added, and the suspension was degassed again with N₂ for 5 min. The reaction mixture was heated to 90 °C for 20 h. The reaction mixture was cooled to rt, diluted with EtOAc (20 mL) and filtered through a pad of celite. The filtrate was concentrated *in vacuo.* The crude product was purified by chromatography on silica gel (0-100% EtOAc in heptane) to afford 4-(3-methoxy-4-nitrophenyl)-1H-pyrazole (107 mg, 0.47 mmol, 54%) as a yellow solid.

UPLC (Method A): m/z 220.1 [M+H]+ at 1.32 min. 1H NMR (400 MHz, DMSO-d6) δ 13.18 (s, 1H), 8.30 (s, 2H), 7.90 (d, J = 8.5 Hz, 1H), 7.52 (d, J = 1.7 Hz, 1H), 7.36 (dd, J = 8.5, 1.7 Hz, 1H), 3.99 (s, 3H).

Preparation of compound 25 **(2-methoxy-4-(1H-pyrazol-4-yl)aniline):**
Using General Procedure F afforded the title compound (88 mg, 393 µmol, 86%) as an off-white solid.

UPLC (Method B): m/z 190.2 [M+H]+ at 0.70 min.

1H NMR (400 MHz, DMSO-d6) δ 12.70 (s, 1H), 7.96 (s, 1H), 7.76 (s, 1H), 7.01 (d, J = 1.8 Hz, 1H), 6.91 (dd, J = 7.9, 1.8 Hz, 1H), 6.60 (d, J = 7.9 Hz, 1H), 4.62 (s, 2H), 3.81 (s, 3H).

Preparation of compound 26 **(2-methoxy-3-nitro-6-(1H-pyrazol-4-yl)pyridine):**
A reaction vessel was charged with 6-chloro-2-methoxy-3-nitropyridine (1.0 eq), Pd(dppf)Cl2 (5 mol%) and K3PO4 (3.8 eq). The mixture was evacuated and purged with nitroden and then a 3:1 mixture of 1,4-dioxane/water was added. The reaction mixture was evacuated and purged with nitrogen three times and then heated to 90 °C and stirred under nitrogen for 18 h. The reaction mixture was cooled to room temperature and then water (20 mL) was added. The resulting precipitate was collected by vacuum filtration to afford the title compound (193 mg, 78%) as a brown/grey solid.

LCMS (Method E): m/z 221.0 [M+H]+ at 1.30 min.

1H NMR (400 MHz, DMSO-d6) δ 13.35 (s, 1H), 8.54 (s, 1H), 8.44 (d, J = 8.3 Hz, 1H), 8.20 (s, 1H), 7.49 (d, J = 8.3 Hz, 1H), 4.10 (s, 3H).

Preparation of compound 27 **(2-methoxy-6-(1H-pyrazol-4-yl)pyridin-3-amine):**
Using General Procedure F afforded the title compound (134 mg, 91%) as a brown solid.

LCMS (Method E): m/z 191.2 [M+H]+ at 0.35 min.

1H NMR (400 MHz, DMSO-d6) δ 12.76 (s, 1H), 8.01 (s, 1H), 7.84 (s, 1H), 7.00 (d, J = 7.7 Hz, 1H), 6.86 (d, J = 7.7 Hz, 1H), 4.80 (s, 2H), 3.91 (s, 3H).

Preparation of compound 28 **(4-(2-(difluoromethoxy)ethoxy)-2-methoxy-1-nitrobenzene):**
In a sealed tube, to a stirred solution of 2-(3-methoxy-4-nitrophenoxy)ethan-1-ol (884 mg, 4.06 mmol) in a mixture of DCM (3 mL) and water (3 mL) at 0 °C was added KHF₂ (3.17 g, 40.6 mmol) and the mixture was stirred for 10 min. (Bromodifluoromethyl)trimethylsilane (3.8 mL, 24 mmol) was added and the reaction mixture was stirred for 2 h at 0 °C before being warmed to rt and stirred for 3 days. Sat. aq. NaHCO₃ (20 mL) was added and the mixture was extracted with DCM (3 × 40 mL), the combined organic phase were washed with brine (20 mL), filtered through a separator phase and solvent was removed *in vacuo* to afford 4-(2-(difluoromethoxy)ethoxy)-2-methoxy-1-nitrobenzene (1.05 g, 3.6 mmol, 88%) as a pale brown powder.

UPLC (Method A): m/z 264.1 [M+H]+ at 1.74 min.

1H NMR (400 MHz, DMSO-d6) δ 7.96 (d, J = 9.1 Hz, 1H), 6.84 (d, J = 2.5 Hz, 1H), 6.76 (t, J = 75.6 Hz, 1H), 6.69 (dd, J = 9.1, 2.5 Hz, 1H), 4.37 - 4.31 (m, 2H), 4.23 - 4.16 (m, 2H), 3.93 (s, 3H).

Preparation of compound 29 **(4-(2-(difluoromethoxy)ethoxy)-2-methoxyaniline):**
Using General Procedure F afforded the title compound (893 mg, 3.83 mmol, 100%) as a dark black-red oil.

UPLC (Method B): m/z 234.2 [M+H]+ at 1.06 min.

1H NMR (400 MHz, DMSO-d6) δ 6.94 - 6.51 (m, 2H), 6.48 (d, J = 2.6 Hz, 1H), 6.31 (dd, J = 8.4, 2.6 Hz, 1H), 4.36 (s, 2H), 4.12 - 4.07 (m, 2H), 4.07 - 4.01 (m, 2H), 3.74 (s, 3H).

Preparation of compound 30 **(2-fluoro-4-(2-methoxyethoxy)-1-nitrobenzene):**
To a solution of 3-fluoro-4-nitrophenol (2.00 g, 12.73 mmol) in DMF (20 mL) were added 1-bromo-2-methoxyethane (5.98 mL, 63.65 mmol) and K₂CO₃ (3.52 mg, 25.46 mmol). The mixture was allowed to stir at 70°C for 4 hours. The solvents were evaporated and the product was extracted with EtOAc, washed with water (3 times) and brine (2 times). The organic phase was dried over MgSO₄, filtered and evaporated. The crude product was purified by flash column chromatography to afford 2-fluoro-4-(2-methoxyethoxy)-1-nitrobenzene (1.95 g, 71%) as a white powder. ¹H NMR (400 MHz, Chloroform-d) δ 8.14 -8.05 (m, 1H), 6.84 -6.73 (m, 2H), 4.23 -4.16 (m, 2H), 3.81 -3.74 (m, 2H), 3.45 (s, 3H).

Preparation of compound 31 **(4-(5-(2-methoxyethoxy)-2-nitrophenyl)morpholine):**
A solution of morpholine (240 µL, 2.79 mmol) in ethanol (2 mL) was slowly added to a solution of compound 18 (200 mg, 0.92 mmol) at 0°C. The reaction was stirred for 30 min at 50°C. After cooling to rt, a solution of NaHCO₃ was added. The product was extracted with DCM. The organic layer was washed with water and brine, dried over MgSO₄, filtered and concentrated under reduce pressure to afford 4-(5-(2-methoxyethoxy)-2-nitrophenyl)morpholine (278 mg, 100%) as a yellow solid.

¹H NMR (400 MHz, Chloroform-d) δ 7.98 (d, J= 9.1 Hz, 1H), 6.64 (d, J= 2.6 Hz, 1H), 6.57 (dd, J= 9.1, 2.6 Hz, 1H), 4.25 -4.13 (m, 2H), 3.87 (ddd, J= 6.3 Hz, 4.6 Hz, 2.9 Hz, 4H), 3.79 -3.73 (m, 2H), 3.46 (s, 3H), 3.06 (ddd, J= 6.3 Hz, 4.6 Hz, 2.9 Hz, 4H).

Preparation of compound 32 **(4-(2-methoxyethoxy)-2-morpholinoaniline):**
Using General Procedure F afforded the title compound (176 mg, 98%) as a purple oil.

¹H NMR (400 MHz, Chloroform-d) δ 6.68 (d, J= 2.7 Hz, 1H), 6.66 (d, J= 8.5 Hz, 1H), 6.55 (dd, J= 8.5, 2.7 Hz, 1H), 4.10 -4.02 (m, 2H), 3.83 (ddd, J= 6.3, 4.6, 2.9 Hz, 4H), 3.75 -3.69 (m, 2H), 3.45 (s, 3H), 2.90 (ddd, J= 6.3, 4.6 Hz, 2.9 Hz, 4H).

Preparation of compound 33 **(7-bromo-N-(2,4-dimethoxyphenyl)quinolin-4-amine, HCl):**

To a solution of 7-bromo-4-chloroquinoline (100 mg, 412 µmol) in EtOH (2.0 mL) at rt was added 2,4-dimethoxyaniline (70.0 mg, 457 µmol). The reaction mixture was stirred at 80 °C overnight. The reaction mixture was diluted with acetone (10 mL) and the precipitate was collected by filtration, washing with acetone (10 mL × 3). The product was dried in a vacuum desiccator overnight to afford 7-bromo-N-(2,4-dimethoxyphenyl)quinolin-4-amine, HCl (135 mg, 0.34 mmol, 82 %) as a dark yellow solid.

UPLC (Method A): m/z 359.2/361.2 [M+H]+ at 0.84 min.

1H NMR (400 MHz, DMSO-d6) δ 14.23 (br s, 1H), 10.65 (s, 1H), 8.65 (d, J = 9.1 Hz, 1H), 8.44 (d, J = 7.0 Hz, 1H), 8.22 (d, J = 2.0 Hz, 1H), 7.97 (dd, J = 9.0, 2.0 Hz, 1H), 7.31 (d, J = 8.7 Hz, 1H), 6.83 (d, J = 2.6 Hz, 1H), 6.70 (dd, J = 8.6, 2.6 Hz, 1H), 6.31 (d, J = 7.0 Hz, 1H), 3.85 (s, 3H), 3.78 (s, 3H).

Preparation of compound 34 **(7-bromo-N-(2-methoxy-4-(2-methoxyethoxy)phenyl)quinolin-4-amine):**
A mixture of 4-((7-bromoquinolin-4-yl)amino)-3-methoxyphenol, HCl (1.87 g, 4.65 mmol), 2-methoxyethyl 4-methylbenzenesulfonate (1.02 mL, 5.35 mmol) and cesium carbonate (3.18 g, 9.77 mmol) in acetone (30.0 mL) was stirred at 60 °C for 18 h. The solvent was removed *in vacuo* and the residue was triturated from water (50 mL) and the solid collected by filtration. The solid was then triturated from *iso*-hexane (30 mL) then collected again by filtration and dried in the vac oven (40 °C) to give 7-bromo-N-(2-methoxy-4-(2-methoxyethoxy)phenyl)quinolin-4-amine (2.00 g, 100 %) as a brown solid.

UPLC (Method A): m/z 403.1/405.1 [M+H]+ at 1.18 min.

1H NMR (400 MHz, DMSO-d6) δ 8.61 (s, 1H), 8.37 (d, J = 9.0 Hz, 1H), 8.30 (d, J = 5.4 Hz, 1H), 8.00 (d, J = 2.1 Hz, 1H), 7.61 (dd, J = 9.0, 2.1 Hz, 1H), 7.18 (d, J = 8.5 Hz, 1H), 6.75 (d, J = 2.6 Hz, 1H), 6.61 (dd, J = 8.6, 2.7 Hz, 1H), 6.10 (d, J = 5.4 Hz, 1H), 4.18 - 4.12 (m, 2H), 3.73 (s, 3H), 3.71 - 3.66 (m, 2H), 3.33 (s, 3H).

Preparation of compound 35 **(7-bromo-N-(2-methoxy-4-(2-(trifluoromethoxy)ethoxy)phenyl)quinolin-4-amine, HCl):**
To a stirred solution of 4-((7-bromoquinolin-4-yl)amino)-3-methoxyphenol, HCl (750 mg, 1.97 mmol) in acetone (10.0 mL) at room temperature was added Cs₂CO₃ (1.50 g, 4.60 mmol) and 1-bromo-2-(trifluoromethoxy)ethane (465 mg, 2.41 mmol). The reaction mixture was stirred at 60 °C for 18 h. The reaction mixture was cooled to rt, water (50 mL) was added and the resulting precipitate was collected by filtration, washing with water (3 × 20 mL) and then *iso-*hexane (2 × 20 mL). The product was dried in a *vacuum* desiccator overnight to afford 7-bromo-N-(2-methoxy-4-(2-(trifluoromethoxy)ethoxy)phenyl)quinolin-4-amine (725 mg, 77 %) as a pale yellow solid.

UPLC (Method A): m/z 457.1/459.1 [M+H]+ at 1.41 min.

1H NMR (400 MHz, DMSO-d6) δ 14.72 (s, 1H), 10.80 (s, 1H), 8.73 (d, J = 9.1 Hz, 1H), 8.44 (d, J = 7.0 Hz, 1H), 8.32 (d, J = 1.9 Hz, 1H), 7.96 (dd, J = 9.1, 1.9 Hz, 1H), 7.32 (d, J = 8.6 Hz, 1H), 6.87 (d, J = 2.5 Hz, 1H), 6.73 (dd, J = 8.6, 2.6 Hz, 1H), 6.30 (d, J = 7.0 Hz, 1H), 4.50 - 4.41 (m, 2H), 4.41 - 4.30 (m, 2H), 3.79 (s, 3H).

Preparation of compound 36 **(4-((7-bromoquinolin-4-yl)amino)-3-mothoxyphenol, HCl):**
A solution of 7-bromo-4-chtoroquinoiine (1.47 g, 6.04 mmol), 4-amino-3-methoxyphenol (801 mg, 5.76 mmol) and HCl (4 M in dioxane) (2.16 mL, 8.63 mmol) in propan-2-ol (15.0 mL) was stirred at 80 °C for 18 hours. The reaction mixture was cooled to room temperature and the solvent was evaporated and the residue was triturated from acetone (30 ml), cooled to 0 °C then the precipitate was collected, by filtration to give 4-((7-bromoquinolin-4-yl)amino)-3-methoxyphenol, HCl (1.87 g, 4.7 mmol, 81 %) as a green solid.

UPLC (Method A): m/z 345.0/347.0 [M+H]+ at 1.05 min.

1H NMR (400 MHz, DMSO-d6) δ 14.44 (s, 1H), 10.66 (s, 1H), 9.99 (s, 1H), 8.67 (d, J = 9.1 Hz, 1H), 8.43 (d, J = 7.0 Hz, 1H), 8.27 (d, J = 2.0 Hz, 1H), 7.96 (dd, J = 9.1, 2.0 Hz, 1H), 7.16 (d, J = 8.5 Hz, 1H), 6.66 (d, J = 2.5 Hz, 1H), 6.53 (dd, J = 8.5, 2.4 Hz, 1H), 6.31 (d, J = 7.0 Hz, 1H), 3.72 (s, 3H).

Preparation of compound 37 **(4-((7-methylquinolin-4-yl)amino)-3-methoxyphenol, HCl):**
Using General Procedure A-2, with purification by chromatography on silica gel, eluting with, 0-100% (10% 0.7 M NH3 in DCM)/DCM, afforded the title compound (30 mg, 64%) as a pink/brown solid.

UPLC (Method A): m/z 284.2 [M+H]+ at 0.98 min.

1H NMR (400 MHz, DMSO-d6) δ 9.56 (s, 1H), 8.31 - 8.17 (m, 3H), 7.59 (s, 1H), 7.28 (dd, J = 8.6,1.8 Hz, 1H), 7.04 (d, J = 8.4 Hz, 1H), 6.56 (d, J = 2.5 Hz, 1H), 6.43 (dd, J = 8.4, 2.5 Hz, 1H), 6.01 (d, J = 5.3 Hz, 1H), 3.67 (s, 3H), 2.47 (s, 3H). HCl peak not observed.

Preparation of compound 38 **(3-(methoxy-d3)-4-((7-methylquinolin-4-yl)amino)phenol, HCl):**
Using General Procedure B, with purification by chromatography on silica gel, eluting with, 0-100% (10% 0.7 M NH3 in DCM)/DCM, afforded the title compound (30 mg, 64%) as a pink/brown solid.

UPLC (Method A): m/z 284.2 [M+H]+ at 0.98 min.

No NMR data obtained.

Preparation of compound 39 **(4-((7-(difluoromethyl)qulnolin-4-yl)amino)-3-methoxyphenol, HCl):**
Using General Procedure B afforded the title compound (26 mg, 69%) as a dark yellow solid.

UPLC (Method A): m/z 317.1 [M+H]+ at 1.64 min.

1H NMR (400 MHz, DMSO-d6) δ 14.48 (s, 1H), 10.69 (s, 1H), 9.98 (s, 1H), 8.86 (d, J = 8.8 Hz, 1H), 8.50 (d, J = 7.0 Hz, 1H), 8.21 (s, 1H), 7.96-7.89 (m, 1H), 7.35 (t, J = 55.2 Hz,1H), 7.17 (d, J = 8.5 Hz, 1H), 6.67 (d, J = 2.5 Hz, 1H), 6.54 (dd, J = 8.4, 2.5 Hz, 1H), 6.37 (d, J = 7.1 Hz, 1H), 3.73 (s, 3H).

Preparation of compound 40 **(3-cyclopropoxy-4-((7-(difluoromethyl)quinolin-4-yl)amino)phenol, HCl):**
Using General Procedure B, with purification by chromatography on silica gel, eluting with, 0-10% (0.7 M NH3/MeOH)/DCM, afforded the title compound (21 mg, 47%) as a dark brown solid.

UPLC (Method A): m/z 343.2 [M+H]+ at 1.13 min.

No NMR data obtained.

Preparation of compound 41 **(4-((7-(difluoromethyl)quinolin-4-yl)amino)-3-(methoxy-d3)phenol, HCl):**
Using General Procedure B, with purification by chromatography on silica gel, eluting with, 0-10% (0.7 M NH3/MeOH)/DCM, afforded the title compound (28 mg, 66%) as a pale green solid.

UPLC (Method A): m/z 320.2 [M+H]+ at 0.95 min.

No NMR data obtained.

Preparation of compound 42 **(3-cyclopropoxy-4-((7-methylquinolin-4-yl)amino)phenol, HCl):**
Using General Procedure B, with purification by chromatography on silica gel, eluting with, 0-10% (0.7 M NH3/MeOH)/DCM, afforded the title compound (17 mg, 35%) as a dark brown solid.

UPLC (Method A): m/z 307.2 [M+H]+ at 1.13 min.

No NMR data obtained.

### Preparation of the examples

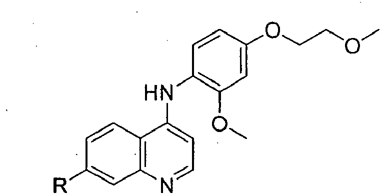

| **Example** | **Name** | **R** | **General Procedure** | **¹H NMR δ (400 MHz)** | **LCMS (ES+) *m*/*z*** |
|---|---|---|---|---|---|
| **1** | N-(2-methoxy-4-(2-methoxyethoxy )phenyl)-7-phenylquinolin-4-amine | 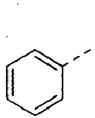 | A from compound 1 | ¹H NMR (400 MHz, DMSO-*d*6) δ 14.38 (br s, 1H), 10.60 (s, 1H), 8.81 (d, J = 8.9 Hz, 1H), 8.46 (d, J = 7.0 Hz, 1H), 8.27 (d, J = 1.8 Hz, 1H), 8.13 (dd, J = 8.9,1.8 Hz, 1H), 7.90 - 7.83 (m, 2H), 7.65 - 7.57 (m, 2H), 7.57 - 7.49 (m, 1H), 7.32 (d, J = 8.6 Hz, 1H), 6.86 (d, J = 2.6 Hz, 1H), 6.72 (dd, J .= 8.6, 2.6 Hz, 1H), 6.30 (d, J = 7.0 Hz, 1H), 4.23 - 4.16 (m, 2H), 3.79 (s, 3H), 3.74 - 3.67 (m, 2H), 3.34 (s, 3H). | (Method C) *m*/*z* 401.2 [M+H]+ at 3.12 min |
| **2** | N-(2-methoxy-4-(2-methoxyethoxy )phenyl)-7-(pyrazin-2-yl)quinolin-4-amine | 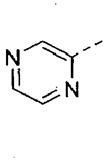 | G from compound 34 | ¹H NMR (400 MHz, DMSO-*d*₆) δ 9.49 - 9.44 (m, 1H), 8.82 - 8.77 (m, 1H), 8.70 - 8.65 (m, 1H), 8.64 - 8.53 (m, 2H), 8.41 - 8.35 (m, 1H), 8.28 - 8.18 (m, 1H), 7.25 - 7.18(m, 1H), 6.79 - 6.74 (m, 1H), 6.66 -. 6.59 (m, 1H), 6.18 - 6.11 (m, 1H), 4.18 - 4.12 (m, 2H), 3.75 (s, 3H), 3.72 - 3.66 (m, 2H), 3.34 (s, 3H) ppm. | (Method C) *m*/*z* 403.2 [M+H]⁺ at 2.19 min |
| **3** | 7-methoxy-N-(2-methoxy-4-(2-methoxyethoxy )phenyl)quinoli n-4-amine | 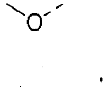 | A from 4-chloro-7-methoxyqui noline | ¹H NMR (400 MHz, DMSO-*d*₆) δ 14.09 (br s, 1H), 10.40 (s, 1H), 8.67 - 8.60 (m, 1H), 8.33 (d, J = 7.0 Hz, 1H), 7.44 - 7.36 (m, 2H), 7.28 (d, J = 8.6 Hz, 1H), 6.84 (d, J = 2.6 Hz; 1H), 6.69 (dd, J = 8.6, 2.6 Hz, 1H), 6.18 (d, J = 7.0 Hz, 1H), 4.21 -4.15 (m, 2H), 3.96 (s, 3H), 3.77 (s, 3H), 3.73 - 3.66 (m, 2H), 3.34 (s, 3H). | (Method C) *m*/*z* 355.2 [M+H]+ at 2.25 min |
| **4** | N-(2-methoxy-4-(2-methoxyethoxy )phenyl)-7-(1-methyl-1H-pyrazol-4-yl)quinolin-4-amine | 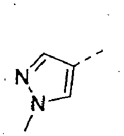 | C from compound 34 | ¹H NMR (400 MHz, DMSO-*d*₆) δ 8.44 - 8.35 (m, 2H), 8.35 - 8.32 (m, 1H), 8.26 (d, J = 5.3 Hz, 1H), 8.07 - 8.02 (m, 1H), 7.97 (d, J = 1.8 Hz, 1H), 7.71 (dd, J = 8.8,1.9 Hz, 1H), 7.18 (d, J = 8.5 Hz, 1H), 6.75 (d, J = 2.6 Hz, 1H), 6.61 (dd, J = 8.6, 2.6 Hz, 1H), 6.03 (d, J = 5.3 Hz, 1H), 4.18 - 4.11 (m, 2H), 3.90 (s, 3H), 3.74 (s, 3H), 3.71 - 3.67 (m, 2H), 3.34 (s, 3H) ppm. | C) (Method *m*/*z* 405.2 [M+H]⁺ at 2.88 min |
| **5** | N-(2-methoxy-4-(2-methoxyethoxy )phenyl)-7-(1-methyl-1,2,3,6-tetrahydropyridi n-4-yl)quinolin-4-amine | 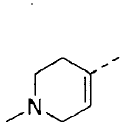 | C from compound 34 | ¹H NMR (400 MHz, DMSO-*d*₆) δ 8.40 (s, 1H), 8.32 (d, J = 8.9 Hz, 1H), 8.27 (d, J = 5.3 Hz, 1H), 7.76 -7.71 (m, 1H), 7.65 (dd, J = 8.9, 2.0 Hz, 1H), 7.17 (d, J = 8.5 Hz, 1H), 6.75 (d, J = 2.6 Hz, 1H), 6.61 (dd, J = 8.5, 2.6 Hz, 1H), 6.46 - 6.40 (m, 1H), 6.04 (d, J = 5.3 Hz, 1H), 4.20 - 4.11 (m, 2H), 3.73 (s, 3H), 3.72 - 3.66 (m, 2H), 3.34 (s, 3H), 3.11 - 3.05 (m, 2H), 2.64 - 2.59 (m, 4H), 2.31 (s, 3H). | (Method D) *m*/*z* 420.3 [M+H]⁺ at 3.73 min |
| **6** | N-(2-methoxy-4-(2-methoxyethoxy )phenyl)-7-(4-methylpiperazin -1-yl)quinolin-4-amine | 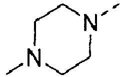 | D from compound 34 | ¹H NMR (400 MHz, DMSO-*d*₆) δ 10.08 (br s, 1H), 8.46 (d, J = 9.5 Hz, 1H); 8.20 (d, J = 7.0 Hz, 1H), 7.55 (dd, J = 9.6, 2.4 Hz, 1H), 7.25 (d, J = 8.6 Hz, 1H), 7.07 (d, J = 2.5 Hz, 1H), 6.82 (d, J = 2.6 Hz, 1H), 6.67 (dd, J = 8.7, 2.6 Hz, 1H), 6.04 (d, J = 7.0 Hz, 1H), 4.21 - 4.14 (m, 2H), 3.77 (s, 3H), 3.73 - 3.66 (m, 2H), 3.52 - 3.45 (m, 4H), 3.33 (s, 3H), 2.65 - 2.58 (m, 4H), 2.34 (s, 3H) ppm. | (Method D) *m*/*z* 423.0 [M+H]+ at 3.25 min |
| **7** | 7-(4,4-difluoropiperidi n-1-yl)-N-(2-methoxy-4-(2-methoxyethoxy )phenyl)quinoli n-4-amine | 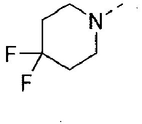 | D from compound 34 | ¹H NMR (400 MHz, DMSO-*d*₆) δ 8.28 - 8.21. (m, 2H), 8.16 (d, J = 5.3 Hz, 1H), 7.34 (dd, J = 9.3, 2.6 Hz, 1H), 7.18 - 7.12 (m, 2H), 6.73 (d, J = 2.6 Hz, 1H), 6.60 (dd, J = 8.5, 2.6 Hz, 1H), 5.95 - 5.89 (m, 1H), 4.17 -4.11 (m, 2H), 3.72 (s, 3H), 3.71 - 3.66 (m, 2H), 3.52 - 3.46 (m, 4H), 3.33 (s, 3H), 2.17 - 2.02 (m, 4H) ppm. | (Method C) *m*/*z* 444.3 [M+H]+ at 3.02 min |
| **8** | 7-(3,3-difluoroazetidin -1-yl)-N-(2-methoxy-4-(2-methoxyethoxy )phenyl)quinoli n-4-amine | 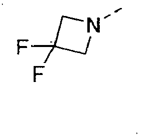 | D from compound 34 | ¹H NMR (400 MHz, DMSO-*d*₆) δ 8.33 - 8.26 (m, 2H), 8.17 (d, J = 5.4 Hz, 1H), 7.16 (d, J = 8.5 Hz, 1H), 6.87 (dd, J = 9.0, 2.5 Hz, 1H), 6.81 (d, J = 2.4 Hz, 1H), 6.74 (d, J = 2.6 Hz, 1H), 6.60 (dd, J = 8.6, 2.7 Hz, 1H), 5.93 (d, J = 5.3 Hz, 1H), 4.40 (t, J = 12.3 Hz, 4H), 4.1.7 -4.10 (m, 2H), 3.73 (s, 3H), 3.71 - 3.65 (m, 2H), 3.33 (s, 3H) ppm. | (Method C) *m*/*z* 416.2 [M+H]+ at 2.69 min |
| **9** | 7-(difluoromethyl) -N-(2-methoxy-4-(2-methoxyethoxy )phenyl)quinoli n-4-amine | 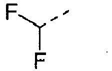 | A from compound 2 | ¹H NMR (400 MHz, DMSO-*d*₆) δ 14.90 (s, 1H), 10.82 (s, 1H), 8.93 (d, J = 8.8 Hz, 1H), 8.51 (d, J = 7.0 Hz, 1H), 8.31 - 8.26 (m, 1H), 7.96 - 7.88 (m, 1H), 7.36 (t, J = 55.2 Hz, 1H), 7.31 (d, J = 8.6 Hz, 1H), 6.85 (d, J = 2.6 Hz, 1H), 6.71 (dd, J = 8.7, 2.6 Hz, 1H), 6.35 (d, J = 7.0 Hz, 1H), 4.22 - 4.16 (m, 2H), 3.78 (s, 3H), 3.73 - 3.67 (m, 2H), 3.34 (s, 3H). | (Method C) *m*/*z* 375.2 [M+H]+ at 2.17 min |
| **10** | N-(2-methoxy-4-(2-methoxyethoxy)phenyl)-7-methylquinolin-4-amine | 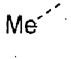 | A from 4-chloro-7-methylquinoline | ¹H NMR (400 MHz, DMSO-*d*₆) δ 14.35 (br s, 1H), 10.49 (s, 1H), 8.63 (d, J = 8.7 Hz, 1H), 8.37 (d, J = 7.0 Hz, 1H), 7.83 - 7.78 (m, 1H), 7.62 (dd, J = 8.8, 1.7 Hz, 1H), 7.29 (d, J = 8.6 Hz, 1H), 6.84 (d, J = 2.6 Hz, 1H), 6.70 (dd, J = 8.6, 2.6 Hz, 1H), 6.24 (d, J = 7.0 Hz, 1H), 4.22 - 4.15 (m, 2H), 3.77 (s, 3H), 3.73 - 3.66 (m, 2H), 3.34 (s, 3H), 2.56 (s, 3H). | (Method C) *m*/*z* 339.1 [M+H]⁺ at 2.28 min |
| **11** | N-(2-methoxy- . 4-(2-methoxyethoxy )phenyl)-7-(1-methylpiperidin -4-yl)quinolin-4-amine | 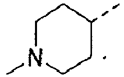 | C from compound 34 then F* | ¹H NMR (400 MHz, DMSO-*d*₆) δ 8.35 (s, 1H), 8:31 (d, J = 8.7 Hz, 1H), 8.25 (d, J = 5.3 Hz, 1H), 7.61 (d, J = 1.8 Hz, 1H), 7.38 (dd, J = 8.7, 1.9 Hz, 1H), 7.16 (d, J = 815 Hz, 1H), 6.74 (d, J = 2.6 Hz, 1H), 6.61 (dd, J = 8.5, 2.7 Hz, 1H), 6.07 - 6.01 (m, 1H), 4.18 - 4.11 (m, 2H), 3.72 (s, 3H), 3.72 - 3.65 (m, 2H), 3.34 (s, 3H), 2.94 - 2.87 (m, 2H), 2.70 - 2.57 (m, 1H), 2.22 (s, 3H), 2.07 -1.96 (m, 2H), 1.87 -1.68 (m, 4H) | (Method C) *mlz* 422.3 [M+H]⁺ at 1.30 min |
| **12** | N-(2-methoxy-4-(2-methoxyethoxy )phenyl)-7-(prop-1-en-2-yl)quinolin-4-amine | 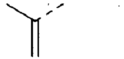 | C from compound 34 | ¹H NMR (400 MHz, DMSO-*d*₆) δ 8.44 (s, 1H), 8.35 (d, J = 8.9 Hz, 1H), 8.29 (d, J = 5.3 Hz, 1H), 7.83 (d, J = 1.9 Hz, 1H), 7.70 (dd, J = 8.9, 2.0 Hz, 1H), 7.18 (d, J = 8.6 Hz, 1H), 6.75 (d, J = 2.7 Hz, 1H), 6.61 (dd, J = 8.6, 2.6 Hz, 1H), 6.06 (d, J = 5.4 Hz, 1H), 5.70 - 5.66 (m, 1H), 5.29 - 5.23 (m, 1H), 4.18 - 4.11 (m, 2H), 3.73 (s, 3H), 3.71 - 3.67 (m, 2H), 3.34 (s, 3H), 2.23 (s, 3H). | (Method C) *m*/*z* 365.2 [M+H]⁺ at 2.66 min |
| **13** | 7-isopropyl-N-(2-methoxy-4-(2-methoxyethoxy )phenyl)quinoli n-4-amine | 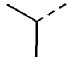 | C from compound 34 then F | ¹H NMR (400 MHz, DMSO-*d*₆) δ 8.45 (br s, 1H), 8.33 (d, J = 8.7 Hz, 1H), 8.26 (d, J = 5.4 Hz, 1H), 7.63 (d, J = 1.8 Hz, 1H), 7.40 (dd, J = 8.7, 1.9 Hz, 1H), 7.17 (d, J = 8.5 Hz, 1H), 6.75 (d, J = 2.6 Hz, 1H), 6.61 (dd, J = 8.6, 2.6 Hz, 1H), 6.05 (d, J = 5.4 Hz, 1H), 4.18 - 4.11 (m, 2H), 3.73 (s, 3H), 3.71 - 3.66 (m, 2H), 3.34 (s, 3H), 3.12 - 3.00 (m, 1H), 1.31 (s, 3H), 1.29 (s, 3H). | (Method C) *m*/*z* 367.2 [M+H]⁺ at 2.88 min |
| **14** | N-(2-methoxy-4-(2-methoxyethoxy )phenyl)-7-(pyridin-2-yl)quinolin-4-amine | 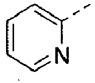 | G from compound 34 | ¹H NMR (400 MHz, DMSO-*d*₆) δ 8.75 (dd, J = 4.9,1.8 Hz, 1H), 8.52 (dd, J = 5.4, 3.4 Hz, 2H), 8.36 (d, J = 5.3 Hz, 1H), 8.26 - 8.15 (m, 2H), 7.94 (td, J = 7.7, 1.9 Hz, 1H), 7.45 - 7.37 (m, 1H), 7.21 (d, J = 8.5 Hz, 1H), 6.77 (d, J = 2.6 Hz, 1H), 6.62 (dd, J = 8.6, 2.6 Hz, 1H), 6.12 (d, J = 5:3 Hz, 1H), 4.19 - 4.12 (m, 2H), 3.75 (s, 3H), 3.73 - 3.66 (m, 2H), 3.34 (s, 3H). | (Method C) *m*/*z* 402.2 [M+H]⁺ at 2.41 min |
| **15** | N-(2-methoxy-4-(2-methoxyethoxy )phenyl)-7-(thiazol-4-yl)quinolin-4-amine | 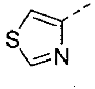 | G from compound 34 | ¹H NMR (400 MHz, DMSO-*d*₆) δ 9.27 (d, J = 1.8 Hz, 1H), 8.51- 8.44 (m, 2H), 8.43 (d, J = 1.9 Hz, 1H), 8.33 (d, J = 5.3 Hz, 1H), 8.10 (dd, J = 8.8,1.9 Hz, 1H). 7.20.(d, J = 8.5 Hz, 1H), 6.76 (d, J = 2.6 Hz, 1H), 6.62 (dd, J = 8.5, 2.6 Hz, 1H), 6.09 (d, J = 5.3 Hz, 1H), 4.18 - 4.11 (m, 2H), 3.75 (s, 3H), 3.72 - 3.67 (m, 2H), 3.34 (s, 3H) | (Method C) *m*/*z* 408.2 [M+H]+ at 2.47 min |
| **16** | N-(2-methoxy-4-(2-methoxyethoxy )phenyl)-7-(4-(oxetan-3-yl)piperazin-1-yl)quinolin-4-amine | 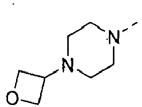 | D from compound 34 | ¹H NMR (400 MHz, DMSO-*d*₆) δ 8.25 - 8.19 (m, 2H), 8.15 (d, J = 5.3 Hz, 1H), 7.29 (dd, J = 9.4, 2.6 Hz, 1H), 7.15 (d, J = 8.5 Hz, 1H), 7.06 (d, J = 2.5 Hz, 1H), 6.73 (d, J = 2.7 Hz, 1H), 6.59 (dd, J = 8.6, 2.6 Hz, 1H), 5.91 (d, J = 5.3 Hz, 1H), 4.59 (t, J = 6.5 Hz, 2H), 4.50 (t, J = 6.0 Hz, 2H), 4.16 - 4.11 (m, 2H), 3.72 (s, 3H), 3.71 - 3.66 (m, 2H), 3.47 (p, J = 6.3 Hz, 1H), 3.33 - 3.29 (m, 7H), 2.45 (t, J = 5.0 Hz, 4H). | (Method C) *m*/*z* 465.3 [M+H]⁺. at 1.61 min |
| **17** | (S)-7-(3,4-dimethylpiperaz in-1-yl)-N-(2-methoxy-4-(2-methoxyethoxy )phenyl)quinoli n-4-amine | 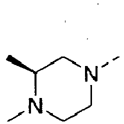 n-4-amine | D from compound 34 | ¹H NMR (400 MHz, DMSO-*d*₆) δ 8.23 - 8.18 (m, 2H), 8.14 (d, J = 5.3 Hz, 1H), 7.29 (dd, J = 9.4, 2.6 Hz, 1H), 7.15 (d, J = 8.6 Hz, 1H), 7.04 (d, J = 2.5 Hz, 1H), 6.73 (d, J = 2.7 Hz, 1H), 6.59 (dd, J = 8.6, 2.7 Hz, 1H), 5.90 (d, J = 5.3 Hz, 1H), 4.17 - 4.11 (m, 2H), 3.76 - 3.66 (m, 7H), 3.33 (s, 3H), 2,90 - 2.79 (m, 2H), 2.48 - 2.43 (m, 1H), 2.34 - 2.25 (m, 1H), 2.23 (s, 3H), 2.21 - 2.13 (m, 1H), 1.09 (d, J = 6.1 Hz, 3H). | (Method C) *mlz* 437.3 [M+H]⁺ at 1.31 min |
| **18** | (R)-7-(3,4-dimethylpiperaz in-1-yl)-N-(2-methoxy-4-(2-methoxyethoxy )phenyl)quinoli n-4-amine | 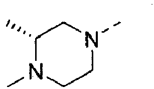 | D from compound 34 | ¹H NMR (400 MHz, DMSO-*d*₆) δ 8.23 - 8.18 (m, 2H), 8.14 (d, J = 5.3 Hz, 1H), 7.29 (dd, J = 9.4, 2.6 Hz, 1H), 7.15 (d, J = 8.6 Hz, 1H), 7.04 (d, J = 2.5 Hz, 1H), 6.73 (d, J = 2.7 Hz, 1H), 6.59 (dd, J = 8.6, 2.7 Hz, 1H), 5.90 (d, J = 5.3 Hz, 1H), 4.17 - 4.10 (m, 2H), 3.72 (s, 3H), 3.71-3.65 (m, 4H), 3.33 (s, 3H), 2.89 - 2.80 (m, 2H), 2.48 - 2.43 (m, 1H), 2.35 - 2.23 (m, 1H), 2.23 (s, 3H), 2.17 (ddt, J = 8.5, 6.2, 3.0 Hz, 1H), 1.09 (d, J = 6.1 Hz, 3H). | (Method C) *mlz* 437.3 [M+H]⁺ at 1.26 min |
| **19** | N-(2-methoxy-methoxyethoxy )phenyl)-7-(4-methyl-1,4-diazepan-1-yl)quinolin-4-amine | 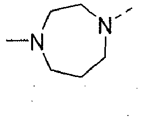 | D from compound 34 | ¹H NMR (400 MHz, DMSO-*d*₆) δ 8.19-8.12 (m, 2H), 8.07 (d, J = 5.3 Hz, 1H), 7.14 (d, J = 8.5 Hz, 1H), 7.07 (dd, J = 2.7, 9.4 Hz, 1H), 6.83 (d, J = 2.6 Hz, 1H), 6.72 (d, J = 2.6 Hz, 1H), 6.59 (dd, J = 2.6, 8.5 Hz, 1H), 5.81 (d, J = 5.3 Hz, 1H), 4.15 -4.11 (m, 2H), 3.72 (s, 3H), 3.70-3.66 (m, 2H), 3.65 - 3.61 (m, 2H), 3.56 (t, J = 6.3 Hz, 2H), 3.33 (s, 3H), 2.68 - 2.62 (m, 2H), 2.47 - 2.42 (m, 2H), 2.27 (s, 3H), 1.94 (p, J = 5.9 Hz, 2H). | (Method C) *m*/*z* 437.4 [M+H]+ at 1.40 min |
| **20** | N-(2-methoxy-4-(2-methoxyethoxy )phenyl)-7-(4-(2-methoxyethyl)pi perazin-1-yl)quinolin-4-amine | 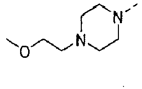 | D from compound 34 | ¹H NMR (400 MHz, DMSO-*d*₆) δ 8.23 (s, 1H), 8.20 (d, J = 9.4 Hz, 1H), 8.14 (d, J = 5.3 Hz, 1H), 7.28 (dd, J = 2.6, 9.4 Hz, 1H), 7.15 (d, J = 8.5 Hz, 1H), 7.04 (d, J = 2.5 Hz, 1H), 6.73 (d, J = 2.6 Hz, 1H), 6.59 (dd, J = 2.6, 8.6 Hz, 1H), 5.90 (d, J = 5.3 Hz, 1H), 4.16 - 4.11 (m, 2H), 3.72 (s, 3H), 3.71 - 3.66 (m, 2H), 3.49 (t, J = 5.8 Hz, 2H), 3.33 (s, 3H), 3.29 - 3.24 (m, 4H), 2.60 (t, J = 5.0 Hz, 4H), 2.54 (t, J = 5.8 Hz, 2H). | (Method C) *mlz* 467.3 [M+H]⁺ at 1.38 min |
| **21** | N-(2-methoxy-4-(2-methoxyethoxy )phenyl)-7-(6-methyl-2,6-diazaspiro[3.3] heptan-2-yl)quinolin-4-amine | 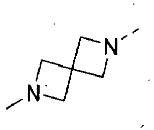 | D from compound 34 | ¹H NMR (400 MHz, DMSO-*d*₆) δ 8.21 - 8.16 (m, 2H), 8.10 (d, J = 5.3 Hz, 1H), 7.14 (d, J = 8.6 Hz, 1H), 6.72 (dt, J = 2.8, 5.3 Hz, 2H), 6.59 (dt, J = 2.6, 5.0 Hz, 2H), 5.84 (d, J = 5.3 Hz, 1H), 4.15 - 4.11 (m, 2H), 3.97 (s, 4H), 3.72 (s, 3H), 3.70 - 3.66 (m, 2H), 3.33 (s, 3H), 3.29 (s, 3H), 2.20 (s, 3H). | (Method C) *mlz* 435.3 [M+H]+ at 1.38 min |
| **22** | 7-(4-ethylpiperazin-1-y))-N-(2-methoxy-4-(2-methoxyethoxy )phenyl)quinoli n-4-amine | 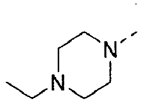 | D from compound 34 | ¹H NMR (400 MHz, DMSO-*d*₆) δ 8.23 - 8.18 (m, 2H), 8.14 (d, J = 5.3 Hz, 1H), 7.28 (dd, J = 2.6, 9.3 Hz, 1H), 7.15 (d, J = 8.5 Hz, 1H), 7.05 (d, J = 2.6 Hz, 1H), 6.73 (d, J = 2.6 Hz, 1H), 6.59 (dd, J = 2.7, 8.6 Hz, 1H), 5.91 (d, J = 5.3 Hz, 1H), 4.16. - 4.12 (m, 2H), 3.72 (s, 3H), 3.70 - 3.66 (m, 2H), 3.33 (s, 3H), 3.30 - 3.26 (m, 4H), 2.54 (t, J = 5.0 Hz, 4H), 2.39 (q, J = 7.2 Hz, 2H), 1.05 (t, J = 7.2 Hz, 3H). | (Method C) *mlz* 437.3 [M+H]+ at 1.35 min |
| **23** | (S)-7-(2,4-dimethylpiperaz in-1-yl)-N-(2-methoxy-4-(2-methoxyethoxy)phenyl)quinoli n-4-amine | 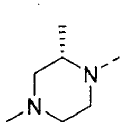 8.13 6.74 | D from compound 34 | ¹H NMR (400 MHz, DMSO-*d*₆) δ 8.49 (s, 1H), 8.26 - 8.21 (m, 2H), 8.13 (d, J = 5.6 Hz, 1H), 7.30 (dd, J = 2.6, 9.5 Hz, 1H), 7.16 (d, J = 8.5 Hz, 1H), 6.99 (d, J = 2.5 Hz, 1H), 6.74 (d, J = 2.7 Hz, 1H), 6.61 (dd, J= 2.7, 8.6 Hz, 1H), 5.91 (d, J = 5.6 Hz, 1H), 4.28 - 4.20 (m, 1H), 4.17 - 4.11 (m, 2H), 3.73 (s, 3H), 3.71-3.65 (m, 2H), 3.50 - 3.41 (m, 1H), 3.08 (td, J = 3.4, 12.0 Hz, 1H), 2.91 - 2.84 (m, 1H), 2.72 (dt, J = 2.1, 10.8 Hz, 1H), 2.27 (dd, J = 3.6, 11.0 Hz, 1H), 2.22 (s, 3H), 2.07 (td, J = 3.5, 11.4 Hz, 1H), 1.09 (d, J = 6.5 Hz, 3H). | (Method C) *m*/*z* 437.3 [M+H]⁺ at 1.42 min |
| **24** | tert-butyl 3-(4-(4-((2-methoxy-4-(2-methoxyethoxy )phenyl)amino) quinolin-7-yl)piperazin-1-yl)azetidine-1-carboxylate | 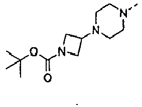 | D from compound 34 | ¹H NMR (400 MHz, DMSO-de) δ 8.23 - 8.19 (m, 2H), 8.15 (d, J = 5.3 Hz, 1H), 7.29 (dd, J = 2.6, 9.4 Hz, 1H), 7.15 (d, J = 8.6 Hz, 1H), 7.05 (d, J = 2.5 Hz, 1H), 6.73 (d, J = 2.6 Hz, 1H), 6.59 (dd, J = 2.7, 8.6 Hz, 1H), 5.91 (d, J = 5.3 Hz, 1H), 4.16 - 4.11 (m, 2H), 3.93 - 3.84 (m, 2H), 3.76 - 3.71 (m, 4H), 3.71 - 3.65 (m, 2H), 3.33 (s, 3H), 3.31 - 3.26 (m, 4H), 3.16 - 3.06 (m, 1H), 1.39 (s, 9H). | (Method C) *m*/*z* 564.3 [M+H]⁺ at 2.53 min |
| **25** | N-(2-methoxy-4-(2-methoxyethoxy )phenyl)-7-(piperazin-1-yl)quinolin-4-amine | 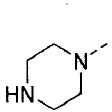 | D from compound 34 then H | ¹H NMR (400 MHz, DMSO-*d*₆) δ 8.22 - 8.18 (m, 2H), 8.14 (d, J = 5.3 Hz, 1H), 7.27 (dd, J = 9.3, 2.6 Hz, 1H), 7.15 (d, J = 8.5 Hz, 1H), 7.03 (d, J = 2.5 Hz, 1H), 6.73 (d, J = 2.7 Hz, 1H), 6.59 (dd, J = 8.6, 2.6 Hz, 1H), 5.90 (d, J = 5.4 Hz, 1H), 4.17 - 4.11 (m, 2H), 3.72 (s, 3H), 3.72 - 3.64 (m, 2H), 3.33 (s, 3H), 3.29 (s, 1H), 3.18 (q, J = 4.3 Hz, 4H), 2.87 (dd, J = 6.1, 3.7 Hz, 4H). | (Method C) *mlz* 409.3 [M+H]⁺ at 1.20 min |
| **26** | N-(2-methoxy-4-(2-methoxyethoxy )phenyl)-7-(4-(1-methylazetidin-3-yl)piperazin-1-yl)quinolin-4-amine | 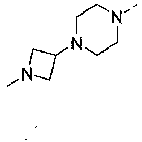 | D from compound 34 then I | ¹H NMR (400 MHz, DMSO-*d*₆) δ 8.32 (s, 1H), 8.22 (d, J = 9.3 Hz, 1H), 8.14 (d, J = 5.4 Hz, 1H), 7.29 (dd, J = 2.5, 9.5 Hz, 1H), 7.15 (d, J = 8.5 Hz, 1H), 7.05 (d, J = 2.5 Hz, 1H), 6.73 (d, J = 2.6 Hz, 1H); 6.60 (dd, J = 2.6, 8.6 Hz, 1H), 5.91 (d, J = 5.4 Hz, 1H), 4.16 - 4.11 (m, 2H), 3.72 (s, 3H), 3.71 - 3.66 (m, 2H), 3.42 (t, J = 6.1 Hz, 2H), 3.33 (s, 3H), 3.28 (t, J = 5.1 Hz, 4H), 2.93 - 2.86 (m, 1H), 2.85 - 2.80 (m, 2H), 2.42 (t, J = 5.0 Hz, 4H), 2.24 (s, 3H). | (Method C) *m*/*z* 478.4 [M+H]⁺ at 1.53 min |
| **27** | N-(2-methoxy-4-(2-methoxyethoxy )phenyl)-7-(4-(2,2,2-trifluoroethyl)pi perazin-1-yl)quinolin-4-amine | 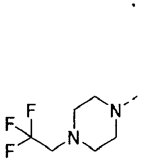 | D from compound 34 | ¹H NMR (400 MHz, DMSO-*d*₆) δ 8.29 (s, 1H), 8.22 (d, J = 9.2 Hz, 1H), 8.15 (d, J = 5.4 Hz, 1H), 7.29 (dd, J = 2.6, 9.5 Hz, 1H), 7.15 (d, J = 8.5 Hz, 1H), 7.06 (d, J = 2.5 Hz, 1H), 6.73 (d, J = 2.6 Hz, 1H), 6.60 (dd, J = 2.6, 8.5 Hz, 1H), 5.92 (dd, J = 2.1, 5.4 Hz, 1H), 4.16-4.12 (m, 2H), 3.72 (s, 3H), 3.70 - 3.67 (m, 2H), 3.33 (s, 3H), 3.31 - 3.22 (m, 2H), 2.81 (t, J = 5.0 Hz, 4H). | (Method C) *m*/*z* 491.2 [M+H]+ at 4.23 min |
| **28** | 7-(4-(azetidin-3-yl)piperazin-1-yl)-N-(2-methoxy-4-(2-methoxyethoxy )phenyl)quinoli n-4-amine | 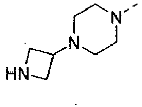 | D from compound 34 then H | ¹H NMR (400 MHz, DMSO-*d*₆) δ 8.21 (d, J = 9.3 Hz, 2H), 8.14 (d, J 5.3 Hz, 1H), 7.31 - 7.23 (m, 1H), 7.15 (d, J = 8.5 Hz, 1H), 7.05 (d, J = 2.5 Hz, 1H), 6.73 (d, J = 2.6 Hz, 1H), 6.59 (dd, J = 2.6, 8.6 Hz, 1H), 5.91 (d, J = 5.3 Hz, 1H), 4.18-4.10 (m, 2H), 3.72 (s, 3H), 3.70 - 3.64 (m, 2H), 3.52 - 3.42 (m, 1H), 3.41 - 3.35 (m, 1H), 3.33 (s, 3H), 3.31 - 3.23 (m, 4H), 3.19 - 3.04 (m, 1H), 2.96 - 2.86 (m, 1H), 2.43 (s, 4H). | (Method C) *m*/*z* 464.4 [M+H]+ at 1.27 min |
| **29** | (R)-(4-(4-((2-methoxy-4-(2-methoxyethoxy )phenyl)amino) quinolin-7-yl)-1-methylpiperazin -2-yl)methanol | 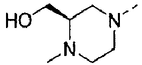 | D from compound 34 then I* | ¹H NMR (400 MHz, DMSO-*d*₆) δ 8.27 - 8.18 (m, 2H), 8.14 (d, J = 5.3 Hz, 1H), 7.26 (dd, J = 2.6, 9.3 Hz, 1H), 7.15 (d, J = 8.6 Hz, 1H), 7.06 (d, J = 2.5 Hz, 1H), 6.73 (d, J = 2.7 Hz, 1H), 6.59 (dd, J = 2.7, 8.6 Hz, 1H), 5.90 (d, J = 5.3 Hz, 1H), 4.64 (s, 1H), 4.17 - 4.11 (m, 2H), 3.87 - 3.80 (m, 1H), 3.72 (s, 3H and m, 1H), 3.71 - 3.66 (m, 3H), 3.40 (dd, J = 6.9, 11.0 Hz, 1H), 3.33 (s, 3H), 2.89 - 2.80 (m, 2H), 2.61 (dd, J = 10.0, 1.2.1 Hz, 1H), 2.34 - 2.28 (m, 1H), 2.27 (s, 3H), 2.17 (ddt, J = 3.4, 6.9,10.2 Hz, 1H). | (Method C) *m*/*z* 453.4 [M+H]⁺ at 1.09 min |
| **30** | (S)-(4-(4-((2-methoxy-4-(2-methoxyethoxy )phenyl)amino) quinolin-7-yl)-1-methylpiperazin -2-yl)methanol | 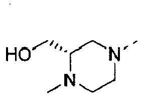 | D from compound 34 then I* | ¹H NMR (400 MHz, DMSO-*d*₆) δ 8.27 - 8.22 (m, 1H), 8.22 (d, J = 9.3 Hz, 1H), 8.14 (d, J = 5.4 Hz, 1H), 7.26 (dd, J = 2.6, 9.4 Hz, 1H), 7.15 (d, J = 8.5 Hz, 1H), 7.06 (d, J = 2.6 Hz, 1H), 6.73 (d, J = 2.7 Hz, 1H), 6.59 (dd, J = 2.7, 8.6 Hz, 1H), 5.91 (d, J = 5.4 Hz, 1H), 4.63 (s, 1H), 4.16 - 4.11 (m, 2H), 3.84 (dt, J = 2.5,11.9 Hz, 1H), 3.73 (s, 4H), 3.71 - 3.66 (m, 3H), 3.40 (dd, J = 6.9, 11.0 Hz, 1H), 3.33 (s, 3H), 2.91 - 2.78 (m, 2H), 2.62 (dd, J = 10.0, 12.1 Hz, 1H), 2.32 (td, J = 3.7, 11.8 Hz, 1H), 2.27 (s, 3H), 2.20 - 2.13 (m, 1H). | (Method C) *m*/*z* 453.5 [M+H]+ at 1.06 min . |
| **31** | tert-butyl 5-(4-((2-methoxy-4-(2-methoxyethoxy )phenyl)amino) quinolin-7-yl)-2,5-diazabicyclo[2. 2.1]heptane-2-carboxylate | 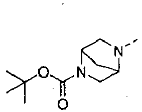 | D from compound 34 | ¹H NMR (400 MHz, DMSO-*d*₆) δ 8.20 (d, J = 9.4 Hz, 1H), 8.17 (s, 1H), 8.10 (d, J = 5.3 Hz, 1H), 7.14 (d, J = 8.5 Hz, 1H), 6.99 (d, J = 9.1 Hz, 1H), 6.80 - 6.74 (m, 1H), 6.73 (d, J = 2.6 Hz, 1H), 6.59 (dd, J = 2.7, 8.6 Hz, 1H), 5.87 - 5.82 (m, 1H), 4.69 (s, 1H), 4.46 (d, J = 21.8 Hz, 1H), 4.18 - 4.10 (m, 2H), 3.73 (s, 3H), 3.71 - 3.67 (m, 2H), 3.65 (d, J = 8.6 Hz, 1H), 3.37 (t, J = 8.8 Hz, 1H), 3.33 (s, 3H), 3.29 - 3.23 (m, 1H), 3.11 (d, J = 9.1 Hz, 1H), 2.02 - 1.91 (m, 2H), 1.36 (d, J = 28.6 Hz, 9H). | (Method C) *m*/*z* 521.3 [M+H]⁺ at 3.27 min |
| **32** | N-(2-methoxy-4-(2-methoxyethoxy )phenyl)-7-(5-methyl-2,5-. diazabicyclo[2. 2.1]heptan-2-yl)quinolin-4-amine | 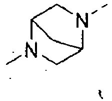 | D from compound 34 then I | ¹H NMR (400 MHz, DMSO-*d₆*) δ 8.16 (m, 2H), 8.09 (d, J = 5.4 Hz, 1H), 7.14 (d, J = 8.5 Hz, 1H), 6.94 (dd, J = 2.5, 9.2 Hz, 1H), 6.73 (d, J = 2.6 Hz, 2H), 6.59 (dd, J = 2.6, 8.6 Hz, 1H), 5.85 - 5.80 (m, 1H), 4.46 (s, 1H), 4.17-4.09 (m, 2H), 3.73 (s, 3H), 3.71 - 3.66 (m, 2H), 3.45 (s, 1H), 3.41 (dd, J = 2.3, 9.2 Hz, 1H), 3.33 (s, 2H), 3.29 (d, J = 9.1 Hz, 2H), 2.84 (dd, J = 2.0, 9.4 Hz, 1H), 2.26 (s, 3H), 1.90 (d, J = 9.6 Hz, 1H), 1.82 (d, J = 9.1 Hz, 1H). One proton under the solvents peaks. | (Method C) *mlz* 435.4 [M+H]+ at 1.18 min |
| **33** | N-(2-methoxy-4-(2-methoxyethoxy )phenyl)-7-(6-methyl-3,6-diazabicyclo[3. 1.1]heptan-3-yl)quinolin-4-amine | 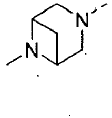 | D from compound 34 then I | ¹H NMR (400 MHz, DMSO-*d*₆) δ 8.22 - 8.15 (m, 2H), 8.10 (d, J = 5.4 Hz, 1H), 7.14 (d, *J* = 8.5 Hz, 1H), 6.79 (dd, J = 2.3, 9.0 Hz, 1H), 6.73 (d, *J* = 2.6 Hz, 1H), 6.67 (d, J = 2.3 Hz, 1H), 6.60 (dd, *J* = 2.7, 8.6 Hz, 1H), 5.87 - 5.83 (m, 1H), 4.39 (d, *J* = 5.6 Hz, 2H), 4.18 - 4.10 (m, 2H), 3.73 (s, 3H), 3.71 - 3.66 (m, 2H), 3.33 (s, 3H), 3.30 - 3.28 (m, 1H), 3.02 (d, *J* = 11.0 Hz, 2H), 2.81 - 2.73 (m, 2H), 2.08 (s, 3H), 2.00 (d, *J* = 7.4 Hz, 1H). | (Method C) *mlz* 435.5 [M+H]⁺ at 1.24 min |
| **34** | 4-(4-((2-methoxy-4-(2-methoxyethoxy )phenyl)amino) qulnolin-7-yl)piparazin-2-one | 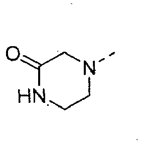 | E* from compound 34 | ¹H NMR (400 MHz, DMSO-*d*₆) δ 9.09 (s, 1H),8.35 (d, J = 9.4 Hz, 1H), 8.21 (s, 1H), 8.20 - 8.13 (m, 2H), 7.38 (dd, J = 9.4, 2.6 Hz, 1H), 7.20 (d, J = 8.5 Hz, 1H), 7.02 (d, J = 2.5 Hz, 1H), 6.77 (d, J = 2.6 Hz, 1H), 6.63 (dd, J =.8.6, 2.6 Hz, 1H), 5.98 (d, J = 6.1 Hz, 1H), 4.19 - 4.13 (m, 2H), 3.91 (s, 2H), 3.74 (s, 3H), 3.71 - 3.67 (m, 2H), 3.61 (dd, J = 6.5, 4.2 Hz, 2H), 3.40 - 3.35 (m, 2H), 3.33 (s, 3H). | (Method C) *m*/*z* 423.3 [M+H]⁺ at 1.97 min |

| | | | | | |
|---|---|---|---|---|---|
| * Purified by prep-HPLC | | | | | |

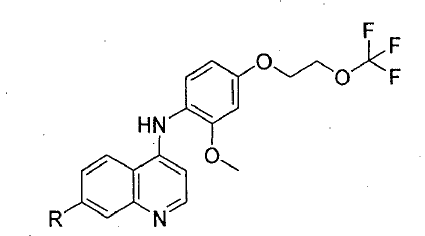

| **Example** | **Name** | **R** | **General Procedure** | **¹H NMR δ (400 MHz)** | **LCMS (ES+) *m*/*z*** |
|---|---|---|---|---|---|
| **35** | . N-(2-methoxy-4-(2-(trifluorometho xy)ethoxy)phe nyl)-7-(1-methyl-1,2,3,6-tetrahydropyri din-4-yl)quinolin-4-amine | 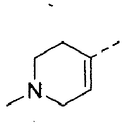 | C from compound 35 | ¹H NMR (400 MHz, DMSO-*d*₆) δ 8.93 (br s, 1H), 8.46 - 8.38 (m, 1H), 8.32 (d, J = 5.7 Hz, 1H), 7.81-7.78 (m, 1H), 7.77 - 7.73 (m, 1H), 7.23 (d, J = 8.5 Hz, 1H), 6.80 (d, J = 2.7 Hz, 1H), 6.67 (dd, J = 8.6, 2.7 Hz, 1H), 6.53 - 6.47 (m, 1H), 6.11 (d, J = 5.7 Hz, 1H), 4.48 - 4.41 (m, 2H), 4.39 - 4.29 (m, 2H), 3.75 (s, 3H), 3.38 - 3.32 (m, 2H), 2.94 - 2.83 (m, 2H), 2.72 - 2.68 (m, 2H). | (Method C) *m*/*z* 474.3 [M+H]⁺ at 1.93 min |
| **36** | N-(2-methoxy-4-(2-(trifluorometho xy)ethoxy)phe nyl)-7-(4-methylpiperazi n-1-yl)quinolin-4-amine | 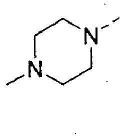 | D from compound 35 | ¹H NMR (400 MHz, DMSO-*d*₆) δ 8.50 (br s, 1H), 8.27 - 8.21 (m, 2H), 8.15 (d, J = 5.6 Hz, 1H), 7.33 (dd, J = 9.3, 2.6 Hz, 1H), 7.19 (d, J = 8.5 Hz, 1H), 7.06 (d, J = 2.5 Hz, 1H), 6.77 (d, J = 2.7 Hz, 1H), 6.64 (dd, J = 8.6, 2.7 Hz, 1H), 5.95 - 5.92 (m, 1H), 4.47 - 4.41 (m, 2H), 4.34 - 4.27 (m, 2H), 3.74 (s, 3H), 3.33 - 3.28 (m, 4H), 2.25 (s, 3H). | (Method C) *m*/*z* 477.3 [M+H]+ at 1.90 min |
| **37** | N-(2-methoxy-4-(2-(trifluorometho xy)ethoxy)phe nyl)-7-(1-methylpiperidin-4-yl)quinolin-4-amine | 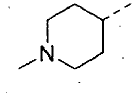 | C from compound 35 then F | ¹H NMR (400 MHz, DMSO-*d*₆) δ 8.72 (br s, J = 36.0 Hz, 1H), 8.39 (d, J = 8.7 Hz, 1H), 8.29 (d, J = 5.6 Hz, 1H), 7.67 - 7.62 (m, 1H), 7.46 - 7.39 (m, 1H), 7.20 (d, J = 8.5 Hz, 1H), 6.78 (d, J = 2.7 Hz, 1H), 6.65 (dd, J = 8.6, 2.6 Hz, 1H), 6.08 (d, J = 5.6 Hz, 1H), 4.46 - 4.40 (m, 2H), 4.34 - 4.27 (m, 2H), 3.73 (s, 3H), 3.18 (dd, J =12.2, 9.2 Hz, 2H), 2.88 - 2.75 (m, 1H), 2.61 - 2.46 (m, 4H), 2.02 - 1.81 (m, 4H). | (Method C) *m*/*z* 476.3 [M+H]⁺ at 1.86 min |
| **38** | N-(2-methoxy-4-(2-(trifluorometho xy)ethoxy)phe nyl)-7-(1-methyl-1H-pyrazol-4-yl)quinolin-4-amine | 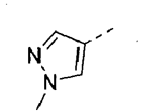 | C from compound 35 | ¹H NMR (400 MHz, DMSO-*d*₆) δ 8.69 (s, 1H), 8.41 (d, J = 8.8 Hz, 1H), 8.35 (s, 1H), 8.28 (d, J = 5.5 Hz, 1H), 8.06 (s, 1H), 7.97 (d, J = 1.8 Hz, 1H), 7.76 (dd, J = 8.7, 1.9 Hz, 1H), 7.23 (d, J = 8.6 Hz, 1H), 6.80 (d, J = 2.6 Hz, 1H), 6.66 (dd, J = 8.6, 2.7 Hz, 1H), 6.06 (d, J = 5.5 Hz, 1H), 4.48 - 4.41 (m, 2H), 4.32 (t, J = 4.1 Hz, 2H), 3.91 (s, 3H), 3.76 (s, 3H). | (Method C) *m*/*z* 459.2 [M+H]+ at 3.06 min |
| **39** | N-(2-methoxy-4-(2-(trifluorometho xy)ethoxy)phe nyl)-7-methylquinolin -4-amine | 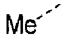 | A from 4-chloro-7-methylquinoline and 4-Amino-3-methoxyphe nol then I | ¹H NMR (400 MHz, DMSO-*d*₆) δ 14.18 (s, 1H), 10.47 (s, 1H), 8.61 (d, J = 8.7 Hz, 1H), 8.38 (d, J = 7.0 Hz, 1H), 7.78 (s, 1H), 7.63 (dd, J = 8.8, 1.6 Hz, 1H), 7.32 (d, J = 8.6 Hz, 1H), 6.87 (d, J = 2.6 Hz, 1H), 6.73 (dd, J = 8.6, 2.6 Hz, 1H), 6.24 (d, J = 7.0 Hz, 1H), 4.54 - 4.42 (m, 2H), 4.35 (t, J = 4.2 Hz, 2H), 3.78 (s, 3H), 2.57 (s, 3H). | (Method C) *m*/*z* 393.1 [M+H]⁺ at 3.06 min |
| **40** | 7-methoxy-N-(2-methoxy-4-(2-(trifluorometho xy)ethoxy)phe nyl)quinolin-4-amine | 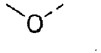 | A from 4-chloro-7-methoxyquin oline and 4-Amino-3-methoxyphe nol then I | ¹H NMR (400 MHz, DMSO-*d*₆) δ 14.02 (s, 1H), 10.41 (s, 1H), 8.62 (d, J = 9.3 Hz, 1H), 8.34 (d, J = 7.0 Hz, 1H), 7.41 (dd, J = 9.3, 2.5 Hz, 1H), 7.38 (d, J = 2.5 Hz, 1H), 7.31 (d, J = 8.6 Hz, 1H), 6.87 (d, J = 2.6 Hz, 1H), 6.73 (dd, J = 8.6, 2.6 Hz, 1H), 6.19 (d, J = 7.0 Hz, 1H), 4.50 - 4.42 (m, 2H), 4.35 (t, J = 4.1 Hz, 2H), 3.96 (s, 3H), 3.78 (s, 3H). | (Method C) *mlz* 409.2 [M+H]⁺ at 3.05 min |
| **41** | N-(2-methoxy-4-(2-(trifluorometho xy)ethoxy)phe nyl)-7-(pyrazin-2-yl)quinolin-4-amine | 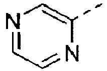 | G from compound 35 | ¹H NMR (400 MHz, DMSO-*d*₆) δ 9.48 (d, J = 1.6 Hz, 1H), 8.93 (s, 1H), 8.82 (dd, J = 2.6, 1.5 Hz, 1H), 8.70 (d, J = 2.5 Hz, 1H), 8.66-8.55 (m, 2H), 8.40 (d, J = 5.5 Hz, 1H), 8.30 (dd, J = 8.8,1.9 Hz, 1H), 7.26 (d, J = 8.5 Hz, 1H), 6.81 (d, J = 2.7 Hz, 1H), 6.68 (dd, J = 8.6, 2.7 Hz, 1H), 6.18 (d, J = 5.6 Hz, 1H), 4.50 - 4.40 (m, 2H), 4.37-4.28 (m, 2H), 3.77 (s, 3H). | (Method C) *mlz* 457.2 [M+H]+ at 2.97 min |
| **42** | N-(2-methoxy-4-(2-(trifluorometho xy)ethoxy)phe nyl)-7-(pyridin-2-yl)quinolin-4-amine | 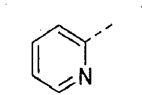 | G from compound 35 | ¹H NMR (400 MHz, DMSO-*d*₆) δ 9.02 (s, 1H), 8.80 - 8.69 (m, 1H), 8.60 - 8.53 (m, 2H), 8.39 (d, J = 5.7 Hz, 1H), 8.28 (dd, J = 8.8, 1.9 Hz, 1H), 8.20 (d, J = 8.0 Hz, 1H), 7.97 (td, J = 7.7,1.9 Hz, 1H), 7.48-7.40 (m, 1H), 7.26 (d, J = 8.5 Hz, 1H), 6.82 (d, J = 2.6 Hz, 1H), 6.68 (dd, J = 8.6, 2.6 Hz, 1H), 6.17 (d, J = 5.7 Hz, 1H), 4.53 - 4.39 (m, 2H), 4.38 - 4.30 (m, 2H), 3.77 (s, 3H). | (Method C) *m*/*z* 456.3 [M+H]⁺ at 3.22 min |
| **43** | N-(2-methoxy-4-(2-(trifluorometho xy)ethoxy)phe nyl)-7-(thiazol-4-yl)quinolin-4-amine | 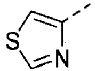 | G from compound 35 | ¹H NMR (400 MHz, DMSO-*d*₆) δ 9.29 (d, J = 1.9 Hz, 1H), 8.98 (s, 1H), 8.54 (d, J = 8.8 Hz, 1H), 8.48 (dd, J = 3.2,1.8 Hz, 2H), 8.36 (d, J = 5.7 Hz, 1H), 8.17 (dd, J = 8.8,1.8 Hz, 1H), 7.26 (d, J = 8.6 Hz, 1H), 6.82 (d, J = 2.6 Hz, H), 6.68 (dd, J = 8.6, 2.6 Hz, 1H), 6.14 (d, J = 5.7 Hz, 1H), 4.50 - 4.42 (m, 2H), 4.35 - 4.29 (m, 2H), 3.77 (s, 3H). | (Method C) *m*/*z* 462.2 [M+H]⁺ at 3.23 min |
| **44** | 7-(difluoromethy l)-N-(2-methoxy-4-(2-(trifluorometho xy)ethoxy)phe nyl)quinolin-4-amine | 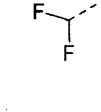 | J from compound 39 | ¹H NMR (400 MHz, DMSO-*d*₆) δ 14.47 (s, 1H), 10.72 (s, 1H), 8.86 (d, J = 8.8 Hz, 1H), 8.52 (d, J = 7.0 Hz, 1H), 8.21 (d, J =1.6 Hz, 1H), 7.94 (dd, J = 9.0,1.8 Hz, 1H), 7.36 (t, J = 55.2 Hz, 1H), 7.34 (d, J = 8.6 Hz, 1H), 6.89 (d, J = 2.6 Hz, 1H), 6.75 (dd, J = 8.6, 2.6 Hz, 1H), 6.37 (d, J = 7.0 Hz, 1H), 4.49 - 4.43 (m, 2H), 4.39- 4.32 (m, 2H), 3.79 (s, 3H). | (Method C) *mlz* 429.2 [M+H]⁺ at 2.77 min |

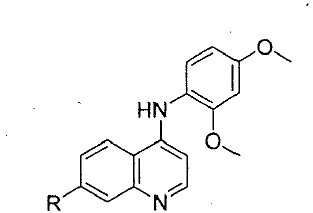

| **Example** | **Name** | **R** | **General Procedure** | **¹H NMR δ (400 MHz)** | **LCMS** (ES+) ***m*/*z*** |
|---|---|---|---|---|---|
| **45** | N-(2,4-dimethoxyphe nyl)-7-phenylquinolin -4-amine | 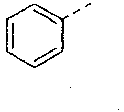 | C from compound 33 | ¹H NMR (400 MHz, DMSO-*d*₆) δ 14.58 (br s, 1H), 10.67 (s, 1H), 8.85 (d, J = 8.9 Hz, 1H), 8.45 (d, J = 7.0 Hz, 1H), 8.32 (d, J = 1.8 Hz, 1H), 8.13 (dd, J = 8.9,1.8 Hz, 1H), 7.90 - 7.82 (m, 2H), 7.65 - 7.57 (m, 2H), 7.57 -7.48 (m, 1H), 7.33 (d, J = 8.6 Hz, 1H), 6.84 (d, J = 2.6 Hz, 1H), 6.71 (dd, J = 8.6, 2.6 Hz, 1H), 6.29 (d, J = 7.0 Hz, 1H), 3.86 (s, 3H), 3.79 (s, 3H). | (Method C) *m*/*z* 357.4 [M+H]⁺ at 3.18 min |

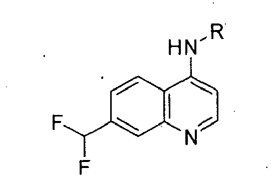

| **Example** | **Name** | **R** | **General Procedure** | **¹H NMR δ (400 MHz)** | **LCMS (ES+) *m*/*z*** |
|---|---|---|---|---|---|
| **46** | 7-(difluoromethy l)-N-(4-methoxy-6-(2-methoxyethox y)pyridin-3-yl)quinolin-4-amine | 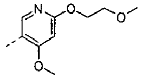 | A from compound 2 and 15 | ¹H NMR (400 MHz, DMSO-*d*₆) δ 14.76 (s, 1H), 10.76 (s, 1H), 8.89 (d, J = 8.8 Hz, 1H), 8.56 (d, J = 6.9 Hz, 1H), 8.26 (d, J = 1.8 Hz, 1H), 8.11 (s, 1H), 7.99 -7.92 (m, 1H), 7.37 (t, J = 55.2 Hz, 1H), 6.74 (s, 1H), 6.46 (d, J = 6.9 Hz, 1H), 4.47 - 4.40 (m, 2H), 3.84 (s, 3H), 3.72 - 3.65 (m, 2H), 3.32 (s, 3H). | (Method C) *m*/*z* 376.2 [M+H]⁺ at 1.80 min |
| **47** | 7-(difluoromethy l)-N-(2-(methoxy-d3)-4-(2-methoxyethox y)phenyl)quin olin-4-amine | 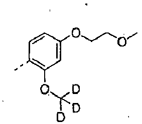 | A from compound 2 and 5 | ¹H NMR (400 MHz, DMSO-*d*₆) δ 14.51 (s, 1H), 10.69 (s, 1H), 8.86 (d, J = 8.8 Hz, 1H), 8.51 (d, J = 7.0 Hz, 1H), 8.21 (d, J = 1.7 Hz, 1H), 7.97 - 7.89 (m, 1H), 7.35 (t, J = 55.2 Hz, 1H), 7.31 (d, J = 8.6 Hz, 1H), 6.85 (d, J = 2.6 Hz, 1H), 6.71 (dd, J = 8.7, 2.6 Hz, 1H), 6.36 (d, J = 7.0 Hz, 1H), 4.22 - 4.16 (m, 2H), 3.74 - 3.67 (m, 2H), 3.34 (s, 3H). | (Method C) *mlz* 378.2 [M+H]⁺ at 2.15 min |
| **48** | 7-(difluoromethy l)-N-(2-methoxy-6-(2-methoxyethox y)pyridin-3-yl)quinolin4-amine | 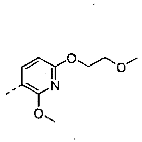 | A from compound 2 and 17 | ¹H NMR (400 MHz, DMSO-*d*₆) δ 14.61 (s, 1H), 10.70 (s, 1H), 8.86 (d, J = 8.8 Hz, 1H), 8.55 (d, J = 6.9 Hz, 1H), 8.23 (d, J = 1.7 Hz, 1H), 7.95. (dd, J = 9.0, 1.7 Hz, 1H), 7.78 (d, J = 8.3 Hz, 1H), 7.36 (t, J = 55.2 Hz, 1H), 6.60 (d, J = 8.3 Hz, 1H), 6.46 (d, J = 6.9 Hz, 1H), 4.50 - 4.43 (m, 2H), 3.89 (s, 3H), 3.75 - 3.68 (m, 2H), 3.33 (s, 3H). | (Method C) *m*/*z* 376.2 [M+H]⁺ at 2.18 min |
| **49** | 7-(difluoromethy l)-N-(2-methoxy-6-(2-(trifluorometho xy)ethoxy)pyri din-3-yl)quinolin-4-amine | 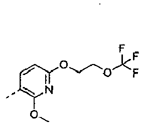 | A from compound 2 and 20 | ¹H NMR (400 MHz, DMSO-*d*₆) δ 14.63 (s, 1H), 10.74 (s, 1H), 8.87 (d, J = 8.8 Hz, 1H), 8.57 (d, J = 7.0 Hz, 1H), 8.24 (d, J = 1.6 Hz, 1H), 7.96 (dd, J = 8.8, 1.6 Hz, 1H), 7.82 (d, J = 8.3 Hz, 1H), 7.36 (t, J =55.2 Hz, 1H), 6.65 (d, J = 8.3 Hz, 1H), 6.47 (d, J = 6.9 Hz, 1H), 4.65 -4.59 (m, 2H), 4.52 - 4.45 (m, 2H), 3.90 (s, 3H). | (Method C) *mlz* 430.1 [M+H]⁺ at 3.11 min |
| **50** | 7-(difluoromethy l)-N-(2-methoxy-4-(1H-pyrazol-4-yl)phenyl)quin olin-4-amine | 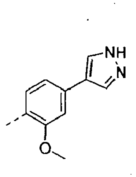 | A from compound 2 and 25 | ¹H NMR (400 MHz, DMSO-*d*₆) δ 14.53 (s, 1H), 13.06'(s, 1H), 10.81 (s, 1H), 8.90 (d, J = 8.8 Hz, 1H), 8.53 (d, J = 7.0 Hz, 1H), 8.31 (s, 1H), 8.23 (d, J = 1.6 Hz, 1H), 8.11 (s, 1H), 7.95 (dd, J = 8.9, 1.7 Hz, 1H), 7.53-7.20 (m, 4H), 6.46 (d, J = 7.0 Hz, 1H), 3.87 (s, 3H). | (Method C) *m*/*z* 367.1 [M+H]⁺ at 1.95 min |
| **51** | 7-(difluoromethy l)-N-(2-methoxy-4-(2-(trifluorometho xy)ethoxy)phe nyl)quinolin-4-amine | 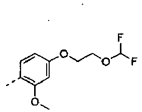 | A from compound 2 and 29 | ¹H NMR (400 MHz, DMSO-*d*₆) δ 8.64 (s, 1H), 8.55 (d, J = 8.8 Hz, 1H), 8.38 (d, J = 5.3 Hz, 1H), 8.04 - 7.98 (m, 1H), 7.61 (dd, J = 8.8, 1.8 Hz, 1H), 7.37 - 7.06 (m, 2H), 7.00 - 6.55 (m, 2H), 6.64 (dd, J = 8.6, 2.7 Hz, 1H), 6.16 (d, J = 5.3 Hz, 1H), 4.28 - 4.22 (m, 2H), 4.22 - 4.16 (m, 2H), 3.74 (s, 3H). | (Method C) *m*/*z* 411.3 [M+H]⁺ at 2.65 min |
| **52** | 7-(difluoromethy l)-N-(4-fluoro-2-methoxypheny l)quinolln-4-amine | 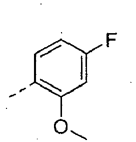 | A from compound 2 . and 4-fluoro-2-methoxyaniline | ¹H NMR (400 MHz, DMSO-*d*₆) δ 14.59 (s, 1H), 10.79 (s, 1H), 8.88 (d, J = 8.8 Hz, 1H), 8.55 (d, J = 7.0 Hz, 1H), 8.23 (d, J = 1.6 Hz, 1H), 7.96 (dd, J = 8.8, 1.6 Hz, 1H), 7.53 -7.19 (m, 3H), 7.00 (td, J = 8.4, 2.7 Hz, 1H), 6.40 (d, J = 7.0 Hz, 1H), 3.81 (s, 3H). | (Method C) *mlz* 319.4 [M+H]+ at 1.94 min |
| **53** | N-(4-chloro-2-methoxypheny l)-7-(difluoromethy l)quinolin-4-amine | 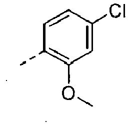 | A from compound 2 and 4-chloro-2-methoxyaniline | ¹H NMR (400 MHz, DMSO-*d*₆) δ 14.75 (br s, 1H), 10.88 (s, 1H), 8.90 (d, J = 8.8 Hz, 1H), 8.56 (d, J = 7.0 Hz, 1H), 8.26 (d, J = 1.7 Hz, 1H), 7.96 (dd, J = 9.0, 1.7 Hz, 1H), 7.53 -7.16 (m, 4H), 6.45 (d, J = 6.9 Hz, 1H), 3.83 (s, 3H). | (Method C) *m*/*z* 335.1/337.1 [M+H]⁺ at 1.99 min |
| **54** | 7-(difluoromethy l)-N-(4-methoxy-6-(2-methoxyethox y)pyridin-3-yl)quinolin-4-amine | 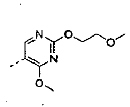 | A from compound 2 . and 19 | ¹H NMR (400 MHz, DMSO-*d₆*) δ 8.77 (s, 1H), 8.50 (d, J = 8.8 Hz, 1H), 8.45 (d, J = 5.3 Hz, 1H), 8.34 (s, 1H), 8.05 (d, J = 1.7 Hz, 1H), 7.67 (d, J = 8.7 Hz, 1H), 7.23 (t, J = 55.7 Hz, 1H), 6.24 (dd, J = 1.9, 5.2 Hz, 1H), 4.48 - 4.44 (m, 2H), 3.90 (s, 3H), 3.73 - 3.68 (m, 2H), 3.33 (s, 3H). | (Method C) *m*/*z* 377.0 [M+H]+ at 1.49 min |
| **55** | 7-(difluoromethy l)-N-(4-(2-methoxyethox y)-2-morpholinoph enyl)quinolin-4-amine | 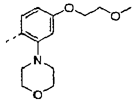 | A from compound 2 and 32 | ¹H NMR (400 MHz, DMSO-*d*₆) δ 8.62 (s, 1H), 8.57 (d, J = 8.7 Hz, 1H), 8.42 (d, J = 5.4 Hz, 1H), 8.02 (d, J = 1.8 Hz, 1H), 7.62 (dd, J = 8.9, 1.8 Hz, 1H), 7.22 (t, J = 55.8 Hz, 1H), 7.20 (d, J = 8.5 Hz, 1H), 6.70 (dd, J = 8.6, 2.7 Hz, 1H), 6.66 (d, J = 2.7 Hz, 1H), 6.33 (d, J = 5.3 Hz, 1H), 4.16-4.09 (m, 2H), 3.71 - 3.64 (m, 2H), 2.89 (t, J = 4.6 Hz, 4H). 7 protons (two CH₂ and one CH₃) hidden by DMSO peak, confirmed by HSQC. | (Method C) *mlz* 430.2 [M+H]⁺ at 1.93 min |
| **56** | N-(2-cyclopropoxy-4-(2-methoxyethox y)phenyl)-7-(difluoromethy l)quinolin-4amine | 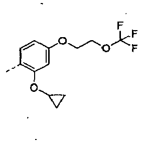 | J from compound 40 | ¹H NMR (400 MHz, DMSO-*d₆*) δ 14.46 (br s, 1H), 10.70 (s, 1H), 8.84 (d, J = 8.8 Hz, 1H), 8.51 (d, J = 7.0 Hz, 1H), 8.20 (d, J = 1.6 Hz, 1H), 7.94 (dd, J = 9.0,1.6 Hz, 1H), 7.36 (t, J = 55.2 Hz, 1H), 7.33 (d, J = 8.7 Hz, 1H), 7.11 (d, J = 2.7 Hz, 1H), 6.78 (dd, J = 8.7, 2.7 Hz, 1H), 6.33 (d, J = 7.0 Hz, 1H), 4.50 - 4.44 (m, 2H), 4.38 - 4.32 (m, 2H), 4.01 - 3.92 (m, 1H), 0.81 - 0.71 (m, 2H), 0.57-0.50 (m, 2H). | (Method C) *m*/*z* 455.2 [M+H]⁺ at 3.19 min |
| **57** | 7-(difluoromethy l)-N-(2-(methoxy-d3)-4-(2-(trifluorometho xy)ethoxy)phe nyl)quinolin-4-amine | 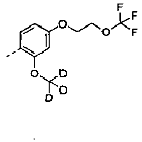 | J from compound 41 | ¹H NMR (400 MHz, DMSO-*d*₆) δ 14.48 (br s, 1H), 10.73 (s, 1H), 8.87 (d, J = 8.8 Hz, 1H), 8.52 (d, J = 7.0 Hz, 1H), 8.21 (d, J = 1.6 Hz, 1H), 7.94 (dd, J = 9.1,1.8 Hz, 1H), 7.36 (t, J = 55.2 Hz, 1H), 7.34 (d, J = 8.6 Hz, 1H), 6.88 (d, J = 2.6 Hz, 1H), 6.75 (dd, J = 8.6, 2.7 Hz, 1H), 6.37 (d, J = 7.0 Hz, 1H), 4.49 - 4.43 (m, 2H), 4.43 - 4.32 (m, 2H). | (Method C) *m*/*z* 432.2 [M+H]+ at 2.81 min |
| **58** | N-(4-chloro-2-methoxypheny l)-7-(difluoromethy l)quinolin-4-amine | 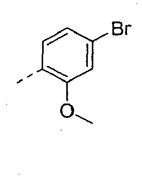 | A from compound 2 and 4-bromo-2-methoxyaniline | ¹H NMR (400 MHz, DMSO-*d*₆) δ 14.73 (br s, 1H), 10.87 (s, 1H), 8.90 (d, J = 8.8 Hz, 1H), 8.56 (d, J = 7.0 Hz, 1H), 8.25 (d, J = 1.7 Hz, 1H), 7.96 (dd, J = 8.9, 1.7 Hz, 1H), 7.52 (d, J = 1.9 Hz, 1H), 7.51 - 7.22 (m, 3H), 6.45 (d, J = 6.9 Hz, 1H), 3.83 (s, 3H). | (Method C) *m*/*z* 379.1/381.1 [M+H]⁺ at 2.45 min |
| **59** | N-(4-(1,1-difluoroethyl)-2-methoxypheny l)-7-(difluoromethy l)quinolin-4-amine | 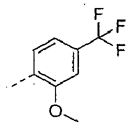 | A from compound 2 and 2-methoxy-4-(trifluorometh yl)aniline | ¹H NMR (400 MHz, DMSO-*d*₆) δ 14.85 (br s, 1H), 11.02 (s, 1H), 8.94 (d, J = 8.8 Hz, 1H), 8.58 (d, J = 7.0 Hz, 1H), 8.30 - 8.26 (m, 1H), 7.98 (dd, J = 8.9, 1.6 Hz, 1H), 7.68 (d, J = 8.1 Hz, 1H), 7.62 - 7.57 (m, 1H), 7.56 - 7.49 (m, 1H), 7.52 - 7.23 (m, 1H), 6.52 (d, J = 6.9 Hz, 1H), 3.90 (s, 3H). | (Method C) *m*/*z* 369.4 [M+H]⁺ at 2.65 min |
| **60** | 7-(difluoromethy l)-N-(2,4-dimethoxyphe nyl)quinolin-4-amine | 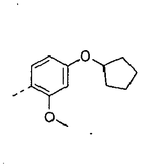 | A from compound 2 and 11 | ¹H NMR (400 MHz, DMSO-*d*₆) δ 14.27 (br s, 1H), 10.65 (s, 1H), 8.83 (d, J = 8.7 Hz, 1H), 8.51 (d, J = 7.0 Hz, 1H), 8.17 (s, 1H), 7.94 (d, J = 8.5 Hz, 1H), 7.35 (t, J = 55.2 Hz, 1H) ,7.29 (d, J = 8.6 Hz, 1H), 6.77 (d, J = 2.5 Hz, 1H), 6.68 (dd, J = 2.6, 8.7 Hz, 1H), 6.42 - 6.35 (m, 1H), 4.97 - 4.88 (m, 1H), 3.77 (s, 3H), 2.03 -1.89 (m, 2H), 1.81 -1.69 (m, 4H), 1.67 - 1.56 (m, 2H).. | (Method C) *m*/*z* 385.5 [M+H]+ at 3.28 min |
| **61** | 7-(difluoromethy l)-N-(4-methoxy-2-(2-methoxyethox y)pyrimidin-5-yl)quinolin-4-amine | 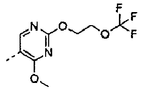 | A from compound 2 and 23 | ¹H NMR (400 MHz, DMSO-*d*₆) δ 8.79 (s, 1H), 8.51 (d, J = 8.8 Hz, 1H), 8.45 (d, J = 5.3 Hz, 1H), 8.37 (s, 1H), 8.09 - 8.03 (m, 1H), 7.72-7.64 (m, 1H), 7.24 (t, J = 55.7 Hz, 1H), 6.26 (d, J = 5.3 Hz, 1H), 4.64 -4.57 (m, 2H), 4.51 - 4.45 (m, 2H), 3.91 (s, 3H). | (Method C) *m*/*z* 431.3 [M+H]⁺ at 2.57 min |
| **62** | 7-(difluoromethy l)-N-(2-methoxy-4-(methylsulfony l)phenyl)quinol in-4-amine | 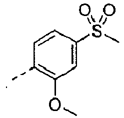 | A from compound 2 and 2-methoxy-4-(methylsulfon yl)aniline | ¹H NMR (400 MHz, DMSO-*d*₆) δ 14.58 (br s, 1H), 10.91 (s, 1H), 8.88 (d, J = 8.8 Hz, 1H), 8.62 (d, J = 6.9 Hz, 1H), 8.23 (d, J = 1.6 Hz, 1H), 8.00 (d, J = 8.8 Hz, 1H), 7.76 (d, J = 1.6 Hz, 1H), 7.73 - 7.68 (m, 2H), 7.37 (t, J = 55.2 Hz, 1H), 6.56 (d, J = 6.9 Hz, 1H), 3.92 (s, 3H), 3.35 (s, 3H). | (Method C) *mlz* 379.0 [M+H]⁺ at 1.46 min |
| **63** | N-cyclopropyl-4-((7-(difluoromethy l)quinolin-4-yl)amino)-3-methoxybenza mide | 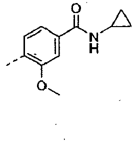 | A from compound 2 and 4-amino-N-cyclopropyl-3-methoxybenz amide | ¹H NMR (400 MHz, DMSO-*d*₆) δ 14.45 (br s, 1H), 10.83 (s, 1H), 8.86 (d, J = 8.8 Hz, 1H), 8.61 (d, J = 4.1 Hz, 1H), 8.58 (d, J = 6.9 Hz, 1H), 8.20 (s, 1H), 7.97 (d, J = 8.8 Hz, 1H), 7.70 (d, J = 1.8 Hz, 1H), 7.61 (dd, J = 8.1,1.8 Hz, 1H), 7.50 (d, J = 8.0 Hz, 1H), 7.36 (t, J = 55.2 Hz, 1H), 6.45 (d, J = 6.9 Hz, 1H), 3.86 (s, 3H), 2.97 - 2.79 (m, 1H), 0.79-0.70 (m, 2H), 0.67 - 0.50 (m,2H). | (Method C) *mlz* 384.2 [M+H]⁺ at 1.70 min |
| **64** | 7-(difluoromethy l)-N-(2-methoxy-4-(2-(trifluorometho xy)ethoxy)phe nyl)quinolin-4-amine | 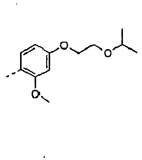 | A from compound 2 and 13 | ¹H NMR (400 MHz, DMSO-*d*₆) δ 14.51 (br s, 1H), 10:68 (s, 1H), 8.86 (d, J = 8.8 Hz, 1H), 8.51 (d, J = 6.9 Hz, 1H), 8.21 (d, J 1.6 Hz, 1H), 7.96 - 7.89 (m,1 H), 7.35 (t, J = 55.1 Hz, 1H), 7.30 (d, J = 8.6 Hz, 1H), 6.86 (d, J = 2.6 Hz, 1H), 6.71 (dd, J = 8.6, 2.6 Hz, 1H), 6.36 (d, J = 7.0 Hz, 1H), 4.20 - 4.13 (m, 2H), 3.78 (s, 3H), 3.76 - 3.70 (m, 2H), 3.66 (hept, J = 6.1 Hz, 1H), 1.14 (d, J = 6.1 Hz, 6H). | (Method C) *m*/*z* 403.4 [M+H]⁺ at 2.85 min |
| **65** | 7-(difluoromethy l)-N-(2-methoxy-6-(1H-pyrazol-4-yl)pyridin-3-yl)quinolin-4-amine | 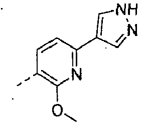 | A from compound 2 and 27 | ¹H NMR (400 MHz, DMSO-*d*₆) δ 14.71 (br s, 1H), 10.93 (s, 1H), 8.91 (d, J = 8.9 Hz, 1H), 8.58 (d, J = 7.0 Hz, 1H), 8.27 (d, J = 5.0 Hz, 3H), 7.98 (dd, J = 9.1,1.6 Hz, 1H), 7.84 (d, J = 7.9 Hz, 1H), 7.49 (d, J = 7.9 Hz, 1H), 7.38 (t, J = 55.2 Hz, 1H), 6.55 (d, J = 7.0 Hz, 1H). CH₃ hidden by DMSO, pyrazole NH missing. | (Method C) *mlz* 368.4 [M+H]⁺ at 1.95 min |

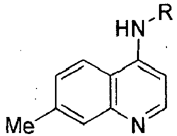

| **Example** | **Name** | **R** | **General Procedure** | **¹H NMR δ (400 MHz)** | **LCMS (ES+) m/z** |
|---|---|---|---|---|---|
| **66** | N-(4-(3,5-dimethyl-1H-pyrazol-4-yl)-2-methoxypheny l)-7-isopropylquino lin-4-amine | 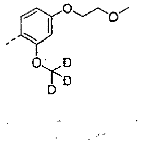 | A from 4-chloro-7-methylquinoline and compound 5 | ¹H NMR (400 MHz, DMSO-*d*₆) δ 8.34 (s, 1H), 8.27 (d, J = 8.6 Hz, 1H), 8.24 (d, J = 5.3 Hz, 1H), 7.63 - 7.58 (m, 1H), 7.30 (dd, J = 8.6, 1.8 Hz, 1H); 7.17 (d, J = 8.6 Hz, 1H), 6.74 (d, J = 2.6 Hz, 1H), 6.60 (dd, J = 8.5, 2.7 Hz, 1H), 6.03 (d, J = 5.3 Hz, 1H), 4.18 - 4.11 (m, 2H), 3.72 - 3.65 (m, 2H), 3.34 (s, 3H), 2.48 (s, 3H). | (Method C) *mlz* 342.2 [M+H]⁺ at 2.26 min |
| **67** | N-(4-methoxy-6-(2-methoxyethox y)pyridin-3-yl)-7-methylquinolin -4-amine | 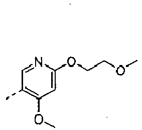 | A from 4-chloro-7-methylquinoline and compound 15 | ¹H NMR (400 MHz, DMSO-*d*₆) δ 14.31 (s, 1H), 10.45 (s, 1H), 8.60 (d, J = 8.7 Hz, 1H), 8.42 (d, J = 6.9 Hz, 1H), 8.09 (s, 1H), 7.80 (s, 1H), 7.65 (dd, J = 8.8, 1.6 Hz, 1H), 6.72 (s, 1H), 6.33 (d, J = 6.9 Hz, 1H), 4.46 -4.40 (m, 2H), 3.83 (s, 3H), 3.72 - 3.65 (m, 2H), 3.32 (s, 3H), 2.57 (s, 3H). | (Method C) *mlz* 340.1 [M+H]⁺ at 1.80 min |
| **68** | N-(2-methoxy-6-(2-methoxyethox y)pyridin-3-yl)-7-methylquinolin -4-amine | 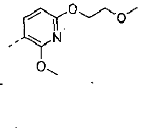 | A from 4-chloro-7-methylquinoline and compound 16 | ¹H NMR (400 MHz, DMSO-*d*₆) δ 14.31 (s, 1H), 10.45 (s, 1H), 8.60 (d, J = 8.7 Hz, 1H), 8.42 (d, J = 6.9 Hz, 1H), 8.09 (s, 1H), 7.80 (s, 1H), 7.65 (dd, J = 8.8, 1.6 Hz, 1H), 6.72 (s, 1H), 6.33 (d, J = 6.9 Hz, 1H), 4.46 - 4.40 (m, 2H), 3.83. (s, 3H), 3.72 - 3.65 (m, 2H), 3.32 (s, 3H), 2.57 (s, 3H). | (Method C) *mlz* 340.1 [M+H]⁺ at 2.16 min |
| **69** | N-(2-methoxy-6-(2-(trifluorometho xy)ethoxy)pyri din-3-yl)-7-methylquinolin -4-amine | 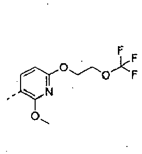 | A from 4-chloro-7-methyl-quinoline and compound 21 | ¹H NMR (400 MHz, DMSO-*d*₆) δ 14.24 (s, 1H), 10.46 (s, 1H), 8.59 (d, J = 8.8 Hz, 1H), 8.42 (d, J = 6.9 Hz, 1H), 7.83 - 7.74 (m, 2H), 7.65 (dd, J = 8.8, 1.7 Hz, 1H), 6.63 (d, J = 8.2 Hz, 1H), 6.33 (d, J = 6.9 Hz, 1H), 4.65 - 4.58 (m, 2H), 4.51 -4.45 (m, 2H), 3.89 (s, 3H), 2.57 (s, 3H). | (Method C) *mlz* 394.2 [M+H]⁺ at 3.09 min |
| **70** | N-(2-methoxy-4-(1H-pyrazol-4-yl)phenyl)-7-methylquinolin -4-amine | 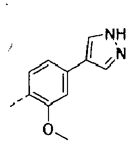 | A from 4-chloro-7-methylquinoline and compound 25 | ¹H NMR (400 MHz, DMSO-*d*₆) δ 14.24 (d, J = 6.2 Hz, 1H), 10.58 (s, 1H), 8.64 (d, J = 8.8 Hz, 1H), 8.39 (t, J = 6.6 Hz, 1H), 8.21 (s, 2H), 7.80 (d, J = 1.7 Hz, 1H), 7.64 (dd, J = 8.8,1.6 Hz, 1H), 7.50 (d, J = 1.5 Hz, 1H), 7.41 -7.32 (m, 2H), 6.34 (d, J = 7.0 Hz, 1H), 3.86 (s, 3H), 2.58 (s, 3H). One exchangeable not observed. | (Method C) m/z 331.4 [M+H]+ at 1.95 min |
| **71** | N-(4-(2-(difluorometho xy)ethoxy)-2-methoxypheny 1)-7-methylquinolin -4-amine | 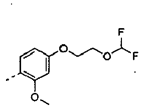 | A from 4-chloro-7-methylquinoline and compound 29 | ¹H NMR (400 MHz, DMSO-*d*₆) δ 9.19 (s, 1H), 8.40 (d, J = 8.6 Hz, 1H), 8.30 (d, J = 6.0 Hz, 1H), 7.66 (t, J = 1.4 Hz, 1H), 7.43 (dd, J = 8.7, 1.7 Hz, 1H), 7.24 (d, J = 8.6 Hz, 1H), 6.80 (d, J = 2.6 Hz, 1H), 6.78 (t, J = 75.8 Hz, 1H), 6.66 (dd, J = 8.6, 2.6 Hz, 1H), 6.12 (d, J = 6.0 Hz, 1H), 4.26 (dd, J = 6.0, 3.0 Hz, 2H), 4.20 (td, J = 4.0, 1.6 Hz, 2H), 3.75 (s, 3H), 2.51 (s, 3H). | (Method C) *mlz* 375.5 [M+H]+ at 2.65 min |
| **72** | N-(4-fluoro-2-methoxypheny 1)-7-methylquinolin -4-amine | 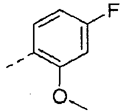 | A from 4-chloro-7-methyl-quinoline and 4-fluoro-2-methoxy - aniline | ¹H NMR (400 MHz, DMSO-*d*₆) δ 14.20 (s, 1H), 10.52 (s, 1H), 8.60 (d, J = 8.7 Hz, 1H), 8.41 (d, J = 6.9 Hz, 1H), 7.78 (t, J = 1.4 Hz, 1H), 7.65 (dd, J = 8.8, 1.7 Hz, 1H), 7.45 (dd, J = 8.7, 6.3 Hz, 1H), 7.24 (dd, J = 11.0, 2.7 Hz, 1H), 6.98 (td, J = 8.4, 2.7 Hz, 1H), 6.28 (d, J = 6.9 Hz, 1H), 3.80 (s, 3H), 2.57 (s, 3H). | (Method C) m/z 283.3 [M+H]⁺ at 1.98 min |
| **73** | N-(4-bromo-2-methoxypheny l)-7-methylquinolin -4-amine | 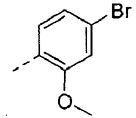 | A from 4-chloro-7-methylquinoline and 4-bromo-2-methoxyaniline | ¹H NMR (400 MHz, DMSO-*d*₆) δ 14.26 (s, 1H), 10.56 (s, 1H), 8.61 (d, J = 8.8 Hz, 1H), 8.42 (d, J = 7.0 Hz, 1H), 7.79 (s, 1H), 7.65 (dd, J = 8.8, 1.7 Hz, 1H), 7.50 (d, J = 2.0 Hz, 1H), 7.38 (d, J = 8.3 Hz, 1H), 7.34 (dd, J = 8.3, 2.0 Hz, 1H), 6.33 (d, J = 6.9 Hz, 1H), 3.82 (s, 3H), 2.57 (s, 3H). | (Method C) *mlz* 343.3/345.3 |
| **74** | N-(4-(2-methoxyethox y)-2-morpholinophe nyl)-7 - methylquinolin -4-amine | 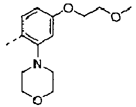 | A from 4-chlpro-7-methylquinoline and compound 32 | ¹H NMR (400 MHz, DMSO-*d*₆) δ 14.06 (br s), 10.50 (s, 1H), 8.64 (d, *J* = 8.7 Hz, 1H), 8.37 (t, *J* = 6.5 Hz, 1H), 7.77 (d, J = 1.7 Hz, 1H), 7.63 (dd, J = 8.8, 1.7 Hz, 1H), 7.28 (d, *J* = 8.6 Hz, 1H), 6.78 (dd, J = 8.6, 2.7 Hz, 1H), 6.75 (d, J = 2.7 Hz, 1H), 6.29 (d. J = 7.0 Hz, 1H), 4.19 - 4.13 (m, 2H), 3.71 - 3.67 (m, 2H), 3.33 (s, 3H), 3.33 - 3.28 (m, 4H), 2.93 - 2.87 (m, 4H), 2.57 (s, 3H). | (Method C) *mlz* 394.2 [M+H]⁺ at 2.43 min |
| **75** | N-(2-methoxy-4-(trifluoromethyl )phenyl)-7-methylquinolin -4-amine | 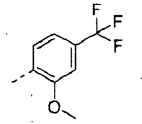 | A from 4-chloro-7-methylquinoline and 2-methoxy-4-(trifluoromet hyl)aniline | ¹H NMR (400 MHz, DMSO-*d*₆) δ 14.20 (br s, 1H), 10.64 (s, 1H), 8.61 (d, J = 8.7 Hz, 1H), 8.45 (d, J = 6.9 Hz, 1H), 7.79 (s, 1H), 7.71 - 7.62 (m, 2H), 7.59 (d, J = 1.9 Hz, 1H), 7.51 (dd, J = 8.2, 1.9 Hz, 1H), 6.41 (d, J = 6.9 Hz, 1H), 3.89 (s, 3H), 2.59 (s, 3H). | (Method C) m/z 333.2 [M+H]⁺ at 2.29 min |
| **76** | N-(2-methoxy-4-(methylsulfony l)phenyl)-7-methylquinolin -4-amine | 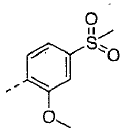 | A from 4-chloro-7-methylquinoline and 2-methoxy-4-(methylsulfo nyl)aniline | ¹H NMR (400 MHz, DMSO-*d*₆) δ 14.41 (br s, 1H), 10.74 (s, 1H), 8.65 (d, J = 8.7 Hz, 1H), 8.48 (d, J = 6.9 Hz, 1H), 7.82 (s, 1H), 7.74 (d, J = 1.6 Hz, 1H), 7.70 - 7.65 (m, 3H), 6.42 (d, J = 6.9 Hz, 1H), 3.91 (s, 3H), 3.34 (s, 3H), 2.59 (s, 3H). | (Method C) *mlz* 343.1 [M+H]⁺ at 1.39 min |
| **77** | N-(2-cyclopropoxy-4-(2-(trifluorometho xy)ethoxy)phe nyl)-7 -methylquinolin -4-amine | 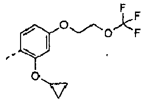 | J from 42 | ¹H NMR (400 MHz, DMSO-*d*₆) δ 14.07 (br s, 1H), 10.42 (s, 1H), 8.57 (d, J = 8.8 Hz, 1H), 8.37 (d, J = 7.0 Hz, 1H), 7.76 (d, J = 1.7 Hz, 1H), 7.63 (dd, J = 8.7,1.7 Hz, 1H), 7.31 (d, J = 8.7 Hz, 1H), 7.10 (d, J = 2.7 Hz, 1H), 6.77 (dd, J = 8.7, 2.7 Hz, 1H), 6.21 (d, J = 6.9 Hz, 1H), 4.52 - 4.43 (m, 2H), 4.39-4.31 (m, 2H),4.00 - 3.91 (m, 1H), 2.57 (s, 3H), 0.80 - 0.70 (m, 2H), 0.56 - 0.47 (m, 2H). | (Method C) *mlz* 419.2 [M+H]⁺ at 3.18 min |
| **78** | N-(2-(methoxy-d3)-4-(2-(trifluorometho xy)ethoxy)phe nyl)-7 - methylquinolin -4-amine | 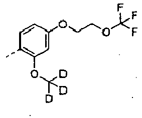 | J from 38 | ¹H NMR (400 MHz, DMSO-*d*₆) δ 14.12 (br s, 1H), 10.44 (s, 1H), 8.59 (d, J = 8.7 Hz, 1H), 8.38 (d, J = 7.0 Hz, 1H), 7.79 - 7.74 (m, 1H), 7.63 (dd, J = 8.8, 1.6 Hz, 1H), 7.32 (d, J = 8.6 Hz, 1H), 6.87 (d, J = 2.6 Hz, 1H), 6.73 (dd, J = 8.6,2.6 Hz, 1H), 6.24 (d, J = 7.0 Hz, 1H), 4.49 -4.42 (m, 2H), 4.38 - 4.32 (m, 2H), 2.57 (s, 3H). | (Method C) *mlz* 396.2 [M+H]⁺ at 2.76 min . |
| **79** | N-(5-bromo-3-methoxypyrazi n-2-yl)-7-methylquinolin -4-amine | 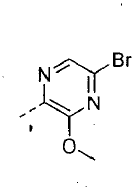 | A from 4-chloro-7-methylquinoline and 5-bromo-3-methoxypyra zin-2-amine | ¹H NMR (400 MHz, DMSO-*d*₆) δ 9.00 (s, 1H), 8.71 (s, 1H), 8.05 (d, J = 8.7 Hz, 1H), 7.94 (s, 1H), 7.92 (s, 1H), 7.77 (s, 1H), 7.43 (d, J = 8.6 Hz, 1H), 4.07 (s, 3H), 2.52 (s, 3H). | (Method C) m/z 345.1/347.1 [M+H]⁺ at 2.10 min |
| **80** | N-(4-methoxy-2-(2-methoxyethox y)pyrimidin-5-yl)-7-methylquinolin -4-amine | 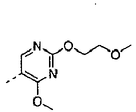 | A from 4-chloro- 7-methylquinoline and 19 | ¹H NMR (400 MHz, DMSO-*d*₆) δ 14.24 (br s, 1H), 10.47 (s, 1H), 8.57 (d, J = 8.7 Hz, 1H), 8.48 (d, J = 6.6 Hz, 2H), 7.79 (s, 1H), 7.69 (dd, J = 1.6, 8.8 Hz, 1H), 6.54 (d, J = 6.9 Hz, 1H), 4.52 - 4.46 (m, 2H), 3.93 (s, 3H), 3.74 - 3.68 (m, 2H), 3.33 (s, 3H), 2.58 (s, 3H). | (Method C) *mlz* 341.1 [M+H]+ at 1.53 min |
| **81** | N-(4-(cyclopentylox y)-2-methoxypheny l)-7-methylquinolin -4-amine | 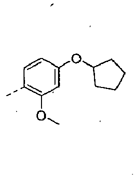 | A from 4-chloro-7-methylquinoline and 11 | ¹H NMR (400 MHz, DMSO-*d*₆) δ 13.97 (br s, 1H), 10.41 (s, 1H), 8.62 - 8.53 (m, 1H), 8.37 (d, J = 7.0 Hz, 1H), 7.74 (s, 1H), 7.63 (dd, J = 1.7, 8.7 Hz, 1H), 7.27 (d, J = 8.6 Hz, 1H), 6.76 (d, J = 2.6 Hz, 1H), 6.69 - 6.64 (m, 1H), 6.26 (d, J = 6.9 Hz, 1H), 4.95 - 4.88 (m, 1H), 3.76 (s, 3H), 2.57 (s, 3H), 2.02 -1.90 (m, 2H), 1.81 - 1.69 (m, 4H), 1.67 -1.57 (m, 2H). | (Method C) *mlz* 349.5 [M+H]+ at 3.28 min |
| **82** | N-(4-methoxy-2-(2-(trifluorometho xy)ethoxy)pyri midin-5-yl)-7-methylquinolin -4-amine | 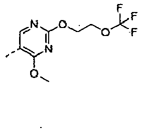 | A from 4-chloro-7-methylquinoline and 23 | ¹H NMR (400 MHz, DMSO-*d*₆) δ 8.55 (br s, 1H), 8.36 - 8.28 (m, 2H), 8.24 (d, J = 8.6 Hz, 1H), 7.65 (s, 1H), 7.39 - 7.32 (m, 1H), 6.09 (d, J = 5.3 Hz, 1H), 4.63 - 4.56 (m, 2H), 4.51-4.44 (m, 2H), 3.90 (s, 3H), 2.49 (s, 3H). | (Method C) *mlz* 395.4 [M+H]⁺ at 2.55 min |
| **83** | N-(4-chloro-2-methoxypheny 1)-7-methylquinolin -4-amine | 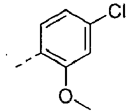 | A from 4-chloro-7-methylquinoline and 4-chloro-2-methoxyaniline | ¹H NMR (400 MHz, DMSO-*d*₆) δ 14.00 (br s, 1H), 10.52 (s, 1H), 8.58 (d, J = 8.7 Hz, 1H), 8.43 (d, J = 7.0 Hz, 1H), 7.75 (s, 1H). 7.66 (d, J = 8.9 Hz, 1H), 7.44 (d, J = 8.3 Hz, 1H), 7.40 (d, J = 2.2 Hz, 1H), 7.21 (dd, J = 8.3, 2.3 Hz, 1H), 6.33 (d, J = 7.0 Hz, 1H), 3.82 (s, 3H), 2.58 (s, 3H). | (Method C) *mlz* 299.0 [M+H]⁺ at 2.14 min |
| **84** | N-(4-(2-isopropoxyeth oxy)-2-methoxypheny l)-7-methylquinolin -4-amine | 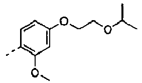 | A from 4-chloro-7-methylquinoline and 13 | ¹H NMR (400 MHz, DMSO-*d*₆) δ 14.14 (br s, 1H), 10.44 (s, 1H), 8.60 (d, J = 8.7 Hz, 1H), 8.37 (d, J = 7.0 Hz, 1H), 7.80 - 7.74 (m, 1H), 7.66 - 7.59 (m, 1H), 7.28 (d, J = 8.6 Hz, 1H), 6.84 (d, J = 2.6 Hz, 1H), 6.70 (dd, J = 8.6, 2.6 Hz, 1H), 6.24 (d, J = 7.0 Hz, 1H), 4.19 - 4.12 (m, 2H), 3.77 (s, 3H), 3.75 - 3.72 (m, 2H), 3.65 (hept, J = 6.1 Hz, 1H), 2.57 (s, 3H), 1.13 (d, J = 6.1 Hz, 6H). | (Method C) *mlz* 367.5 [M+H]⁺ at 2.83 min |
| **85** | N-(2-methoxy-6-(1H-pyrazol-4-yl)pyridin-3-yl)-7-methylquinolin -4-amine | 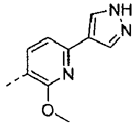 | A from 4-chloro-7-methylquinoline and 27 | ¹H NMR (400 MHz, DMSO-*d*₆) δ 14.35 (br s, 1H), 10.65 (s, 1H), 8.64 (d, J =.8.7 Hz, 1H), 8.43 (t, J = 6.2 Hz, 1H), 8.26 (s, 2H), 7.86 - 7.79 (m, 2H), 7.66 (d, J = 8.8 Hz, 1H), 7.46 (dd, J = 7.9,1.4 Hz, 1H), 6.41 (dd, J = 6.9, 1.3 Hz, 1H), 2.58 (s, 3H). CH₃ hidden by DMSO; pyrazole NH missing. | (Method C) *mlz* 332.4 [M+H]⁺ at 1.95 min |
| **86** | N-(2-methoxy-4-(1H-pyrazol-1-yl)phenyl)-7 - methylquinolin -4-amine | 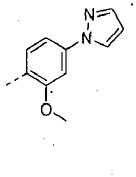 | A from 4-- chloro-7-methylquinoline and 2-Methoxy-4-(1h-pyrazol-1-yl)aniline | ¹H NMR (400 MHz, DMSO-*d*₆) δ 14.37 (br s, 1H), 10.65 (s, 1H), 8.67 (d, *J* = 8.7 Hz, 1H), 8.46 (s, 1H), 8.43 (d, *J* = 8.9 Hz, 2H), 7.83 (s, 1H), 7.66 (m, 3H), 7.56 (d, *J* = 8.5 Hz, 1H), 7.50 (d, *J* = 8.6 Hz, 1H), 6.37 (d, *J* = 6.9 Hz, 1H), 3.89 (s, 3H), 2.59 (s, 3H), 2.15 (s, 3H). | (Method E) m/z 331.4 [M+H]⁺ at 3.16 min |
| **87** | N-(4-(3,5-dimethyl-1H-pyrazol-4-yl)-2-methoxypheny 1)-7-methylquinolin -4-amine | 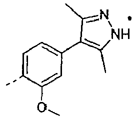 | K from example 73 | ¹H NMR (400 MHz, DMSO-*d*₆) δ 12.31 (s, 1H), 8.66 (s, 1H), 8.36-8.30 (m, 2H), 7.65 (s, 1H), 7.44-. 7.30 (m, 2H), 7.04 (s, 1H), 6.95 (d, 3J= 8.0 Hz, 1H), 6.29 (d, 3J= 5.5 Hz, 1H), 3.79 (s, 3H), 2.27 (s, 6H). | (Method E) *mlz* 359.6 [M+H]+ at 5.35 min |
| **88** | N-(4-(furan-2-yl)-2-methoxypheny l)-7-methylquinolin -4-amine | 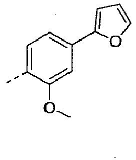 | K from example 73 | ¹H NMR (400 MHz, DMSO-*d*₆) δ 14.43 (s, 1H), 10.66 (s, 1H), 8.66 (d, 3J= 8.8 Hz, 1H), 8.41 (d,3J= 6.8 Hz, 1H), 7.85 -7.80 (m, 2H), 7.65 (dd, 3J= 8.7Hz,4J =1.7Hz, 1H), 7.54 (d, 4J= 1.5 Hz, 1H), 7.51 -7.42 (m, 2H), 7.14 (d, 3J= 3,4 Hz, 1H), 6.66 (dd, 3J= 3.4Hz, 4J= 1.5Hz, 1H), 6.35 (d, 3J= 6.8Hz, 1H), 3.87 (s, 3H), 2.58 (s, 3H). | (Method E) *mlz* 331.4 [M+H]⁺ at 6.16 min |
| **89** | N-(4-(furan-3-yl)-2-methoxypheny l)-7-methylquinolin -4-amine | 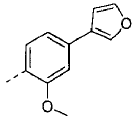 | K from example 73 | ¹H NMR (400 MHz, DMSO-*d*₆) δ 14.37 (s, 1H), 10.63 (s, 1H), 8.66 (d, 3J = 8.7 Hz, 1H), 8.40 (d, 3J= 7.0 Hz, 1H), 8.35 (s, 1H), 7.84 - 7.77 (m, 2H), 7.64 (d, 3J= 8.9 Hz, 1H), 7.49 (s, 1H), 7.45 -7.34 (m, 2H), 7.10 (d, 4J= 1.9 Hz, 1H), 6.34 (d, 3J= 7.0 Hz, 1H), 3.86 (s, 3H), 2.57 (s, 3H). | (Method E) *mlz 331.6* [M+H]⁺ at 6.02 min |
| **90** | N-(2-methoxy-4-(1H-pyrazol-5-yl)phenyl)-7 - methylquinolin -4-amine | 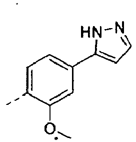 | K from example 73 | ¹H NMR (400 MHz, DCM-d2) δ 8.53 (d, 3J= 5.4 Hz, 1H), 7.94 (d, 3J= 8.5 Hz, 1H), 7.83 (s, 1H), 7.65 (d,3J= 2.3 Hz, 1H), 7.54 (d, 3J= 8.2 Hz, 1H), 7.49 (d, 4J= 1.8 Hz, 1H), 7.44-7.37 (m, 2H), 7.13 (d, 3J= 5.4 Hz, 1H), 6.65 (d, 3J= 2.3 Hz, 1H), 3.99 (s, 3H), 2.56 (s, 3H). | (Method E) *m*/*z* 331.40 [M+H]⁺ at 5.34 min |
| **91** | N-(2-methoxy-4-(4-methyl-1H-pyrazol-1-yl)phenyl)-7-methylquinolin -4-amine | 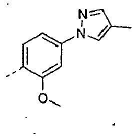 | A from 4-chloro-7-methylquinoline and 2-methoxy-4-(4-methyl-1H-pyrazol-1-yl)aniline | ¹H NMR (400 MHz, DMSO-*d*₆) δ 14.37 (s, 1H), 10.65 (s, 1H), 8.67 (d, *J* = 8.7 Hz, 1H), 8.46 (s, 1H), 8.43 (d, *J* = 8.9 Hz, 2H), 7.83 (s, 1H), 7.72 - 7.62 (m, 2H), 7.60 - 7.46 (m, 2H), 6.37 (d, *J* = 6.9 Hz, 1H), 3.89 (s, 3H), 2.59 (s, 3H), 2.15 (s, 3H). | (Method E) *mlz* 345.40 [M+H]⁺ at 3.20 min |
| **92** | N-(4-(1,3-dimethyl-1H-pyrazol-4-yl)-2-methoxypheny 1)-7-methylquinolin -4-amine ' | 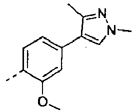 | K from example 73 | ¹H NMR (400 MHz, DCM-d2) δ 8.52 (d, 3J= 5.3 Hz, 1H), 7.92 (d, 3J= 8.6 Hz, 1H), 7.81 (s, 1H), 7.52 -7.46 (m, 2H), 7.39 (dd, 3J= 8.6 Hz, 4J= 1.9Hz, 1H), 7.07 (d, 3J= 5.3 Hz, 1H), 7.07 -7.00 (m, 2H), 3.93 (s, 3H), 3.85 (s, 3H), 2.56 (s, 3H), 2.40 (s, 3H) | (Method E) *mlz* 359.40 [M+H]⁺ at 5.56 min |
| **93** | N-(2-methoxy-4-(thiophen-2-yl)phenyl)-7 - methylquinolin -4-amine | 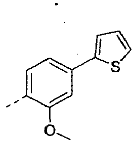 | K from example 73 | ¹H NMR (400 MHz, DMSO-d6) δ 14.39 (s, 1H), 10.65 (s, 1H), 8.66 (s, 1H), 8.41 (d,3J= 6.9 Hz, 1H), 7.82 (s, 1H), 7.71 -7.60 (m, 3H), 7.51 (s, 1H), 7.42 (s, 2H), 7.20 (s, 1H), 6.37 (d,37.0 Hz, 1H), 3.88 (s, 3H), 2.58 (s, 3H). | (Method E) *mlz* 347.40 [M+H]+ at 6.25 min |
| **94** | N-(2-methoxy-4-(thiophen-3-yl)phenyl)-7-methylquinolin -4-amine | 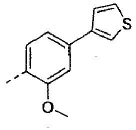 | K from example 73 | ¹H NMR (400 MHz, DMSO-d6) δ 14.02 (s, 1H), 10.57 (s, 1H), 8.62 (d, 3J= 8.8 Hz, 1H), 8.42 (d, 3J= 7.0Hz, 1H), 8.07 (s, 1H), 7.80 - 7.39 (m, 7H), 6.36 (d, 3J= 7.0 Hz, 1H), 3.89 (s, 3H), 2.58 (s, 3H). | (Method E) *mlz* 347.40 [M+H]+ at 6.13 min |
| **95** | N-(7-bromobenzo[d ][1,3]dioxol-4-yl)-7-methylquinolin -4-amine | 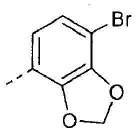 | A from 4-chloro-7-methylquinoline and 7-bromobenzo[ d][1,3]dioxol-4-amine | ¹H NMR (400 MHz, DMSO-*d*₆) δ 14.53 (br s, 1H), 10.83 (s, 1H), 8.65 (d, *J* = 8.7 Hz, 1H), 8.52 (d, *J* = 6.9 Hz, 1H), 7.85 (s, 1H), 7.68 (dd, *J* = 8.7,1.7 Hz, 1H), 7.24 (d, *J* = 8.8 Hz, 1H), 6.96 (d, *J* = 8.8 Hz, 1H), 6.70 (d, *J* = 6.9 Hz, 1H), 6.23 (s, 2H), 2.59 (s, 3H). | (Method E) *mlz* 357.20 [M+H]⁺ at 3.17 min |
| **96** | N-(7-(1H-pyrazol-4-yl)benzo[d][1,3 ]dioxol-4-yl)-7-methylquinolin -4-amine | 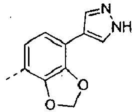 | K from example 95 | ¹H NMR (400 MHz, Methanol-*d*₄) δ 8.25 (d, *J* = 6.1 Hz, 1H), 8.17 (d, *J* = 8.6 Hz, 1H), 7.99 (m, 2H), 7.60 (s, 1H), 7.40 (dd, *J* = 8.7, 1.7 Hz, 1H), 7.18 (d, *J* = 8.6 Hz, 1H), . 6.84 (d, *J* = 8.6 Hz, 1H), 6.51 (d, *J* = 6.1 Hz, 1H), 6.04 (s, 2H), 2.48 (s, 3H). | (Method E) *mlz* 345.40 [M+H]+ at 2.98 min |

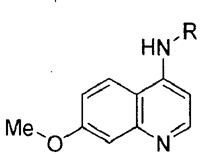

| **Example** | **Name** | **R** | **General Procedure.** | **¹H NMR δ (400 MHz)** | **LCMS (ES+) m/z** |
|---|---|---|---|---|---|
| **97** | 7-methoxy-N-(2-(methoxy-d3)-4-(2-methoxyethox y)phenyl)quin olin-4-amine | 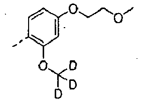 | A from 4-chloro-7-methoxyquin oline and compound 5 | ¹H NMR (400 MHz, DMSO-*d₆*) δ 8.34 (s, 1H), 8.29 (d, J = 9.3 Hz, 1H), 8.21 (d, J = 5.3 Hz, 1H), 7.19 (d, J = 2.6 Hz, 1H), 7.16 (d, J = 8.5 Hz, 1H), 7.09 (dd, J = 9.2, 2.7 Hz, 1H), 6.74 (d, J = 2.6 Hz, 1H), 6.60 (dd, J = 8.6, 2.7 Hz, 1H), 5.98 (d, J = 5.4 Hz, 1H), 4.18 - 4.11 (m, 2H), 3.88 (s, 3H), 3.72 - 3.65 (m, 2H), 3.33 (s, 3H). | (Method C) *mlz* 358.2 [M+H]⁺ at 2.27 min |
| **98** | N-(4-(2-(difluorometho xy)ethoxy)-2-methoxypheny l)-7-methoxyquinol in-4-amine | 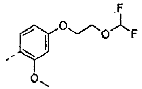 | A from 4-chloro-7-methoxyquin oline and compound 29 | ¹H NMR (400 MHz, DMSO-*d*₆) δ 8.86 (s, 1H), 8.37 (d, J = 9.2 Hz, 1H), 8.24 (d, J = 5.8 Hz, 1H), 7.25 - 7.20 (m, 2H), 7.18 (dd, J = 9.2, 2.6 Hz, 1H), 6.98 - 6.58 (m, 2H), 6.65 (dd, J = 8.6, 2.7 Hz, 1H), 6.03 (d, J = 5.8 Hz, 1H), 4.28 - 4.22 (m, 2H), 4.22 - 4.16 (m, 2H), 3.90 (s, 3H), 3.75 (s, 3H). | (Method C) *m*/*z* 391.4 [M+H]⁺ at 2.63 min |

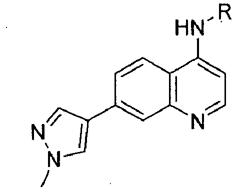

| **Example** | **Name** | **R** | **General Procedure** | **¹H NMR δ (400 MHz)** | **LCMS (ES+) m/z** |
|---|---|---|---|---|---|
| **99** | N-(4-(2-(difluorometho xy)ethoxy)-2-methoxypheny l)-7-(1-methyl-1H-pyrazol-4-yl)quinolin-4-amine | 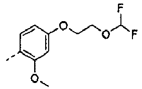 | A from compound 3 and 29 | ¹H NMR (400 MHz, DMSO-*d*₆) δ 8.53 (s, 1H), 8.38 (d, J = 8.8 Hz, 1H), 8.34 (s, 1H), 8.27 (d, J = 5.4 Hz, 1H), 8.05 (d, J = 0.8 Hz, 1H), 7.97 (d, J = 1.8 Hz, 1H), 7.73 (dd, J = 8.7, 1.9 Hz, 1H), 7.21 (d, J = 8.5 Hz, 1H), 6,78 (t, J = 75.8 Hz, 1H), 6.78 (d, J = 2.6 Hz, 1H), 6.64 (dd, J = 8.6, 2.7 Hz, 1H), 6.04 (d, J = 5.4 Hz, 1H), 4:28 - 4.22 (m, 2H), 4.22 - 4.16 (m, 2H), 3.90 (s, 3H), 3.75 (s, 3H). | (Method C) *m*/*z* 441.4 [M+H]⁺ at 2.67 min |

### Pharmalonical Part

### Example 100: Evalution of pharmacological activities of inhibiting proliferation, inducing senescence, and degrading NIK

### Proliferation and Senescence

A375 (American Type Culture Collection; CRL-1619) cells were seeded into 96 well plates (1,200 cells/well; Perkin Elmer; 6005225) and incubated overnight at 37°C, 5% CO₂. Compounds prepared in media (Dulbecco's modified Eagle medium + 10% foetal bovine serum + 1% penicillin/streptomycin) were added to the cells and incubated for four days at 37°C, 5% CO₂. Media was then aspirated from the cells and freshly prepared compounds were added to the cells, which were then incubated for a further three days at 37°C, 5% CO₂. After a total of seven days in the presence of compounds, media was removed, and the cells were labelled with Hoechst (1 in 1,000; Thermo Fisher; 62249), TOTO^{™}-3 (1 in 10,000; Thermo Fisher; T3604) and SPiDER βGAL (1.0 µM; Sigma-Aidrich; 94433) prepared in Hanks' Balanced Salt Solution supplemented with 1.0 mM HEPES buffer for 20 minutes at 37°C. Cells were imaged by confocal microscopy using an ImageXpress (Molecular Devices) at x10 magnification. Images were analysed using MetaXpress software (Molecular Devices).

### NIK degradation Western-Blot experiments

A375 cells were plated in 6-well dishes at 2,000 cells/cm². The day after, cells were treated for 72 hrs with compounds of the examples at 10 µM. Cells were treated during the last 4 hours of the treatment period with 5 µM MG132 to inhibit proteasomal degradation. For protein isolation, cells were washed twice with PBS and then lysed using RIPA Buffer (1X) containing protease and phosphatase inhibitors. Following sonication, the lysate was centrifuged at 20,000 g for 10 min at 4°C and the supernatants were collected and assayed for protein concentration using the BCA kit (Pierce).

Total protein (20 µg) was separated by electrophoresis on 10% SDS-polyacrylamide gels and transferred to PVDF membranes (Millipore). Blocking was performed in Tris-buffer saline containing 3% BSA. Membranes were incubated with the appropriate primary antibodies overnight at 4°C (NIK, CST#4994, 1/2000) and β-actin (Santa-Cruz, sc-47778, 1/5000). After washing three times, membranes were incubated with horseradish peroxidase-conjugated secondary antibodies for 1 h. Bound antibodies were detected using an enhanced chemiluminescence plus kit (GE Healthcare). NIK degradation was tested and confirmed for the compounds of Examples 3, 4, 5, 6, 10 and 11 (see the corresponding table below). For illustration, the Western blot analysis of NIK degradation in cells treated with the compound of Example 3 (at 10 µM for 72 h) is shown in Figure 1.

### Proliferation activity

| | **Examples** |
|---|---|
| Proliferation IC₅₀ > 5 µM and < 10 µM | 2, 3, 6, 12, 13, 14, 15, 16, 20, 23, 24, 26, 28, 29, 30, 32, 34, 52, 56, 58, 59, 61, 72, 74, 75, 77, 79, 80, 82, 83, 84, 97 |
| Proliferation IC₅₀ < 5 µM | 1, 4, 5, 7, 8, 9, 10, 11, 17, 18, 19, 21, 22, 25, 27, 31, 33, 35, 36, 37, 38, 39, 40, 41, 42, 43, 44, 45, 46, 47, 48, 49, 50, 51, 53, 54, 55, 57, 60, 62, 63, 64, 65, 66, 67, 68, 69, 70, 71, 73, 76, 78, 81, 85, 86, 87, 88, 89, 90, 98, 99 |

### Senescence activity

| | Examples |
|---|---|
| Senescence EC₅₀ > 5 µM and < 10 µM | 2, 3, 6, 13, 16, 17, 18, 19, 23, 24, 27, 28, 29, 30, 31, 32, 33, 34, 37, 41, 42, 43, 45, 46, 48, 52, 54, 55, 56, 58, 59, 61, 62, 63, 72, 74, 75, 77, 79, 80, 82, 84, 97 |
| Senescence EC₅₀ < 5 µM | 1, 4, 5, 7, 8, 9, 10, 11, 12, 14, 15, 20, 21, 22, 25, 26, 35, 36, 38, 39, 40, 44, 47, 49, 50, 51, 53, 57, 60, 66, 67, 68, 69, 70, 71, 73, 76, 78, 81, 83, 85, 86, 87, 88, 89, 90, 98, 99 |

### NIK degradation

| | **Examples** |
|---|---|
| NIK degradation activity | 3, 4, 5, 6, 10, 11 |

## Claims

1. A compound of the following formula (I): 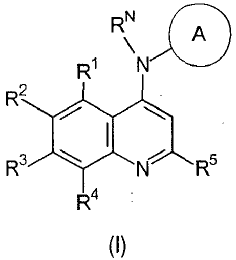 or a pharmaceutically acceptable salt thereof, wherein:
ring A is an aromatic group selected from the groups (A-1), (A-2), (A-3), (A-4), (A-5), (A-6), and (A-7): 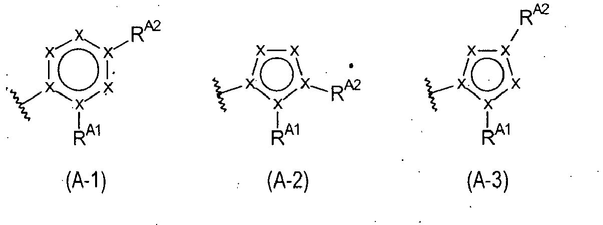 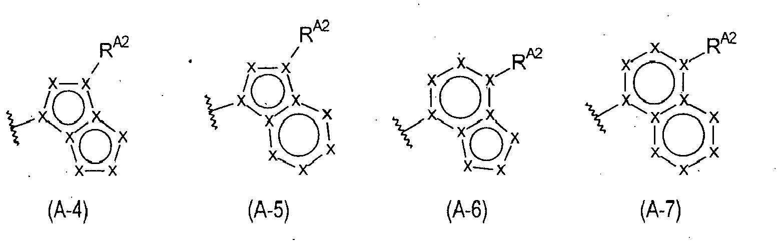
wherein the group (A-1) is phenyl or a 6-membered monocyclic heteroaryl, wherein said phenyl or said heteroaryl is substituted with a group R^{A1} and a group R^{A2} in the indicated positions, and is optionally further substituted with one or more groups R^{A3};
wherein the groups (A-2) and (A-3) are each a 5-membered monocyclic heteroaryl which is substituted with a group R^{A1} and a group R^{A2} in the indicated positions, and is optionally further substituted with one or more groups R^{A3};
wherein the group (A-4) is an 8-membered bicyclic heteroaryl which is substituted with a group R^{A2} in the indicated position and is optionally further substituted with one or more groups R^{A3}; wherein the groups (A-5) and (A-6) are each a 9-membered bicyclic heteroaryl which is substituted with a group R^{A2} in the indicated position and is optionally further substituted with one or more groups R^{A3}; and
wherein the group (A-7) is naphthyl or a 10-membered bicyclic heteroaryl, wherein said naphthyl or said heteroaryl is substituted with a group R^{A2} in the indicated position and is optionally further substituted with one or more groups R^{A3};
R^{A1} is selected from -O(C₁₋₅ alkyl), -O(C₂₋₅ alkenyl), C₁₋₅ alkyl, C₂₋₅ alkenyl, -N(C₁₋₅ alkyl)(C₁₋₅ alkyl), halogen, C₁₋₅ haloalkyl, -O-(C₁₋₅ haloalkyl), -CN, -(C₀₋₃ alkylene)-cycloalkyl, and -(C₀₋₃ alkylene)-heterocycloalkyl, wherein the cycloalkyl group in said -(C₀₋₃ alkylene)-cycloalkyl and the heterocycloalkyl group in said -(C₀₋₃ alkylene)-heterocycloalkyl are each optionally substituted with one or more groups R^{Cyc}, and further wherein one or more -CH₂- units comprised in the alkylene in said -(C₀₋₃ alkylene)-cycloalkyl or said -(C₀₋₃ alkylene)-heterocycloalkyl are each optionally replaced by a group independently selected from -O-, -NH- , -N(C₁₋₅ alkyl)-, -CO-, -S-, -SO-, and -SO₂-;
R^{A2} is selected from C₁₋₁₂ alkyl, -(C₁₋₁₂ alkylene)-OH, -(C₀₋₅ alkylene)-O(C₁₋₁₂ alkyl), -(C₀₋₅ alkylene)-O(C₁₋₁₂ haloalkyl), -(C₀₋₅ alkylene)-O-(C₁₋₁₂ alkylene)-OH, -(C₀₋₅ alkylene)-O-(C₁₋₅ alkylene)-O(C₁₋₁₂ alkyl), -(C₀₋₅ alkylene)-O-(C₁₋₅ alkylene)-O(C₁₋₁₂ haloalkyl), -CO(C₁₋₁₂ alkyl), -CO(C₁₋₁₂ haloalkyl), -SO₂-(C₁₋₁₂ alkyl), -SO₂-(C₁₋₁₂ haloalkyl), halogen, C₁₋₅ haloalkyl, -(C₀₋₅ alkylene)-carbocyclyl, and -(C₀₋₅ alkylene)-heterocyclyl, wherein one or more -CH₂- units in said C₁₋₁₂ alkyl, said -(C₁₋₁₂ alkylene)-OH, said -(C₀₋₅ alkylene)-O(C₁₋₁₂ alkyl), said -(C₀₋₅ alkylene)-O(C₁₋₁₂ haloalkyl), said -(C₀₋₅ alkylene)-O-(C₁₋₁₂ alkylene)-OH, said -(C₀₋₅ alkylene)-O-(C₁₋₅ alkylene)-O(C₁₋₁₂ alkyl), said -(C₀₋₅ alkylene)-O-(C₁₋₅ alkylene)-O(C₁₋₁₂ haloalkyl), said -CO(C₁₋₁₂ alkyl), said -CO(C₁₋₁₂ haloalkyl), said -SO₂-(C₁₋₁₂ alkyl) or said SO₂ (C₁₋₁₂ haloalkyl) arc cach optionally replaced by a group independently selected from -O-, -NH-, -N(C₁₋₅ alkyl)-, -CO-, and -CH=CH-, wherein the carbocyclyl group in said -(C₀₋₅ alkylene)-carbocyclyl and the heterocyclyl group in said -(C₀₋₅ alkylene)-heterocyclyl are each optionally substituted with one or more groups R^{Cyc}, and further wherein one or more -CH₂- units comprised in the alkylene in said -(C₀₋₅ alkylene)-carbocyclyl or said -(C₀₋₅ alkylene)-heterocyclyl are each optionally replaced by a group independently selected from -O-, -NH-, -N(C₁₋₅ alkyl)-, -CO-, -S-, -SO-, and -SO₂-;
each R^{A3}, if present, is independently selected from C₁₋₅ alkyl, C₂₋₅ alkenyl, C₂₋₅ alkynyl, -(C₀₋₃ alkylene)-OH, -(C₀₋₃alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH_{z}, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂₋(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-;
R^{N} is selected from hydrogen, C₁₋₅ alkyl, and -CO-(C₁₋₅ alkyl);
R¹, R⁴ and R⁵ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH2, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, -(C₀₋₃ alkylene)-heterocyclyl, and -R⁶-R⁷, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-;
R² is selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CN, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(C₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-carbocyclyl, and -(C₀₋₃ alkylene)-heterocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, and -SO-;
R³ is selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-S(C₁₋₅ alkylene)-SH, -(C₀₋₃ alkylene)-S(C₁₋₅ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-NH-OH, -(C₀₋₃ alkylene)-N(C₁₋₅ alkyl)-OH, -(C₀₋₃ alkylene)-NH-O(C₁₋₅ alkyl), -(C₀₋₃ alkylene)-N(C₁₋₅ alkyl)-O(C₁₋₅ alkyl), -(C₀₋₃ alkylene)-CHF₂, -(C₀₋₃ alkylene)-CH₂F, -(C₀₋₃ alkylene)-(C₂₋₅ haloalkyl), -(C₀₋₃ alkylene)-O-(C₁₋₅ haloalkyl), -(C₀₋₃ alkylene)-CHO, -(C₀₋₃ alkylene)-CO-(C₁₋₅ alkyl), -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-COO(C₁₋₅ alkyl), -(C₀₋₃ alkylene)-N(C₁₋₅ alkyl)-COO(₁₋₅ alkyl), -(C₀₋₃ alkylene)-O-CO-NH(C₁₋₅ alkyl), -(C₀₋₃ alkylene)-O-CO-N(C₁₋₅ alkyl)(C₁₋₅ alkyl), -(C₀₋₃ alkylene)-SO₂-NH_{z}, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl group in said -(C₀₋₃ alkylene)-carbocyclyl and the heterocyclyl group in said -(C₀₋₃ alkylene)-heterocyclyl are each optionally substituted with one or more groups R^{Cyc}, and further wherein one or more -CH₂- units comprised in the alkylene in said -(C₀₋₃ alkylene)-carbocyclyl or said -(C₀₋₃ alkylene)-heterocyclyl are each optionally replaced by a group independently selected from -O-, -NH-, -N(C₁₋₅ alkyl)-, -CO-, -S-, -SO-, and -SO₂-; provided that R³ is not morpholin-4-yl or -NH-phenyl, wherein the phenyl group in said -NH-phenyl is optionally substituted with one or more groups R^{Cyc};
each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -(C₁₋₃ alkylene)-OH, -(C₁₋₃ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(G₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), -(C₀₋₃ alkylene)-cycloalkyl, -(C₀₋₃ alkylene)-heterocycloalkyl, and -R⁶-R⁷, wherein the cycloalkyl group in said -(C₀₋₃ alkylene)-cycloalkyl and the heterocycloalkyl group in said -(C₀₋₅ alkylene)-heterocycloalkyl are each optionally substituted with one or more groups independently selected from C₁₋₅ alkyl, C₂₋₅ alkenyl, C₂₋₅ alkynyl, halogen, C₁₋₅ haloalkyl, -O-(C₁₋₅ haloalkyl), -CN, -OH, -O(C₁₋₅ alkyl); -SH, -S(C₁₋₅ alkyl), -NH₂, -NH(C₁₋₅ alkyl), -N(C₁₋₅ alkyl)(C₁₋₅ alkyl), -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), and -SO-(C₁₋₅ alkyl);
each R⁶ is independently selected from a covalent bond, C₁₋₅ alkylene, C₂₋₅ alkenylene, and C₂₋₅ alkynylene, wherein said alkylene, said alkenylene and said alkynylene are each optionally substituted with one or more groups independently selected from halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl), and further wherein one or more -CH₂- units comprised in said alkylene, said alkenylene or said alkynylene are each optionally replaced by a group independently selected from -O-, -NH-, -N(C₁₋₅ alkyl)-, -CO-, -S-, -SO-, and -SO₂-;and
each R⁷ is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O-(C₁₋₅ haloalkyl), -CN, -CHO, -CO(C₁₋₅ alkyl), -COOH, -COO(C₁₋₅ alkyl), -O-CO(C₁₋₅. alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -O-(C₁₋₅ haloalkyl), -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).

2. The compound of claim 1, wherein ring A is a group (A-1): 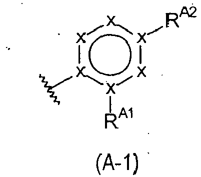 wherein the group (A-1) is phenyl or pyridinyl, wherein said phenyl or said pyridinyl is substituted with a group R^{A1} and a group R^{A2} in the indicated positions, and wherein said phenyl or said pyridinyl is optionally further substituted with one or more groups R^{A3}.

3. The compound of claim 1 or 2, wherein ring A is 2-R^{A1}-4-R^{A2}-phenyl which is optionally substituted with one or more groups R^{A3}.

4. The compound of any one of claims 1 to 3, wherein R^{A1} is selected from -O(C₁₋₅ alkyl), -O(C₂₋₅ alkenyl), C₁₋₅ alkyl, C₂₋₅ alkenyl, -N(C₁₋₅ alkyl)(C₁₋₅ alkyl), halogen, C₁₋₅ haloalkyl, -O-(C₁₋₅ haloalkyl), -CN, cycloalkyl, -O-cycloalkyl, heterocycloalkyl, and -O-heterocycloalkyl; preferably wherein R^{A1} is -O(C₁₋₅ alkyl) or halogen.

5. The compound of any one of claims 1 to 4, wherein R^{A2} is selected from C₁₋₅ alkyl, -(C₁₋₅ alkylene)-OH, -O(C₁₋₅ alkyl), -O-(C₁₋₅ alkylene)-OH, -O-(C₀₋₅ alkylene)-O(C₁₋₅ alkyl), -(C₁₋₅ alkylene)-O-(C₁₋₅ alkylene)-OH, -(C₁₋₅ alkylene)-O-(C₁₋₅ alkylene)-O(C₁₋₅ alkyl), -O(C₁₋₅ alkylene)-O(C₁₋₅ alkyl), -O(C₁₋₃ alkylene)-O(C₁₋₃ alkylene)-O(C₁₋₃ alkyl), -O(C₂₋₅ alkenyl), -(C₁₋₅ alkylene)-O(C₁₋₅ alkyl), -(C₁₋₅ alkylene)-O(C₁₋₅ alkylene)-O(C₁₋₅ alkyl), (C₁₋₃ alkylene)-O(C₁₋₃ alkylene)-O(C₁₋₃ alkylene)-O(C₁₋₃ alkyl), -O-(C₁₋₅ haloalkyl), -O(C₁₋₅ alkylene)-O-(C₁₋₅ haloalkyl), -O(C₁₋₃ alkylene)-O(C₁₋₃ alkylene)-O-(C₁₋₃ haloalkyl), -(C₁₋₅ alkylene)-O-(C₁₋₅ haloalkyl), -(C₁₋₅ alkylene)-O(C₁₋₅ alkylene)-O-(C₁₋₅ haloalkyl), -(C₁₋₃ alkylene)-O(C₁₋₃ alkylene)-O(C₁₋₃ alkylene)-O-(C₁₋₃ haloalkyl), -O(C₁₋₅ alkylene)-N(C₁₋₅ alkyl)(C₁₋₅ alkyl), -(C₁₋₅ alkylene)-O(C₁₋₅ alkylene)-N(C₁₋₅ alkyl)(C₁₋₅ alkyl), -CO-NH-(C₁₋₅ alkyl), -CO-N(C₁₋₅ alkyl)(C₁₋₅ alkyl), -SO₂-(C₁₋₁₂ alkyl), -SO₂-(C₁₋₁₂ haloalkyl), halogen, C₁₋₅ haloalkyl, (C₀₋₅ alkylene) carbocyclyl, (C₀₋₅ alkylene) heterocyclyl, O(C₀₋₄ alkylene) carbocyclyl, -O-(C₀₋₄ alkylene)-heterocyclyl, -(C₀₋₄ alkylene)-O-carbocyclyl, -(C₀₋₄ alkylene)-O-heterocyclyl, -CO-(C₀₋₄ alkylene)-carbocyclyl, and -CO-(C₀₋₄ alkylene)-heterocyclyl, wherein the carbocyclyl in said -(C₀₋₅ alkylene)-carbocyclyl, the heterocyclyl in said -(C₀₋₅ alkylene)-heterocyclyl, the carbocyclyl in said -O-(C₀₋₄ alkylene)-carbocyclyl, the heterocyclyl in said O-(C₀₋₄ alkylene) heterocyclyl, the carbocyclyl in said (C₀₋₄ alkylene)-O-carbocyclyl, the heterocyclyl in said -(C₀₋₄ alkylene)-O-heterocyclyl, the carbocyclyl in said -CO-(C₀₋₄ alkylene)-carbocyclyl, and the heterocyclyl in said -CO-(C₀₋₄ alkylene)-heterocyclyl are each optionally substituted with one or more groups R^{Cyc},

6. The compound of any one of claims 1 to 5, wherein R² is selected from hydrogen, C₁₋₅ alkyl, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), and -CN.

7. The compound of any one of claims 1 to 6, wherein R³ is selected from C₁₋₅ alkyl, C₂₋₅ alkenyl, -OH, -O(C₁₋₅ alkyl), -SH, -S(C₁₋₅ alkyl), -NH₂, -NH(C₁₋₅ alkyl), -N(C₁₋₅ alkyl)(C₁₋₅ alkyl), -CHF₂, -CH₂F, C₂₋₅ haloalkyl, -O-(C₁₋₅ haloalkyl), phenyl, cycloalkyl, cycloalkenyl, heteroaryl, heterocycloalkyl, and heterocycloalkenyl, wherein said phenyl, said cycloalkyl, said cycloalkenyl, said heteroaryl, said heterocycloalkyl and said heterocycloalkenyl are each optionally substituted with one or more groups R^{Cyc}, provided that R³ is not morpholin-4-yl.

8. The compound of claim 1, wherein said compound is selected from:
N-(2-methoxy-4-(2-methoxyethoxy)phenyl)-7-phenylquinolin-4-amine;
N-(2-methoxy-4-(2-methoxyethoxy)phenyl)-7-(pyrazin-2-yl)quinolin-4-amine;
7-methoxy-N-(2-methoxy-4-(2-methoxyethoxy)phenyl)quinolin-4-amine;
N-(2-methoxy-4-(2-methoxyethoxy)phenyl)-7-(1-methyl-1H-pyrazol-4-yl)quinolin-4-amine;
N-(2-methoxy-4-(2-methoxyethoxy)phenyl)-7-(1-methyl-1,2,3,6-tetrahydropyridin-4-yl)quinolin-4-amine;
N-(2-methoxy-4-(2-methoxyethoxy)phenyl)-7-(4-methylpiperazin-1-yl)quinolin-4-amine;
7-(4,4-difluoropiperidin-1-yl)-N-(2-methoxy-4-(2-methoxyethoxy)phenyl)quinolin-4-amine;
7-(3,3-difluoroazetidin-1-yl)-N-(2-methoxy-4-(2-methoxyethoxy)phenyl)quinolin-4-amine;
7-(difluoromethyl)-N-(2-methoxy-4-(2-methoxyethoxy)phenyl)quinolin-4-amine;
N-(2-methoxy-4-(2-methoxyethoxy)phenyl)-7-methylquinolin-4-amine;
N-(2-methoxy-4-(2-methoxyethoxy)phenyl)-7-(1-methylpiperidin-4-yl)quinolin-4-amine;
N-(2-methoxy-4-(2-methoxyethoxy)phenyl)-7-(prop-1-en-2-yl)quinolin-4-amine;
7-isopropyl-N-(2-methoxy-4-(2-methoxyethoxy)phenyl)quinolin-4-amine;
N-(2-methoxy-4-(2-methoxyethoxy)phenyl)-7-(pyridin-2-yl)quinolin-4-amine;
N-(2-methoxy-4-(2-methoxyethoxy)phenyl)-7-(thiazol-4-yl)quinolin-4-amine;
N÷(2-methoxy-4-(2-methoxyethoxy)phenyl)-7-(4-(oxetan-3-yl)piperazin-1-yl)quinolin-4-amine;
(S)-7-(3,4-dimethylpiperazin-1-yl)-N-(2-methoxy-4-(2-methoxyethoxy)phenyl)quinolin-4-amine;
(R)-7-(3,4-dimethylpiperazin-1-yl)-N-(2-methoxy-4-(2-methoxyethoxy)phenyl)quinolin-4-amine;
N-(2-methoxy-4-(2-methoxyethoxy)phenyl)-7-(4-methyl-1,4-diazepan-1-yl)quinolin-4-amine;
N-(2-methoxy-4-(2-methoxyethoxy)phenyl)-7-(4-(2-methoxyethyl)piperazin-1-yl)quinolin-4-amine;
N-(2-methoxy-4-(2-methoxyethoxy)phenyl)-7-(6-methyl-2,6-diazaspiro[3.3]heptan-2-yl)quinolin-4-amine;
7-(4-ethylpiperazin-1-yl)-N-(2-methoxy-4-(2-methoxyethoxy)phenyl)quinolin-4-amine;
(S)-7-(2,4-dimethylpiperazin-1-yl)-N-(2-methoxy-4-(2-methoxyethoxy)phenyl)quinolin-4-amine;
tert-butyl 3-(4-(4-((2-methoxy-4-(2-methoxyethoxy)phenyl)amino)quinolin-7-yl)piperazin-1-yl)azetidine-1-carboxylate;
N-(2-methoxy-4-(2-methoxyethoxy)phenyl)-7-(piperazin-1-yl)quinolin-4-amine;
N-(2-methoxy-4-(2-methoxyethoxy)phenyl)-7-(4-(1-methylazetidin-3-yl)piperazin-1-yl)quinolin-4-amine;
N-(2-methoxy-4-(2-methoxyethoxy)phenyl)-7-(4-(2,2,2-trifluoroethyl)piperazin-1-yl)quinolin-4-amine;
7-(4-(azetidin-3-yl)piperazin-1-yl)-N-(2-methoxy4-(2-methoxyethoxy)phenyl)quinolin-4-amine;
(R)-(4-(4-((2-methoxy-4-(2-methoxyethoxy)phenyl)amino)quinolin-7-yl)-1-methylpiperazin-2-yl)methanol;
(S)-(4-(4-((2-methoxy-4-(2-methoxyethoxy)phenyl)amino)quinolin-7-yl)-1-methylpiperazin-2-yl)methanol;
tert-butyl 5-(4-((2-methoxy-4-(2-methoxyethoxy)phenyl)amino)quinolin-7-yl)-2,5-diazabicyclo[2.2.1]heptane-2-carboxylate;
N-(2-methoxy-4-(2-methoxyethoxy)phenyl)-7-(5-methyl-2,5-diazabicyclo[2.2.1]heptan-2-yl)quinolin-4-amine;
N-(2-methoxy-4-(2-methoxyethoxy)phenyl)-7-(6-methyl-3,6-diazabicyclo[3.1.1]heptan-3-yl)quinolin-4-amine;
4-(4-((2-methoxy-4-(2-methoxyethoxy)phenyl)amino)quinolin-7-yl)piperazin-2-one;
N-(2-methoxy-4-(2-(trifluoromethoxy)ethoxy)phenyl)-7-(1-methyl-1,2,3,6-tetrahydropyridin-4-yl)quinolin-4-amine;
N-(2-methoxy-4-(2-(trifluoromethoxy)ethoxy)phenyl)-7-(4-methylpiperazin-1-yl)quinolin-4-amine;
N-(2-methoxy-4-(2-(trifluoromethoxy)ethoxy)phenyl)-7-(1-methylpiperidin-4-yl)quinolin-4-amine;
N-(2-methoxy-4-(2-(trifluoromethoxy)ethoxy)phenyl)-7-(1-methyl-1H-pyrazol-4-yl)quinolin-4-amine;
N-(2-methoxy-4-(2-(trifluoromethoxy)ethoxy)phenyl)-7-methylquinolin-4-amine;
7-methoxy-N-(2-methoxy-4-(2-(trifluoromethoxy)ethoxy)phenyl)quinolin-4-amine;
N-(2-methoxy-4-(2-(trifluoromethoxy)ethoxy)phenyl)-7-(pyrazin-2-yl)quinolin-4-amine;
N-(2-methoxy-4-(2-(trifluoromethoxy)ethoxy)phenyl)-7-(pyridin-2-yl)quinolin-4-amine;
N-(2-methoxy-4-(2-(trifluoromethoxy)ethoxy)phenyl)-7-(thiazol-4-yl)quinolin-4-amine;
7-(difluoromethyl)-N-(2-methoxy-4-(2-(trifluoromethoxy)ethoxy)phenyl)quinolin-4-amine;
N-(2,4-dimethoxyphenyl)-7-phenylquinolin-4-amine;
7-(difluoromethyl)-N-(4-methoxy-6-(2-methoxyethoxy)pyridin-3-yl)quinolin-4-amine;
7-(difluoromethyl)-N-(2-(methoxy-d3)-4-(2-methoxyethoxy)phenyl)quinolin-4-amine;
7-(difluoromethyl)-N-(2-methoxy-6-(2-methoxyethoxy)pyridin-3-yl)quinolin-4-amine;
7-(difluoromethyl)-N-(2-methoxy-6-(2-(trifluoromethoxy)ethoxy)pyridin-3-yl)quinolin-4-amine;
7-(difluoromethyl)-N-(2-methoxy-4-(1H-pyrazol-4-yl)phenyl)quinolin-4-amine;
7-(difluoromethyl)-N-(2-methoxy-4-(2-(trifluoromethoxy)ethoxy)phenyl)quinolin-4-amine;
7-(difluoromethyl)-N-(4-fluoro-2-methoxyphenyl)quinolin-4-amine;
N-(4-chloro-2-methoxyphenyl)-7-(difluoromethyl)quinolin-4-amine;
7-(difluoromethyl)-N-(4-methoxy-6-(2-methoxyethoxy)pyridin-3-yl)quinolin-4-amine;
7-(difluoromethyl)-N-(4-(2-methoxyethoxy)-2-morpholinophenyl)quinolin-4-amine;
N-(2-cyclopropoxy-4-(2-methoxyethoxy)phenyl)-7-(difluoromethyl)quinolin-4-amine;
7-(difluoromethyl)-N-(2-(methoxy-d3)-4-(2-(trifluoromethoxy)ethoxy)phenyl)quinolin-4-amine;
N-(4-chloro-2-methoxyphenyl)-7-(difluoromethyl)quinolin-4-amine;
N-(4-(1,1-difluoroethyl)-2-methoxyphenyl)-7-(difluoromethyl)quinolin-4-amine;
7-(difluoromethyl)-N-(2,4-dimethoxyphenyl)quinolin-4-amine;
7-(difluoromethyl)-N-(4-methoxy-2-(2-methoxyethoxy)pyrimidin-5-yl)quinolin-4-amine;
7-(difluoromethyl)-N-(2-methoxy-4-(methylsulfonyl)phenyl)quinolin-4-amine;
N-cyclopropyl-4-((7-(difluoromethyl)quinolin-4-yl)amino)-3-methoxybenzamide;
7-(difluoromethyl)-N-(2-methoxy-4-(2-(trifluoromethoxy)ethoxy)phenyl)quinolin-4-amine;
7-(difluoromethyl)-N-(2-methoxy-6-(1H-pyrazol-4-yl)pyridin-3-yl)quinolin-4-amine;
N-(4-(3,5-dimethyl-1H-pyrazol-4-yl)-2-methoxyphenyl)-7-isopropylquinolin-4-amine;
N-(4-methoxy-6-(2-methoxyethoxy)pyridin-3-yl)-7-methylquinolin-4-amine;
N-(2-methoxy-6-(2-methoxyethoxy)pyridin-3-yl)-7-methylquinolin-4-amine;
N-(2-methoxy-6-(2-(trifluoromethoxy)ethoxy)pyridin-3-yl)-7-methylquinolin-4-amine;
N-(2-methoxy-4-(1H-pyrazol-4-yl)phenyl)-7-methylquinolin-4-amine;
N-(4-(2-(difluoromethoxy)ethoxy)-2-methoxyphenyl)-7-methylquinolin-4-amine;
N-(4-fluoro-2-methoxyphenyl)-7-methylquinolin-4-amine;
N-(4-bromo-2-methoxyphenyl)-7-methylquinolin-4-amine;
N-(4-(2-methoxyethoxy)-2-morpholinophenyl)-7-methylquinolin-4-amine;
N-(2-methoxy-4-(trifluoromethyl)phenyl)-7-methylquinolin-4-amine;
N-(2-methoxy-4-(methylsulfonyl)phenyl)-7-methylquinolin-4-amine;
N-(2-cyclopropoxy-4-(2-(trifluoromethoxy)ethoxy)phenyl)-7-methylquinolin-4-amine;
N-(2-(methoxy-d3)-4-(2-(trifluoromethoxy)ethoxy)phenyl)-7-methylquinolin-4-amine;
N-(5-bromo-3-methoxypyrazin-2-yl)-7-methylquinolin-4-amine;
N-(4-methoxy-2-(2-methoxyethoxy)pyrimidin-5-yl)-7-methylquinolin-4-amine;
N-(4-(cyclopentyloxy)-2-methoxyphenyl)-7-methylquinolin-4-amine;
N-(4-methoxy-2-(2-(trifluoromethoxy)ethoxy)pyrimidin-5-yl)-7-methylquinolin-4-amine;
N-(4-chloro-2-methoxyphenyl)-7-methylquinolin-4-amine;
N-(4-(2-isopropoxyethoxy)-2-methoxyphenyl)-7-methylquinolin-4-amine;
N-(2-methoxy-6-(1H-pyrazol-4-yl)pyridin-3-yl)-7-methylquinolin-4-amine;
N-(2-methoxy-4-(1H-pyrazol-1-yl)phenyl)-7-methylquinolin-4-amine;
N-(4-(3,5-dimethyl-1H-pyrazol-4-yl)-2-methoxyphenyl)-7-methylquinolin-4-amine;
N-(4-(furan-2-yl)-2-methoxyphenyl)-7-methylquinolin-4-amine;
N-(4-(furan-3-yl)-2-methoxyphenyl)-7-methylquinolin-4-amine;
N-(2-methoxy-4-(1H-pyrazol-5-yl)phenyl)-7-methylquinolin-4-amine;
N-(2-methoxy-4-(4-methyl-1H-pyrazol-1-yl)phenyl)-7-methylquinolin-4-amine;
N-(4-(1,3-dimethyl-1H-pyrazol-4-yl)-2-methoxyphenyl)-7-methylquinolin-4-amine;
N-(2-methoxy-4-(thiophen-2-yl)phenyl)-7-methylquinolin-4-amine;
N-(2-methoxy-4-(thiophen-3-yl)phenyl)-7-methylquinolin-4-amine;
N-(7-bromobenzo[d][1,3]dioxol-4-yl)-7-methylquinolin-4-amine;
N-(7-(1H-pyrazol-4-yl)benzo[d][1,3]dioxol-4-yl)-7-methylquinolin-4-amine;
7-methoxy-N-(2-(methoxy-d3)-4-(2-methoxyethoxy)phenyl)quinolin-4-amine;
N-(4-(2-(difluoromethoxy)ethoxy)-2-methoxyphenyl)-7-methoxyquinolin-4-amine;
N-(4-(2-(difluoromethoxy)ethoxy)-2-methoxyphenyl)-7-(1-methyl-1H-pyrazol-4-yl)quinolin-4-amine; or a pharmaceutically acceptable salt of any one of these compounds.

9. A pharmaceutical composition comprising the compound of any one of claims 1 to 8 and a pharmaceutically acceptable excipient.

10. The compound of any one of claims 1 to 8 or the pharmaceutical composition of claim 9 for use in the treatment or prevention of cancer, an inflammatory disease, or an autoimmune disease.

11. The compound for use according to claim 10 or the pharmaceutical composition for use according to claim 10, wherein said compound or said pharmaceutical composition is for use in the treatment or prevention of cancer, and wherein said cancer is selected from breast cancer, prostate cancer, liver cancer, lung cancer, colon cancer, colorectal cancer, gastrointestinal cancer, pancreatic cancer, cervical cancer, ovarian cancer, kidney cancer, head cancer, neck cancer, blood cancer, Merkel carcinoma, lymphoma, leukemia, bone cancer, bone marrow cancer, brain cancer, esophagus cancer, gum cancer, nasopharynx cancer, skin cancer, melanoma, stomach cancer, tongue cancer, uterus cancer, anal cancer, genitourinary cancer, bladder cancer, endometrial cancer, vaginal cancer, vulvar cancer, testicular cancer, biliary tract cancer, hepatobiliary cancer, neuroblastoma, epidermoid cancer, squamous cell carcinoma, fibrosarcoma, Ewing's sarcoma, malignant mesothelioma, laryngeal cancer, mouth cancer, thymoma, neuroendocrine cancer, and multiple myeloma.

12. The compound for use according to claim 10 or the pharmaceutical composition for use according to claim 10, wherein said compound or said pharmaceutical composition is for use in the treatment or prevention of an inflammatory disease, and wherein said inflammatory disease is selected from arthritis, rheumatoid arthritis, ankylosing spondylitis, osteoarthritis, psoriatic arthritis, juvenile idiopathic arthritis, juvenile rheumatoid arthritis, arthritis uratica, gout, chronic polyarthritis, periarthritis humeroscapularis, cervical arthritis, lumbosacral arthritis, enteropathic arthritis, ankylosing spondylitis, asthma, dermatitis, psoriasis, scleroderma, polymyositis, dermatomyositis, juvenila dermatomyositis, primary biliary cirrhosis, fibrosis, cystic fibrosis, pulmonary fibrosis, cirrhosis, endomyocardial fibrosis, dediastinal fibrosis, myelofibrosis, retroperitoneal fibrosis, nephrogenic fibrosis, Keloids, scleroderma, arthrofibrosis, post-transplantation late and/or chronic solid organ rejection, multiple sclerosis, systemic lupus erythematosus, lupus nephritis, pemphigus, pemphigus vulgaris, pemphigus herpetiformis, pemphigus vegetans, IgA pemphigus, pemphigus erythematosus, bullous pemphigoid, pemphigoid gestationis, mucous membrane dermatosis, pemphigoid nodularis, linear IgA bullous dermatosis, bullous lichen planus, epidermolysis bullosa acquisita, autoimmune diabetes, diabetic retinopathy, diabetic nephropathy, diabetic vasculopathy, ocular inflammation, uveitis, rhinitis, ischemiareperfusion injury, post-angioplasty restenosis, chronic obstructive pulmonary disease, glomerulonephritis, Graves disease, gastrointestinal allergy, conjunctivitis, atherosclerosis, coronary artery disease, angina, small artery disease, acute disseminated encephalomyelitis, idiopathic thrombocytopenic purpura, systemic sclerosis, antiphospholipid syndrome, Sjögren's syndrome, autoimmune hemolytic anemia, colitis, Crohn's disease, ulcerative colitis, inflammatory bowel disease, embolism, pulmonary embolism, arterial embolism, venous embolism, allergic inflammation, cardiovascular disease, graft-related disease, graft-versus-host disease, disorder associated with graft transplantation rejection, degeneration after trauma, stroke, transplant rejection, an allergic condition, hypersensitivity, allergic rhinitis, allergic eczema, and a skin/dermal inflammatory disorder.

13. The compound for use according to claim 10 or the pharmaceutical composition for use according to claim 10, wherein said compound or said pharmaceutical composition is for use in the treatment or prevention of an autoimmune disease, and wherein said autoimmune disease is selected from lupus, Hashimoto's thyroiditis, Wegener's disease, primary myxedema, Graves' disease, pernicious anemia, autoimmune atrophic gastritis, Addison's disease, diabetes, good pasture's syndrome, myasthenia gravis, pemphigus, an intestinal inflammatory disease, Crohn's disease, ulcerative colitis, sympathetic ophthalmia, autoimmune uveitis, multiple sclerosis, autoimmune hemolytic anemia, idiopathic thrombocytopenia, primary biliary cirrhosis, chronic action hepatitis, ulcerative colitis, Sjögren's syndrome, arthritis, rheumatoid arthritis, psoriatic arthritis, ankylosing spondylitis, juvenile idiopathic arthritis, polymyositis, scleroderma, psoriasis, primary sclerosing cholangitis, asthma, transplant rejection, host-versus-graft disease, graft-versus-host disease, and mixed connective tissue disease.

14. *In vitro* use of the compound of any one of claims 1 to 8 as a NIK inhibitor.
